# EUROPEAN PATENT APPLICATION

(11) **EP 1 333 031 A1**
(43) Date of publication of application: **06.08.2003**
(21) Application number: 01982731.0
(22) Date of filing: 01.11.2001
(51) Int. Cl.: C07D 471/04, C07D 513/04, C07D 487/04

(54) **BICYCLIC TRIAZOLONE DERIVATIVES AND HERBICIDES CONTAINING THE SAME**

(30) Priority: 08.11.2000 JP 2000340525; 17.10.2001 JP 2001319530
(71) Applicant: Sumitomo Chemical Takeda Agro Company, Limited, Tokyo 103-0027 (JP)
(72) Inventor: KURAGANO, Takashi, Tsukuba-shi, Ibaraki 305-0821 (JP); TANAKA, Yasushi, Tsukuba-shi, Ibaraki 305-0821 (JP)
(74) Representative: Teipel, Stephan, Dr.
(86) International application number: JP0109608
(87) International publication number: WO02038562

(57) **Abstract**

The present invention provides a bicyclic triazolone derivative represented by the formula:

J-Ar (I)

[wherein, J is 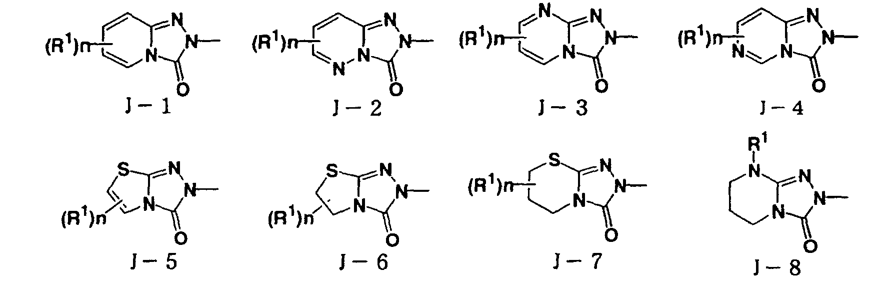 and Ar is 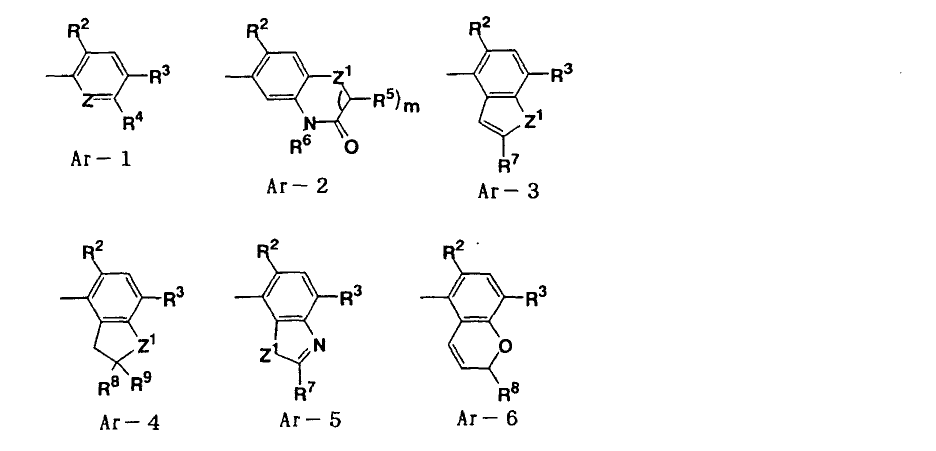 ], which has excellent selective weeding activity and weed killer containing the said bicyclic triazolone derivative.

## Description

### Technical Field

This invention relates to bicyclic triazolone derivatives and herbicides containing the bicyclic triazolone derivatives. The bicyclic triazolone derivatives in this invention exert excellent weeding effects on weeds grown in paddy fields or farmlands. Moreover, the triazolone derivatives in this invention are useful as selective herbicides for weeds in paddy fields or farmlands, and are not harmful to a rice plant, wheat, barley, maize, cotton, soybeans and so on.

### Background Art

Heretofore, several triazolone derivatives as cyclic imide herbicides have ever been reported (Japanese Patent Publication for Laid-Open 105495/1978, Japanese Patent Publication for Laid-Open 293744/1994). However, the past herbicides containing cyclic imide were not sufficient in weeding effects on weeds, phytotoxic effects on crops, toxic effects on mammals, fishes or shellfishes, environmental pollution and so on. From these aspects, the development of improved selective herbicides is desired.

### Disclosure of Invention

Taking these actual situation into consideration, this invention is to provide herbicides containing cyclic imide which exert excellent selective weeding effects on weeds in paddy fields or farmlands.

The present inventors have made intensive efforts in order to develop selective herbicides which can exert excellent weeding effects on weeds in paddy fields or farmlands, and are not phytotoxic to crops. As the results, they have found that the bicyclic triazolone derivatives represented by the formula (I) or salts thereof have a strong weeding action, and that the phytotoxic effect on a rice plant, wheat, barley, maize, cotton, soybeans and so on can be remarkably lowered using the above-mentioned triazolone derivatives or salts thereof, and that high selective weeding action can be shown. The present inventors carried out an intensive research based on and encouraged by these findings and have completed the present invention. No report has been reported regarding the bicyclic triazolone derivatives in this invention and its weeding action, thus the compound represented by formula (I) is a completely new compound.

Thus,the present invention relates to ;
[1] A bicyclic triazolone derivative of the formula (I) :

   J-Ar (I)

   [Wherein J is one of the groups represented by the formula described bellow; 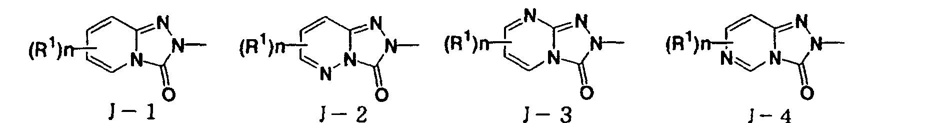 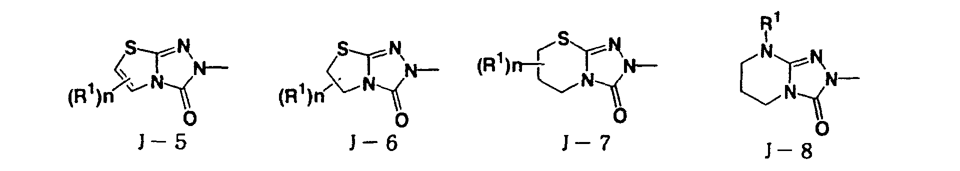 (wherein R¹ is hydrogen, halogen, a C₁₋₆alkyl group, a C₂₋₆alkenyl group, a C₂₋₆alkynyl group, a C₃₋₆cycloalkyl group, a C₇₋₁₂aralkyl group, a C₆₋₁₀aryl group, a C₁₋₆alkoxyC₁₋₄alkyl group, a C₁₋₆alkoxy group, a C₂₋₆alkenyloxy group, a C₂₋₆alkynyloxy group, a C₇₋₁₂aralkyloxy group, a C₁₋₆haloalkoxy group, a C₂₋₆haloalkenyl group, a C₆₋₁₀aryloxy group, a C₁₋₆alkylthio group, a C₂₋₆alkenylthio group, a C₂₋₆alkynylthio group, a C₇₋₁₂aralkylthio group, a C₆₋₁₀arylthio group, a C₁₋₆alkylsulfonyl group, a C₂₋₆alkenylsulfonyl group, a C₂₋₆alkynylsulfonyl group, a C₇₋₁₂aralkylsulfonyl group, a C₆₋₁₀arylsulfonyl group, a cyclic-1,3-dioxa-2-yl group, a cyclic-1,3-dithio-2-yl group, a C₁₋₇alkanoyl group, a C₇₋₁₁arylcarbonyl group, a C₂₋₇alkoxycarbonyl group, a C₈₋₁₃aralkyloxycarbonyl group, a mono-C₁₋₄ or di(C₁₋₄)alkylcarbamoyl group, an amino group, a C₁₋₇alkanoylamino group, a mono-C₁₋₄ or di(C₁₋₄)alkylamino group, a C₁₋₂alkylenedioxy group, nitro group, hydroxy group, mercapto group, cyano group, carboxyl group, or sulfo group, n is an integer from 1 to a substitutable maximum number),
   Ar is a phenyl group which may be substituted, a pyridyl group or a condensed heterocyclic group with the phenyl group or the pyridyl group which may be substituted] or a salt thereof.
[2] A bicyclic triazolone derivative or a salt thereof as described in [1] above, wherein Ar is one of the groups 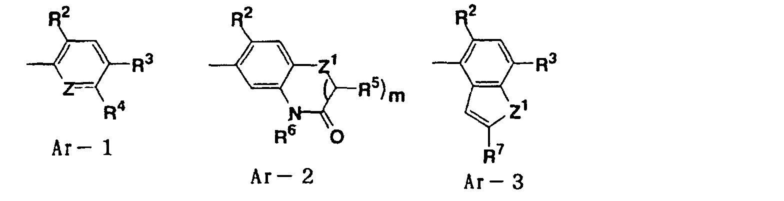 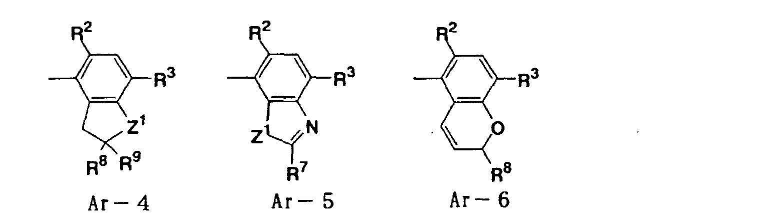 [wherein R² is hydrogen, halogen, a C₁₋₆alkyl group, a C₁₋₆haloalkyl group, a C₁₋₆alkoxy group or a C₁₋₆haloalkoxy group,
   R³ is halogen, nitro group, cyano group, carbamoyl group, a hydroxylC₁₋₄alkyl group, a C₁₋₆alkoxyC₁₋₄alkyl group, a C₂₋₇alkoxycarbonyl group, a C₃₋₇alkenyloxycarbonyl group, a C₃₋₇alkynyloxycarbonyl group, or a C₇₋₁₂aralkyloxy group which may be substituted by a substituent or substituents selected from halogen, a C₁₋₆alkyl group, a C₁₋₆haloalkyl group, a C₁₋₆alkoxy group, a C₁₋₆haloalkoxy group, a C₂₋₆alkenyloxy group, a C₂₋₆alkynyloxy group, a C₂₋₇alkoxycarbonyl group, a C₃₋₇alkenyloxycarbonyl group, a C₃₋₇alkynyloxycarbonyl group, a C₂₋₇alkoxycarbonylC₁₋₄alkoxy group, a C₃₋₇alkenyloxycarbonylC₁₋₄alkoxy group, and a C₃₋₇alkynyloxycarbonylC₁₋₄alkoxy group, the number of the substituent for the substitution is an integer from 1 to a substitutable maximum number),
   R⁴ is hydrogen, a C₁₋₆alkyl group, a C₃₋₆cycloalkyl group, a C₁₋₆haloalkyl group, hydroxyl group, mercapto group, a C₁₋₆alkoxy group, a C₁₋₆alkoxyC₁₋₄alkoxy group, a C₃₋₆cycloalkyloxy group, a C₁₋₆haloalkoxy group, a C₂₋₆alkenyloxy group, a C₂₋₆haloalkenyloxy group, a C₂₋₆alkynyloxy group, a C₆₋₁₀aryloxy group, a C₇₋₁₂aralkyloxy group, a C₁₋₆alkylthio group, a C₁₋₆alkoxyC₁₋₄alkylthio group, a C₃₋₆cycloalkylthio group, a C₁₋₆haloalkylthio group, a C₂₋₆alkenylthio group, a C₂₋₆haloalkenylthio group, a C₂₋₆alkynylthio group, a C₆₋₁₀arylthio group, a C₇₋₁₂aralkylthio group, C₁₋₆alkylsulfonyl group, a C₃₋₆cycloalkylsulfonyl group, a C₁₋₆haloalkylsulfonyl group, a C₂₋₆alkenylsulfonyl group, a C₂₋₆alkynylsulfonyl group, a cyclic amino group having one or two atoms selected from oxygen, sulfur and nitrogen or one of the groups represented by formulas; 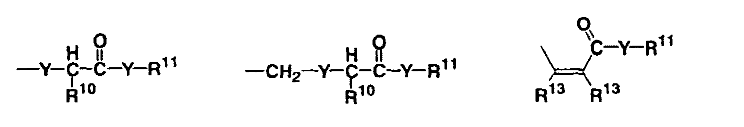 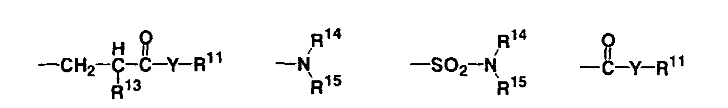 (Wherein Y is oxygen, sulfur or -N-R¹²,
   R¹⁰ is hydrogen or a C₁₋₆alkyl group,
   R¹¹ is hydrogen, a C₁₋₆alkyl group, a C₁₋₆haloalkyl group, a C₃₋₆cycloalkyl group, a C₂₋₆alkenyl group, a C₂₋₆haloalkenyl group, a C₂₋₆alkynyl group, a C₆₋₁₀aryl group, a C₇₋₁₂aralkyl group, a C₁₋₆alkoxyC₁₋₄alkyl group, a C₂₋₆alkenyloxyC₁₋₄alkyl group, a C₂₋₆alkynyloxyC₁₋₄alkyl group, a C₃₋₆cycloalkoxyC₁₋₄alkyl group, a C₂₋₇alkoxycarbonylC₁₋₄alkyl group, a C₃₋₇alkenyloxycarbonylC₁₋₄alkyl group, a C₃₋₇alkynyloxycarbonylC₁₋₄alkyl group, a C₄₋₇cycloalkoxycarbonylC₁₋₄alkyl group, a C₂₋₇haloalkoxycarbonylC₁₋₄alkyl group, a C₃₋₇haloalkenyloxycarbonylC₁₋₄alkyl group, or a C₇₋₁₂aralkyloxycarbonylC₁₋₄alkyl group,
   R¹² is hydrogen, a C₁₋₆alkyl group, a C₁₋₆alkoxyC₁₋₄alkyl group, a C₁₋₇alkanoyl group, a C₇₋₁₁arylcarbonyl group, C₂₋₇alkoxycarbonyl group, a C₂₋₇haloalkoxycarbonyl group, or a C₃₋₇haloalkenyloxycarbonyl group,
   R¹³ is hydrogen, halogen, or a C₁₋₆alkyl group,
   each of R¹⁴ and R¹⁵ is same or differnetly hydrogen, a C₁₋₆alkyl group, a C₂₋₆alkenyl group, a C₂₋₆alkynyl group, a C₃₋₆cycloalkyl group, a C₁₋₆alkoxyC₁₋₄alkyl group, a C₆₋₁₂aralkyl group, a C₁₋₄alkyl group which is substituted with 5- or 6- membered hetero ring containing nitrogen, oxygen or sulfur, a C₁₋₇alkanoyl group, a C₆₋₁₂arylcarbonyl group, a C₂₋₇haloalkylcarbonyl group, a C₂₋₇alkoxycarbonyl group, a C₃₋₇alkenyloxycarbonyl group, a C₃₋₇alkynyloxycarbonyl group, a C₄₋₇cycloalkyloxycarbonyl group, a C₂₋₇haloalkoxycarbonyl group, a C₃₋₇haloalkenyloxycarbonyl group, a C₁₋₆alkylsulfonyl group, a C₂₋₇alkenylsulfonyl group, a C₂₋₇alkynylsulfonyl group, a C₃₋₆cycloalkylsulfonyl group, a C₁₋₆haloalkylsulfonyl group, a C₆₋₁₀arylsulfonyl group, a C₇₋₁₂aralkylsulfonyl group, or a group represented by formula; 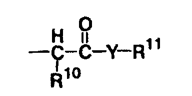 (wherein each of R¹⁰, R¹¹ and Y has the same meaning as described above),
   R⁵ is hydrogen or a C₁₋₆alkyl group; m is 0 or 1,
   R⁶ is hydrogen, a C₁₋₆alkyl group, a C₁₋₆haloalkyl group, a C₂₋₆alkenyl group, a C₂₋₆alkynyl group, a C₁₋₆alkoxyC₁₋₄alkyl group, a C₁₋₇alkanoyl group, a C₆₋₁₀arylcarbonyl group, a C₂₋₇alkoxycarbonyl group, a C₂₋₇haloalkoxycarbonyl group, a C₃₋₇alkenyloxycarbonyl group, a C₃₋₇haloalkenyloxycarbonyl group, a C₃₋₇alkynyloxycarbonyl group, a C₄₋₇cycloalkyloxycarbonyl group, a C₂₋₇alkoxycarbonylC₁₋₄alkyl group, a C₂₋₇haloalkoxycarbonylC₁₋₄alkyl group, a C₃₋₇alkenyloxycarbonylC₁₋₄alkyl group, a C₃₋₇haloalkenyloxycarbonylC₁₋₄alkyl group, or a C₃₋₇alkynyloxycarbonylC₁₋₄alkyl group,
   R⁷ is hydrogen, halogen, a C₁₋₆alkyl group, a C₁₋₆alkoxy group, a C₁₋₆haloalkoxy group, a C₂₋₆alkenyloxy group, a C₂₋₆alkynyloxy group, a C₁₋₆alkylthio group, a C₁₋₆haloalkylthio group, a C₂₋₆alkenylthio group, a C₂₋₆alkynylthio group, a C₁₋₆haloalkyl group, a C₁₋₆alkoxyC₁₋₄alkyl group, a C₁₋₆alkylthioC₁₋₄alkyl group, or a C₁₋₆alkylsulfonylC₁₋₄alkyl group,
   R⁸ is hydrogen, or a C₁₋₆alkyl group,
   R⁹ is a C₁₋₆alkyl group,
   Z is CH or nitrogen,
   Z¹ is oxygen, sulfur or methylene.].
[3] A bicyclic triazolone derivative or a salt thereof as described in [1] above, wherein J in the formula (I) is J-1.
[4] A bicyclic triazolone derivative or a salt thereof as described in [1] above, wherein J in the formula (I) is J-2.
[5] A bicyclic triazolone derivative or a salt thereof as described in [1] above, wherein J in the formula (I) is J-3.
[6] A bicyclic triazolone derivative or a salt thereof as described in [1] above, wherein J in the formula (I) is J-4.
[7] A bicyclic triazolone derivative or a salt thereof as described in [1] above, wherein J in the formula (I) is J-5.
[8] A bicyclic triazolone derivative or a salt thereof as described in [1] above, wherein J in the formula (I) is J-6.
[9] A bicyclic triazolone derivative or a salt thereof as described in [1] above, wherein J in the formula (I) is J-7.
[10] A bicyclic triazolone derivative or a salt thereof as described in [1] above, wherein J in the formula (I) is J-8.
[11] A herbicidal composition, which comprises a bicyclic triazolone derivative or a salt thereof as described in any of [1] to [10] above.

### Best Mode for Carrying Out the Invention

In formula (I), R¹ is hydrogen, halogens (for example, fluorine, chlorine, bromine, iodine and so on), C₁₋₆alkyl group (for example, straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl and so on), C₂₋₆alkenyl group (for example, allyl, 1-buten-3-yl, 3-buten-1-yl and so on), C₂₋₆alkynyl group (for example, propargyl, 2-butyn-1-yl, 3-butyn-1-yl and so on), C₃₋₆cycloalkyl group (for example, cyclopropyl, cyclobutyl, cyclopentyl, cyclohexyl and so on), C₇₋₁₂ aralkyl group(for example, benzyl, phenethyl and so on), C₆₋₁₀aryl group(for example, phenyl, naphthyl and so on), C₁₋₆alkoxyC1-4alkyl group (for example, methoxymethyl, ethoxymethyl, n-propoxymethyl, isopropoxymethyl, 2-methoxyethyl and so on), C₁₋₆alkoxy group (for example, methoxy, ethoxy, n-propoxy, isopropoxy, n-butoxy, isobutoxy, sec-butoxy, tert-butoxy, n-pentyloxy and so on), C₂₋₆alkenyloxy group (for example, allyloxy, 1-buten-3-yloxy, 3-buten-1-yloxy and so on), C₂₋₆alkynyloxy group (for example, propargyloxy,2-butyn-1-yloxy, 3-butyn-2-yloxy and so on), C₇₋₁₂aralkyloxy group (for example, benzyloxy, phenethyloxy and so on), C₁₋₆haloalkoxy group (for example, 2-chloroethoxy, trifluoromethoxy and so on), C₂₋₆haloalkenyloxy group (for example, 2-chloro-2-propen-1-yloxy and so on), C₆₋₁₀aryloxy group (for example, phenoxy and so on), C₁₋₆alkylthio group (for example, methylthio, ethylthio, n-propylthio, isopropylthio, sec-butylthio, n-pentylthio and so on), C₂₋₆alkenylthio group (for example, allylthio, 1-buten-3-ylthio, 3-buten-1-ylthio and so on), C₂₋₆alkynylthio group (for example, propargylthio, 2-butyn-1-ylthio, 3-butyn-2-ylthio and so on), C₇₋₁₂aralkylthio group (for example, benzylthio, phenethylthio and so on), C₆₋₁₀arylthio group (for example, phenylthio, naphthylthio and so on), C₁₋₆alkylsulfonyl group (for example, methylsulfonyl, ethylsulfonyl, n-propylsulfonyl, isopropylsulfonyl, n-butylsulfonyl, isobutylsulfonyl, sec-butylsulfonyl, tert-butylsulfonyl, n-pentylsulfonyl, sec-pentylsulfonyl, isopentylsulfonyl, neopentylsulfonyl, n-hexylsulfonyl, isohexylsulfonyl and so on), C₂₋₆alkenylsulfonyl group (for example, allylsulfonyl, 1-buten-3-ylsulfonyl, 3-buten-1-ylsulfonyl and so on), C₂₋₆alkynylsulfonyl group (for example, propalgylsulfonyl, 2-butyn-1-ylsulfonyl, 3-butyn-2-ylsulfonyl and so on), C₇₋₁₂aralkylsulfonyl group (for example, benzylsulfonyl, phenethylsulfonyl and so on), C₆₋₁₀arylsulfonyl group (for example, phenylsulfonyl, naphthylsulfonyl and so on), cyclic-1,3-dioxa-2-yl group (for example, 1,3-dioxan-2-yl, 1,3-dioxolan-2-yl and so on), cyclic-1,3-dithio-2-yl group (for example, 1,3-dithian-2-yl, 1,3-dithiolan-2-yl and so on), C₁₋₇alkanoyl group (for example, formyl, acetyl, propionyl, butyryl, isobutyryl, pentanoyl, hexanoyl and so on), C₆₋₁₂arylcarbonyl group (for example, benzoyl, naphthalenecarbonyl and so on), C₂₋₇alkoxycarbonyl group (for example, methoxycarbonyl, ethoxycarbonyl, n-propoxycarbonyl, isopropoxycarbonyl, n-butoxycarbonyl, isobutoxycarbonyl, n-pentyloxycarbonyl and so on), C₈₋₁₃aralkyloxycarbonyl group (for example, benzyloxycarbonyl, phenethyloxycarbonyl and so on), mono-C₁₋₄ or di(C₁₋₄)alkylcarbamoyl group (for example, monomethylcarbamoyl, monoethylcarbamoyl, dimethylcarbamoyl, diethylcarbamoyl and so on), amino group, C₁₋₇alkanoyl amino group(for example, acetylamino, propionylamino and so on), mono-C₁₋₄ or di(C₁₋₄)alkylamino group (for example, monomethylamino, diethylamino and so on), C₁₋₃alkylenedioxy group (for example, methylenedioxy, ethylenedioxy and so on), nitro group, hydroxyl group, mercapto group, cyano group, carboxyl group, or sulfo group (-SO₃H).
n represents an integer from 1 to substitutable maximum number, and the groups may be same or different in the case where n is more than 2.

R² is hydrogen, halogens (for example, fluorine, chlorine, bromine, iodine and so on), C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl and so on), C₁₋₆haloalkyl group (for example, chloromethyl, 2-chloroethyl, trifluoromethyl and so on), C₁₋₆alkoxy group (for example, methoxy, ethoxy, n-propoxy, isopropoxy, n-butoxy, isobutoxy, sec-butoxy, tert-butoxy, n-pentyloxy and so on), or C₁₋₆haloalkoxy group (for example, trifluoromethoxy and so on).

R³ is halogens (for example, fluorine, chlorine, bromine, iodine and so on), nitro group, cyano group, carbamoyl group, hydroxyC₁₋₄alkyl group (for example, hydroxymethyl, 1-(hydroxy)ethyl and so on), C₁₋₆alkoxyC₁₋₄alkyl group (for example, methoxymethyl, ethoxymethyl, n-propoxymethyl, isopropoxymethyl and so on), C₂₋₇alkoxycarbonyl group (for example, methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl, n-pentyloxycarbonyl and so on), C₃₋₇alkenyloxycarbonyl group (for example, allyloxycarbonyl, 1-buten-3-yloxycarbonyl, 3-buten-1-yloxycarbonyl and so on), C₃₋₇alkynyloxycarbonyl group(for example, propargyloxycarbonyl, 2-butyn-1-yloxycarbonyl, 3-butyn-2-yloxycarbonyl and so on), C₇₋₁₂aralkyloxy group which may be substituted by a substituent or substituents selected from halogen(for example, fluorine, chlorine, bromine, iodine and so on), C₁₋₆alkyl group(straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl and so on), C₁₋₆haloalkyl group (for example, chloromethyl, bromomethyl, 1-chloroethyl, trifluoromethyl and so on), C₁₋₆alkoxy group (for example, methoxy, ethoxy, n-propoxy, isopropoxy, n-butoxy, isobutoxy, sec-butoxy, tert-butoxy, n-pentyloxy and so on), C₁₋₆haloalkoxy group (for example, trifluoromethoxy and so on), C₂₋₆alkenyloxy group (for example, allyloxy, 1-buten-3-yloxy, 3-buten-1-yloxy and so on), C₂₋₆alkynyloxy group (for example, propargyloxy, 2-butyn-1-yloxy, 3-butyn-2-yloxy and so on), C₂₋₇alkoxycarbonyl group (for example, methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl, n-pentyloxycarbonyl and so on), C₃₋₇alkenyloxycarbonyl group (for example, allyloxycarbonyl, 1-buten-3-yloxycarbonyl, 3-buten-1-yloxycarbonyl and so on), C₃₋₇alkynyloxycarbonyl group (for example, propargyloxycarbonyl, 2-butyn-1-yloxycarbonyl, 3-butyn-1-yloxycarbonyl and so on), C₂₋₇alkoxycarbonylC₁₋₄alkoxy group (for example, methoxycarbonylmethoxy, ethoxycarbonylmethoxy, n-propoxycarbonylmethoxy, isopropoxycarbonylmethoxy, n-butoxycarbonylmethoxy, isobutoxycarbonylmethoxy, sec-butoxycarbonylmethoxy, tert-butoxycarbonylmethoxy, n-pentyloxycarbonylmethoxy, 1-(methoxycarbonyl)ethoxy, 1-(ethoxycarbonyl)ethoxy and so on), C₃₋₇alkenyloxycarbonylC₁₋₄alkoxy group (for example, allyloxycarbonylmethoxy, 1-buten-3-yloxycarbonylmethoxy, 3-buten-1-yloxycarbonylmethoxy and so on), or C₃₋₇alkynyloxycarbonylC₁₋₄alkoxy group (for example, propargyloxycarbonylmethoxy, 2-butyn-1-yloxycarbonylmethoxy, 3-butyn-2-yloxycarbonylmethoxy and so on), the number of the substituents is an integer from one to substitutable maximum number. The substituents are the same or different in the case where the number is more than 2.

R⁴ is hydrogen, C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl and so on), C₃₋₆cycloalkyl group (for example, cyclopropyl, cyclobutyl, cyclohexyl and so on), C₁₋₆haloalkyl group (for example, chloromethyl, bromomethyl and so on), hydroxyl group, mercapto group, C₁₋₆alkoxy group (for example, methoxy, ethoxy, n-propoxy, isopropoxy, n-butoxy, isobutoxy, sec-butoxy, tert-butoxy, n-pentyloxy and so on), C₁₋₆alkoxyC₁₋₄alkoxy group (for example, methoxymethoxy, ethoxymethoxy, n-propoxymethoxy, isopropoxymethoxy, n-butoxymethoxy and so on), C₃₋₆cycloalkyloxy group (for example, cyclopropyloxy, cyclobutyloxy, cyclopentyloxy and so on), C₁₋₆haloalkoxy group(for example, trifluoromethoxy and so on), C₂₋₆alkenyloxy group (for example, allyloxy, 1-buten-3-yloxy, 3-buten-1-yloxy and so on), C₂₋₆haloalkenyloxy group (for example, 2-chloro-2-propen-1-yloxy and so on), C₂₋₆alkynyloxy group (for example, propargyloxy, 2-butyn-1-yloxy, 3-butyn-2-yloxy and so on), C₆₋₁₀aryloxy group (for example, phenoxy, naphthyloxy and so on), C₇₋₁₂aralkyloxy group (for example, benzyloxy, phenethyloxy and so on), C₁₋₆alkylthio group(for example, methylthio, ethylthio, n-propylthio, isopropylthio, n-butylthio, isobutylthio, sec-butylthio, tert-butylthio, n-pentylthio, sec-pentylthio, isopentyithio, neopentylthio, n-hexylthio, isohexylthio and so on), C₁₋₆alkoxyC₁₋₄alkylthio group (for example, methoxymethylthio, ethoxymethylthio, methoxyethylthio and so on), C₃₋₆cycloalkylthio group (for example, cyclopropylthio, cyclobutylthio, cyclopentylthio and so on), C₁₋₆haloalkylthio group (for example, trifluoromethylthio and so on), C₂₋₆alkenylthio (for example, allylthio, 1-buten-3-ylthio, 3-buten-1-ylthio and so on), C₂₋₆haloalkenylthio group (for example, 2-chloro-2-propen-1-ylthio and so on), C₂₋₆alkynylthio group (for example, propargylthio, 2-butyn-1-ylthio, 3-butyn-2-ylthio and so on), C₆₋₁₀arylthio group (for example, phenylthio, naphthylthio and so on), C₇₋₁₂aralkylthio group (for example, benzylthio, phenethylthio and so on), C₁₋₆alkylsulfonyl group (for example, methylsulfonyl, ethylsulfonyl, n-propylsulfonyl, isopropylsulfonyl, n-butylsulfonyl, isobutylsulfonyl, sec-butylsulfonyl, tert-butylsulfonyl, n-pentylsulfonyl, sec-pentylsulfonyl, isopentylsulfonyl, neopentylsulfonyl, n-hexylsulfonyl, isohexylsulfonyl and so on), C₃₋₆cycloalkylsulfonyl group (for example, cyclopropylsulfonyl, cyclobutylsulfonyl, cyclopentylsulfonyl and so on), C₁₋₆haloalkylsulfonyl group (for example, chloromethylsulfonyl, trifluoromethylsulfonyl and so on), C₂₋₆alkenylsulfonyl group (for example, allylsulfonyl, metallylsulfonyl and so on), C₂₋₆alkynylsulfonyl group (for example, propargylsulfonyl and so on), or cyclic amino group which including 1 or 2 atom(s) selected from oxygen, sulfur and nitrogen(for example, morpholino, pyrrolidino, piperidino).

Also, R⁴ may be one of the groups represented by any one of the following formulas; 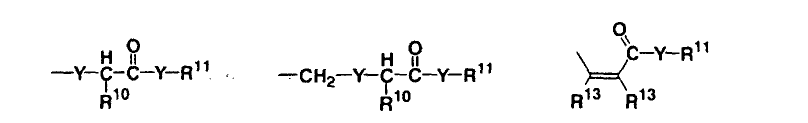 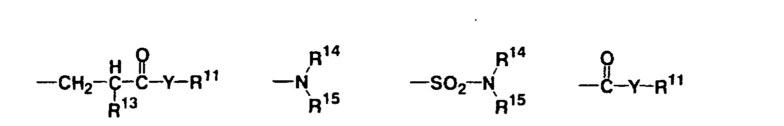 , Y is oxygen, sulfur or -N-R¹², R¹⁰ is hydrogen, C₁₋₆alkyl group (for example, straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl and so on).

R¹¹ is hydrogen, C₁₋₆alkyl group (for example, straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl and so on), C₃₋₆cycloalkyl group (for example, cyclopropyl, cyclobutyl, cyclohexyl and so on), C₁₋₆haloalkyl group (for example, chloromethyl, 2-chloroethyl, trifluoromethyl and so on), C₂₋₆alkenyl group (for example, allyl, 1-buten-3-yl, 3-buten-1-yl and so on), C₂₋₆haloalkenyl group (for example, 2-chloro-2-propen-1-yl and so on), C₂₋₆alkynyl group (for example, propargyl, 2-butyn-1-yl, 3-butyn-2-yl and so on), C₆₋₁₀aryl group (for example, phenyl, naphthyl and so on), C₇₋₁₂aralkyl group (for example, benzyl, phenethyl and so on), C₁₋₆alkoxyC₁₋₄alkyl group (for example, methoxymethyl, ethoxymethyl, 2-methoxyethyl, 2-ethoxyethyl and so on), C₂₋₆alkenyloxyC₁₋₄alkyl group (for example, allyloxymethyl, 1-buten-3-yloxymethyl, 3-buten-1-yloxymethyl and so on), C₂₋₆alkynyloxyC₁₋₄alkyl group (for example, propargyloxymetyl, 2-butyn-1-yloxymethyl, 3-butyn-1-yloxymethyl and so on), C₃₋₆cycloalkoxyC₁₋₄alkyl group (for example, cyclopropyloxymethyl, cyclobutyloxymethyl and so on), C₂₋₇alkoxycarbonylC₁₋₄alkyl group (for example, methoxycarbonylmethyl, ethoxycarbonylmethyl, isopropoxycarbonylmethyl, sec-butoxycarbonylmethyl, 1-(methoxycarbonyl)ethyl and so on), C₃₋₇alkenyloxycarbonylC₁₋₄alkyl group (for example, allyloxycarbonylmethyl, 1-buten-3-yloxycarbonylmethyl, 3-buten-1-yloxycarbonylmethyl and so on), C₃₋₇alkynyloxycarbonylC₁₋₄alkyl group (for example, propargyloxycarbonylmethyl, 2-butyn-1-yloxycarbonylmethyl, 3-butyn-2-yloxycarbonylmethyl and so on), C₄₋₇cycloalkoxycarbonylC₁₋₄alkyl group (for example, cyclopropyloxycarbonylmethyl, cyclohexyloxycarbonylmethyl, 1-(cyclopropyloxycarbonyl)-ethyl and so on), C₂₋₇haloalkoxycarbonylC₁₋₄alkyl group (for example, chloromethoxycarbonylmethyl, 2-chloroethoxycarbonylmethyl, 2-(chloromethoxycarbonyl)ethyl and so on), C₄₋₇haloalkenyloxycarbonylC₁₋₄alkyl group (for example, 2-chloro-2-propenyl-1-ylcarbonylmethyl and so on), or C₇₋₁₂aralkyloxycarbonylC₁₋₄alkyl group (for example, benzyloxycarbonylmethyl, 2-(benzyloxycarbonyl)ethyl, phenethyloxycarbonylmethyl and so on).

R¹² is hydrogen, C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl and so on), C₁₋₆alkoxyC₁₋₄alkyl group (for example, methoxymethyl, ethoxymethyl, 2-methoxyethyl, 2-ethoxyethyl and so on), C₁₋₇alkanoyl group (for example, formyl, acetyl, propionyl, butyryl, isobutyryl, pentanoyl, hexanoyl and so on), C₇₋₁₂arylcarbonyl group (for example, benzoyl, naphtalenecarbonyl and so on), C₂₋₇alkoxycarbonyl group (for example, methoxycarbonyl, ethoxycarbonyl, n-propoxycarbonyl, isopropoxycarbonyl and so on), C₂₋₇haloalkoxycarbonyl group (for example, chloromethoxycarbonyl, 2-chloroethoxycarbonyl and so on), or C₃₋₇haloalkenylcarbonyl group(for example, 2-chloro-2-propenyl-1-ylcarbonyl and so on).

R¹³ is hydrogen, halogen (for example, fluorine, chlorine, bromine, iodine and so on), or C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl and so on).

R¹⁴ and R¹⁵ are same or different, and are hydrogen, C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl and so on), C₂₋₆alkenyl group (for example, allyl, 1-buten-3-yl, 3-buten-1-yl and so on), C₂₋₆alkynyl group (for example, propargyl, 2-butyn-1-yl, 3-butyn-2-yl and so on), C₃₋₆cycloalkyl group (for example, cyclopropyl, cyclobutyl, cyclopentyl, cyclohexyl and so on), C₁₋₆alkoxyC₁₋₄alkyl group (for example, methoxymethyl, ethoxymethyl, 2-methoxyethyl, 2-ethoxyethyl and so on), C₆₋₁₂aralkyl group (for example, benzyl, phenethyl and so on), C₁₋₄alkyl group which may be substituted by 5-6 membered hetero ring including nitrogen, oxygen or sulfur (for example, 4-pyridylmethyl, 2-furylmethyl, 2-thiophenemethyl and so on), C₁₋₇alkanoyl group (for example, formyl, acetyl, propionyl, butyryl, isobutyryl, pentanoyl, hexanoyl and so on), C₆₋₁₂arylcarbonyl group(for example, benzoyl, naphthalenecarbonyl and so on), C₂₋₇haloalkylcarbonyl group (for example, chloroacetyl, trifluoroacetyl and so on), C₂₋₇alkoxycarbonyl group (for example, methoxycarbonyl, ethoxycarbonyl, n-propoxycarbonyl, isopropoxycarbonyl and so on), C₃₋₇alkenyloxycarbonyl group (for example, allyloxycarbonyl, 1-buten-3-yloxycarbonyl, 3-buten-1-yloxycarbonyl and so on), C₃₋₇alkynyloxycarbonyl group (for example, propargyloxycarbonyl, 2-butyn-1-yloxycarbonyl, 3-butyn-2-yloxycarbonyl and so on), C₄₋₇cycloalkyloxycarbonyl group (for example, cyclopropyloxycarbonyl, cyclobutyloxycarbonyl, cyclohexyloxycarbonyl and so on), C₂₋₇haloalkoxycarbonyl group (for example, chloromethoxycarbonyl, 1-chloroethyloxycarbonyl and so on), C₃₋₇haloalkenyloxycarbonyl group (for example, 2-chloro-2-propen-1-yloxycarbonyl and so on), C₁₋₆alkylsulfonyl group (for example, methylsulfonyl, ethylsulfonyl, n-propylsulfonyl, isopropylsulfonyl, n-butylsulfonyl, isobutylsulfonyl and so on), C₂₋₆alkenylsulfonyl group (for example, allylsulfonyl and so on), C₂₋₆alkynylsulfonyl group (for example, propargylsulfonyl and so on), C₃₋₆cycloalkylsulfonyl group (for example, cyclopropylsulfonyl, cyclobutylsulfonyl, cyclohexylsulfonyl and so on), C₂₋₇haloalkylsulfonyl group (for example, chloromethylsulfonyl and so on), C₆₋₁₀arylsulfonyl group (for example, phenylsulfonyl, naphthylsulfonyl and so on), C₇₋₁₂aralkylsulfonyl group (for example, benzylsulfonyl, phenethylsulfonyl and so on), or a group represented by the formula 10.

R⁵ is hydrogen or C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl and so on), m is 0 or 1.

R⁶ is hydrogen, C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl and so on), C₁₋₆haloalkyl group (for example, chloromethyl, chloroethyl and so on), C₂₋₆alkenyl group (for example, allyl, 1-buten-3-yl, 3-buten-1-yl and so on), C₂₋₆alkynyl group (for example, propargyl, 2-butyn-1-yl, 3-butyn-2-yl and so on), C₁₋₆alkoxyC₁₋₄alkyl group (for example, methoxymethyl, ethoxymethyl, 2-methoxyethyl, 2-ethoxyethyl and so on), C₁₋₇alkanoyl group (for example, formyl, acetyl, propionyl, butyryl, isobutyryl, pentanoyl, hexanoyl and so on), C₆₋₁₂arylcarbonyl group (for example, benzoyl, naphthalenecarbonyl and so on), C₂₋₇alkoxycarbonyl group (for example, methoxycarbonyl, ethoxycarbonyl, n-propoxycarbonyl, isopropoxycarbonyl and so on), C₂₋₇haloalkoxycarbonyl group (for example, chloromethoxycarbonyl, 1-chloroethoxycarbonyl and so on), C₃₋₇alkenyloxycarbonyl group (for example, allyloxycarbonyl, 1-buten-3-yloxycarbonyl, 3-buten-1-yloxycarbonyl and so on), C₃₋₇haloalkenyloxycarbonyl group (for example, 2-chloro-2-propen-1-yloxycarbonyl and so on), C₃₋₇alkynyloxycarbonyl group (for example, propargyloxycarbonyl, 2-butyn-1-yloxycarbonyl, 3-butyn-2-yloxycarbonyl and so on), C₄₋₇cycloalkyloxycarbonyl group (for example, cyclopropyloxycarbonyl, cyclopentyloxycarbonyl, cyclohexyloxycarbonyl and so on), C₂₋₇alkoxycarbonylC₁₋₄alkyl group (for example, methoxycarbonylmethyl, 1-(methoxycarbonyl)ethyl, ethoxycarbonylmethyl, n-propoxycarbonylmethyl, isopropoxycarbonylmethyl and so on), C₂₋₇haloalkoxycarbonylC₁₋₄alkyl group (for example, chloromethoxycarbonylmethyl, 1-chloroethoxycarbonylmethyl and so on), C₃₋₇alkenyloxycarbonylC₁₋₄alkyl group (for example, allyloxycarbonylmethyl, 1-(allyloxycarbonyl)ethyl, 1-buten-3-yloxycarbonylmethyl, 3-buten-1-yloxycarbonylmethyl and so on), C₃₋₇alkenyloxycarbonylC₁₋₄alkyl group (for example, 2-chloro-2-propen-1-yloxycarbonylmethyl and so on), or C₃₋₇alkynyloxycarbonylC₁₋₄alkyl group (for example, 2-butyn-1-yloxycarbonylmethyl, 1-(2-butyn-1-yloxycarbonyl)ethyl, 3-butyn-2-yloxycarbonylmethyl and so on).

R⁷ is hydrogen, halogen (for example, fluorine, chlorine, bromine, iodine), C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl and so on), C₁₋₆alkoxy group (for example, methoxy, ethoxy, n-propyloxy, isopropyloxy, n-butyloxy, isobutyloxy, sec-butyloxy, tert-butyloxy, n-pentyloxy, sec-pentyloxy, isopentyloxy, neopentyloxy, n-hexyloxy, isohexyloxy and so on), C₁₋₆haloalkoxy group (for example, trifluoromethoxy and so on), C₂₋₆alkenyl group (for example, allyl, 1-buten-3-yl, 3-buten-1-yl and so on), C₂₋₆alkynyl group (for example, propargyl, 2-butyn-1-yl, 3-butyn-2-yl and so on), C₁₋₆alkylthio group (straight or branched alkylthio group, for example, methylthio, ethylthio, n-propylthio, isopropylthio, n-butylthio, isobutylthio, sec-butylthio, tert-butylthio, n-pentylthio, sec-pentylthio, isopentylthio, neopentylthio, n-hexylthio, isohexylthio and so on), C₂₋₆alkenylthio group (for example, allylthio, 1-buten-3-ylthio, 3-buten-1-ylthio and so on), C₂₋₆alkynylthio group (for example, propargylthio, 2-butyn-1-ylthio, 3-butyn-2-ylthio and so on), C₁₋₆haloalkyl group (for example, chloromethyl, bromomethyl and so on), C₁₋₆alkoxyC₁₋₄alkyl group (for example, methoxymethyl, ethoxymethyl and so on), C₁₋₆alkylthioC₁₋₄alkyl group (for example, methylthiomethyl, ethylthiomethyl and so on), or C₁₋₆alkylsulfonylC₁₋₄alkyl group (for example, methanesulfonylmethyl, ethanesulfonylmethyl and so on).

R⁸ is hydrogen or C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl and so on).

R⁹ is C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl and so on).

Z is CH or nitrogen, Z¹ is oxygen, sulfur or methylene.

The compound represented by the formula (I) (hereinafter, the compound may be described the compound (I) for short) comprises any compound obtained by combining the groups selected optionally from each symbol described above, the compound described below being especially appropriate.
(1) As the preferable substituent on the group represented by J-1 to J-5 in the formula (I), R¹ is hydrogen, halogen, C₁₋₃alkyl group, or especially preferable is hydrogen, chlorine, bromine and methyl group. As the preferable substituent on the group represented by J-6 to J-8, R¹ is hydrogen or C₁₋₃alkyl group, especially preferable is hydrogen or methyl group.
(2) As the preferable substituent on the group represented by Ar-1 in the formula (I), when Z is CH, R² is hydrogen or halogen, especially preferable is fluorine or chlorine, R³ is halogen, cyano group or nitro group, especially preferable is chlorine, cyano group, nitro group, and so on, R⁴ is C₁₋₄alkoxy group, C₂₋₅alkenyloxy group, C₂₋₅alkynyloxy group, C₁₋₄alkanesulfonylamino group which may have substituent on its nitrogen, C₁₋₆alkoxycarbonylC₁₋₄alkoxy group, C₁₋₆alkoxycarbonylC₁₋₄alkylthio group, especially preferably isopropoxy, isobutoxy, allyloxy, propargyloxy, 3-buten-2-yloxy, methanesulfonylamino, ethanesulfonylamino, isopropylsulfonylamino, methoxycarbonylmethoxy, ethoxycarbonylmethoxy, 1-(methoxycarbonyl)ethoxy, methoxycarbonylmethylthio.
   When Z is nitrogen, R² is hydrogen or halogen, especially preferably, fluorine or chlorine, R³ is halogen or cyano group, especially preferable is cyano group and so on, R⁴ is C₁₋₄alkoxy group, C₂₋₅alkenyloxy group, C₂₋₅alkynyloxy group, C₂₋₄alkoxycarbonylC₁₋₄alkoxy group, especially preferable is isopropoxy, isobutoxy, allyloxy, propargyloxy, 3-buten-2-oxy, methoxycarbonylmethoxy and so on.
(3) As the preferable substituent on the group represented by Ar-2 in the formula (I), R² is hydrogen or halogen, especially preferable is fluorine or chlorine, R⁵ is hydrogen or C₁₋₃alkyl group, especially preferable is methyl or ethyl and then m is 1 preferable, R⁶ is C₁₋₃alkyl group, C₂₋₅alkenyl group, C₂₋₅alkynyl group, C₁₋₃alkoxyC₁₋₃alkyl group, especially preferable is ethyl, n-propyl, propargyl, ethoxymethoxy, Z¹ is oxygen, sulfur or methylene.
(4) As the preferable substituent on the group represented by Ar-3 in the formula (I), R² is hydrogen or halogen, especially preferable is fluorine or chlorine, R³ is halogen, cyano group or nitro group, and especially preferable is chlorine, cyano group or nitro group, R⁷ is C₁₋₃alkyl group, C₁₋₃haloalkyl group, C₁₋₃alkoxyC₁₋₃alkyl group, especially preferable is methyl, chloromethyl, methoxymethyl and so on, Z¹ is oxygen, sulfur or methylene.
(5) As the preferable substituent on the group represented by Ar-4 in the formula (I), R² is hydrogen or halogen, especially preferable is fluorine or chlorine, R³ is halogen, cyano group or nitro group, especially preferable is chlorine, cyano group or nitro group, R⁸ and R⁹ are same or different, and are C₁₋₄alkyl group, especially preferable is methyl.
(6) As the preferable substituent on the group represented by Ar-5 in the formula (I), R² is hydrogen or halogen, especially preferable is fluorine or chlorine, R⁷ is C₁₋₃alkyl group, C₂₋₅alkenyl group, C₂₋₅alkynyl group, C₁₋₃alkoxy group, C₁₋₃alkylthio group, especially preferable is ethyl, n-propyl, propargyl, methoxy, ethoxy, methylthio and so on.
(7) As the preferable substituent on the group represented by Ar-6 in the formula (I), R² is hydrogen or halogen, especially preferable is fluorine or chlorine, R³ is halogen, cyano group or nitro group, especially preferable is chlorine, cyano group, nitro group, R⁸ is C₁₋₃alkyl group, C₃₋₅alkenyl group, C₃₋₅alkynyl group, C₁₋₃alkoxyC₁₋₃alkyl group, especially preferable is methyl, ethyl, n-propyl or propargyl.

The acid group such as sulfo group, carboxyl group and so on of the compound (I) of this invention can react with inorganic base or organic base to produce basic salts agrochemically acceptable, while, the basic group such as nitrogen in molecule, amino group and so on of the compound of this invention can react inorganic acid or organic acid to produce acid addition salts agrochemically acceptable. As inorganic basic salts, the salts mentioned bellow can be used; salts with alkali metals (for example, sodium, potassium and so on), alkaline earth metals (for example, calcium and so on), ammonia and so on. And, as organic basic salts, the salts mentioned bellow can be used; salts with for example, dimethylamine, triethylamine, N,N-dimethylaniline, piperazine, pyrrolidine, piperidine, pyridine, 2-phenylethylamine, benzylamine, ethanolamine, diethanolamine, 1,8-diazabicyclo[5,4,0]-7-undecene (hereafter, mentioned as DBU for short) and so on. As the inorganic acids addition salts of the compound (I), the salts mentioned bellow can be used; the salts with, for example, hydrochloric acid, hydrobromic acid, hydroiodic acid, sulfuric acid, nitric acid, phosphoric acid, perchloric acid and so on. As organic acids addition salts of the compound (I), the salts mentioned bellow can be used; the salts with, for example, formic acid, acetic acid, propionic acid, oxalic acid, succinic acid, benzoic acid, p-toluenesulfonic acid, methanesulfonic acid, trifluoroacetic acid and so on.

The compound (I) of this invention or a salt thereof can be used as agrochemicals such as herbicide, which is excellent in safety. The compound of this invention or a salt thereof is useful especially as herbicide, and even with a low dose, it has a very strong weeding action to an extreamly wide variety of weeds, for example, weed in paddy field such as early watergrass, smallflower umbrella sedge, ducksalad, needle spikerush, arrowhead, common falsepimpernel, Indian toothcup and so on, weed in field such as southern crabgrass, green foxtail, slender amaranth, velvetleaf, goosefoot, tufted knotweed, common purslane, jimsonweed, tall morningglory, common cocklebur, fall panicum, johnsongrass, purple nutsedge, wild oat, downy brome, common chickweed, Indian mustard, sicklepod, wild chamomile, Asiatic dayflower and so on. Moreover, it has little toxicity to crop such as rice, wheat, barley, corn, cotton and so on, and shows high safety. The compound (I) or a salt thereof shows excellent selective weeding effect between crops and various kinds of weeds, has a low toxicity to mammals and fishes, does not pollute environment and can be useful as herbicide for paddy field, field, fruit ranch or non-agricultural land with safe.

When a compound of the present invention or a salt thereof mentioned above is used as herbicidal compositions, they can be applied in the per se known forms for general use of agrochemical compositions. Namely, depending on the objects, one or more than two kinds of compound or salts thereof are taken as the effective constituents and dissolved or dispersed in some appropriate liquid carrier, or mixed with or adsorbed on some appropriate solid carrier to get various forms of compositions, for example, emulsions, oils, prays, wettable powder, powders, DL (driftless) powders, granules, fine granules, fine granules F, flowable preparations, dry-flowable preparations, Jumbo preparations, tablets and so on. These preparations may be further admixtured with emulsifiers, dispersing agents, spreading agents, permeating agents, moistening agents, mucilage, stabilizers to prepare liquid compositions of the present invention by the per se known methods. As liquid carriers (solvents) appropriate for use, there may be mentioned, for example, water, alcohols (for example, methanol, ethanol, 1-propanol,2-propanol, ethylene glycol and so on), ketones (for example, acetone,methyl ethyl ketone and so on), ethers (for example, dioxane, tetrahydrofuran, ethylene glycol monomethyl ether, diethylene glycol monomethyl ether, propylene glycol monomethyl ether and so on), aliphatic hydrocarbons (for example, kerosene, fueloil, machine oil and so on), aromatic hydrocarbons (for example, benzene, toluene, xylene, solvent naphtha, methylnaphthalene and so on), halogenated hydrocarbons (for example, dichloromethane, chloroform, carbon tetrachloride and so on), acid amides (for example, N,N-dimethylformamide, N,N-dimethylacetamide and so on), esters (for example, ethyl acetate, butyl acetate, fatty acid glycerol esters and so on), and nitriles (for example, acetonitrile, propionitrile and so on). These solvents can be used by mixing one or more than two of them in appropriate ratios. As solid carriers (diluents, fillers), there may be mentioned, for example, vegetable powders (for example, soybean powder, tabacco powder, wheat flour, wood flour, and so on), mineral powder (for example, clays such as kaolin, bentonite, acid clay, clay and so on, talcs such as talcum powder, agalmatolite powder, and so on, silicate minerals such as diatomaceous earth, mica, and so on, alumina, sulfur powder, activated carbon, and so on. These fillers can be used by mixing one or more than two of them in appropriate ratios. The liquid carrier or solid carrier can be used about 1 to 99 wt% or so, or preferably about 1 to 80 wt% or so against whole composition.

As surfactants which may be used as emulsifiers, spreading agents, penetrants, dispersing agents, there may be mentioned nonionic or anionic surfactants such as soaps, polyoxyethylene alkylethers (for example, NOIGEN™, E A -142 (E A142™, TM means trade mark.), and so on; Dai-ichi Kogyo Seiyaku Co., Ltd.,), polyoxyethylene alkylaryl esters (for example, NONAL™; manufactured by Toho Chemical Industry Co., Ltd.), alkylsulfuric acid salts (for example, EMAL10™,EMAL40™ ; manufactured by KAO Corp.), alkylsulfonic acid salts such as (for example, NEOGEN™,NEOGEN T™ manufactured by Dai-ichi Kogyo Seiyaku Co.,Ltd.,NEOPEREX; manufactured by KAO Corp.), polyethylene glycol ethers (for example, NONIPOLE 85™, NONIPOLE 100™, NONIPOLE 160™; manufactured by SANYO KASEI CO., Ltd.), polyol esters (for example, Tween 20™, Tween 80™; manufactured by KAO Corp.). It is preferable to add the said surfactants about 0.1 to 50% or so, or more preferably about 0.1 to 25% or so to the total amount of the compositions. In the cases where emulsions, wettable powder or so are to be prepared, the appropriate content ratio of the compound of the present invention or the salts thereof in herbicide is about 1 to 90 wt% or so. In the cases where oils, powders, DL (driftless) or so are to be prepared, it is preferable to add about 0.01 to 10 wt% to the total amount of the compositions. In the cases where fine granules F, granules or so are to be prepared, it is preferable to add about 0.05 to 10 wt% to the total amount of the comopositions, and the concentration described above can be varied depending on the objective. It is preferable to dilute emulsions or wettable powders and so on to an appropriate volume (for example, to about 100 to 100,000 times of volume), for example, with water and so on before use, and to spray it (them).

The amount used may be, in general, about 0.01g to 50g, or more preferably about 0.02g to 10g effective constituents per 1 are of paddy fiels, or about 0.01g to 50g, or more preferably about 0.02 to 10g effective constituents (compound (I) or salts thereof) per 1 are of field, although it can be altered within a wide range according to the place, time, method of applying, target weed and crops and so on. For weed in field, it is preferable to use the compound (I) or salts thereof as soil treating agent before emergence or soil treating agent for leaf and stem. For example, the herbicide of the present invention can be used even 2 or 3 weeks later without exhibiting phytotoxicity.

The herbicide containing the compound (I) of the present invention or a salt can be used, in necessary, in conbination with one or two (preferably one to three) kinds of other agrochemicals, for example, herbicides, plant growth regulating agents, microbicides, insecticides, acaricides, nematicides and so on.

As the other herbicides (herbicidal active components), there may be mentioned, for example, (1) sulfonyl urea herbicides (chlorsulfuron, sulfometuron-methyl, chlorimuron-ethyl, triasulfuron, amidosulfuron, oxasulfuron, tribenuron-methyl, prosulfuron, ethametsulfuron-methyl, triflusulfuron-methyl, thifensulfuron-methyl, flazasulfuron, rimsulfuron, nicosulfuron, flupyrsulfuron, bensulfuron-methyl, pyrazosulfuron-ethyl, imazosulfuron, sulfosulfuron, cinosulfuron, azimsulfuron, metsulfuron-methyl, halosulfuron-methyl ethoxysulfuron, cyclosulfamuron, and so on), (2) pyrazole herbicides (pyraflufen-ethyl, pyrazolate, pyrazoxyfen, benzofenap and so on), (3) carbamate herbicides (di-allate, butylate, tri-allate, phenmedipham, chlorpropham, asulam, phenisopham, benthiocarb, molinate, esprocarb, pyributicarb, dimepiperate, swep and so on), (4) chloroacetanilide herbicides (propachlor, metazachlor, alachlor, acetochlor, metolachlor, butachlor, pretilachlor, thenylchlor and so on), (5)diphenylether herbicide (acifluorfen, oxyfluorfen, lactofen, fomesafen, aclonifen, chlomethoxynil, bifenox, CNP and so on), (6) triazine herbicides (simazine, atrazine, propazine, cyanazine, ametoryn, simetryn, dimethametryn, prometryn and so on), (7) phenoxy acid or benzoic acid herbicides (2,3,6-TBA, dicamba, quinclorac, quinmerac, clopyralid, picloram, triclopyr, fluroxypyr, benazolin, diclofop-methyl, fluazifop-butyl, haloxyfop-methyl, quizalofop-ethyl, cyhalohop-butyl, 2,4-PA, MCP, MCPB, phenothiol and so on), (8) acid amide or urea herbicides (isoxaben, diflufenican, diuron, linuron, fluometuron, difenoxuron, methyl-daimuron, isoproturon, isouron, tebuthiuron, methabenzthiazuron, propanil, mefenacet, clomeprop, naproanilide, bromobutide, daimuron, cumyluron, etobenzanid, 3-(1-(3,5-dichlorophenyl)-1-methylethyl)-2,3-dihydro-6-methyl-5-phenyl-4H-1,3-oxazin-4-one) and so on), (9) organic phospholic herbicides (glyphosate, bialaphos, amiprofos-methyl, anilofos, bensulide, piperophos, butamifos, anilofos and so on), (10) dinitroaniline herbicides (bromoxynil, ioxynil, dinoseb, trifluralin, prodiamine and so on), (11) cyclohexanedion herbicides (alloxydim, sethoxydim, cloproxydim, clethodim, cycloxydim, tralkoxydim and so on), (12) imidazoline herbicides (imazamethabenz, imazapyr, imazamethapyr, imazethapyr, imazamox, imazaquin and so on), (13) bipyridium herbicides (paraquat, diquat and so on), (14) other herbicides (bentazon, tridiphane, indanofan, amitrole, carfentrazon-ethyl, sulfentrazon, fenchlorazole-ethyl, fentrazamide, isoxaflutole, clomazone, maleic hydrazide, pyridate, chloridazon, norflurazon, pyrithiobac, bromacil, terbacil, metribuzin, oxaziclomefone, cinmethylin, flumiclorac-pentyl, cinidon-ethyl, flumioxazin, fluthiacet-methyl, azafenidin, benfuresate, oxadiazon, oxadiargyl, pentoxazone cyhalofop-butyl, cafenstrole, pyriminobac-methyl, bispyribac-sodium, pyribenzoxim, 7-(4,6-dimethoxypyrimidin-2-ylthio)-3-methylphthalid e,1-(2-chlorophenyl)-4-(N-cyclohexyl-N-ethylcarbamoy 1)-5(4H)-tetrazolinone, 2-(2-(3-chlorophenyl)-2,3-epoxypropyl)-2-ethylindane -1,3-dione), ACN, 3-(2-chloro-4-methylsulfonylbenzoyl)-4-phenylthiobic yclo[3.2.1]oct-3-en-2-one, dithiopyr, dalapon, chlorthiamid and so on)and so on.

As plant growth regulating agents (plant growth regulating active components), there may be mentioned, for example, hymexazol, paclobutrazol, uniconazole-P, inabenfide, prohexadione-calcium and so on. As fungicides (fungicidal active components), there may be mentioned, for example, (1) polyhaloalkylthio fungicides (captan and so on), (2) organophospate fungicides (IBP, EDDP, tolclofos-methyl and so on), (3) benzimidazol fungicides (benomyl, carbendazim, thiophanate-methyl and so on), (4) carboxyamide fungicides (mepronil, flutolanil, thifluzamid, furametpyr, teclofthalam, pencycuron, carpropamid, diclocymet and so on), (5) acylalanine fungicides (metalaxyl and so on), (6) azole fungicides (triflumizole, ipconazole, pefurazoate, prochloraz and so on), (7) methoxyacryl fungicides (azoxystrobin, metominostrobin and so on), (8) antibiotic fungicides (validamycin A, blasticidin S, kasugamycin, polyoxin and so on), (9) other fungicides (fthalide, probenazole, isoprothiolane, tricyclazole, pyroquiln, ferimzone, acibenzolar S-methyl, diclomezine, oxolinic acid, phenazine oxide, TPN, iprodione and so on) and so on. As insecticide (insecticidal active components), there may be mentioned, for example, (1) organic phosphate insecticides (fenthion, fenitrothion, pirimiphos-methyl, diazinon, quinalphos, isoxathion, Pyridafenthion, chlorpyrifos-methyl, vamidothion, malathion, phenthoate, dimethoate, disulfoton, monocrotophos, tetrachlorvinphos, chlorfenvinphos, propaphos, acephate, trichlorphon, EPN, pyraclofos and so on), (2) carbamate insecticides (carbaryl, metolcarb, isoprocarb, BPMC, propoxur, XMC, carbofuran, carbosulfan, benfuracarb, furathiocarb, methomyl, thiodicarb and so on), (3) synthetic pyrethroide insecticides (cycloprothrin, ethofenprox and so on), (4) Neristoxin insecticides (cartap, bensultap, thiocyclam and so on), (5) neonicotinoide insecticides (imidacloprid, nitenpyram, acetamiprid, thiamethoxam), 3-(6-chloro-3-pyridylmethyl)-1,3-thiazolidin-2-ylide necyanamide, 1-methy-2-nitro-3-(tetrahydrofuran-3-ylmethyl)guanid ine, (E)-1-(2-chloro-1,3-thiazol-5-ylmethyl)-3-methy-2-ni troguanidine and so on), (6) other insecticides (buprofezin, tebufenozide, fipronil and so on) and so on.

As acaricide (acaricidal active components), there may be mentioned, for example, hexythiazox, pyridaben, fenpyroximate, tebufenpyrad, chlorfenapyr, etoxazole, Pyrimidifen and so on. As nematicide (nematicidal active components), there may be mentioned, for example, fosthiazate and so on. The other agrochemical active components (for example, herbicidal active components, plant growth regulating active components, fungicidal active components, insecticidal active components, acaricidal active components, nematicidal active components and so on) can be used about 0.1 to 20 wt% or so, or preferably about 0.1 to 10 wt% or so to the total amount of the compositons. Moreover, the herbicide containing the compound (I) of the present or a salt thereof can be, in necessary, mixed with a synergist (for example, piperonyl butoxide and so on), an attractant (for example, eugenol and so on), a repellent (for example, creosote and so on), a colloring agent (for example, edible blue No.1 and so on), fertilizers (for example, urea and so on) and so on.

Although the compound (I) of the present invention or the salt thereof is a new compound, they can be manufactured by any per se known methods. The compound (I) of the present invention or the salt thereof can be manufacutured by the manufacturing method 1 to 16 described hereinafter, however, the manufacturing method will not be limited to them. 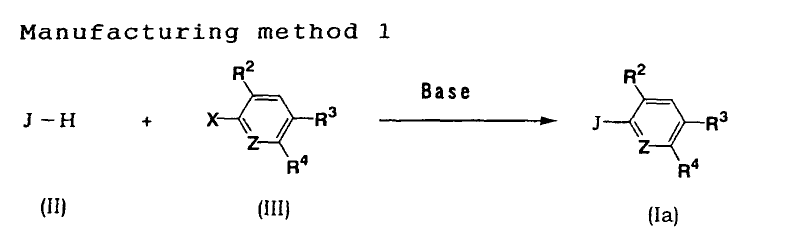 [Wherein each of J, R², R³, R⁴ and Z has the same meaning as mentioned above, X is halogen.]

In this reaction, the compound (II) is used usually about 0.8 to 3 times of mol, or preferably about 0.9 to 1.3 times of mol to the amount of the compound (III). The present reaction is carried out under solvent, which does not influence on the reaction. As the preferable solvent, there can be mentioned, for example, aromatic hydrocarbon such as benzene, toluene, and so on, halogenated hydrocarbons such as chloroform, carbon tetrachloride, dichloromethane, and so on, ethers such as diethyl ether, diisopropyl ether, dioxane, tetrahydrofuran (THF), and so on, ketones such as acetone, methyl ethyl ketone, and so on, nitriles such as acetonitrile, and so on, aliphatic amides such as dimethylformamide (DMF), dimethylacetamide, N-methylpyrrolidone, and so on, sulfoxides such as dimethylsulfoxide (DMSO), and so on, phosphoric amides suc as hexamethylphosphoric triamide (HMPA), and so on, sulfones such as sulfolan, and so on. These solvents can be used mixed or singly. As the base employable, there can be mentioned, for example, organic base such as triethylamine, tri-n-propylamine, pyridine, dimethylaniline, dimethylaminopyridine, 1,8-diazabicyclo[5,4,0]-7-undecene (DBU), 1,4-diazabicyclo[2,2,2]octane (DBO), and so on, inorganic base such as alkali metal hydroxides such as sodium hydroxide, potassium hydroxide, and so on, alkaline-earth metal hydroxides such as calcium hydroxide, and so on, alkali metal hydrogencarbonates such as sodium hydrogencarbonate, and so on, alkaline-earth metal carbonates such as calcium carbonate, and so on, metal hydroxides such as potassium hydroxide, sodium hydroxide, and so on, potassium fluoride, and so on. The amount of the base is about 0.7 to 4.0 equivalents, or preferably about 0.9 to 2.0 equivalents to the compound (II). The reactions can be carried out at temperatures of about -20 to 250 °C, or more preferably at about -10 to 180 °C. And the reaction time is varied depending on the reaction temperature, usually from about 10 minutes to 14 hours, or more preferably from about 30 minutes to 8 hours. The reaction can be confirmed by thin-layer chromatography or high-speed liquid chromatography. 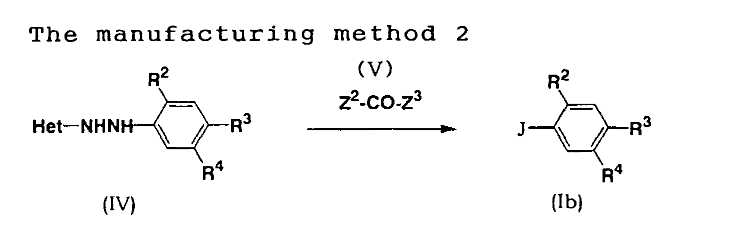 [Wherein each of J, R², R³ and R⁴ has the same meaning as described above, Het is 2-pyridyl, pyridazine-3-yl, pyrimidine-2-yl, pyrimidine-4-yl, thiozole-2-yl, thiazoline-2-yl, 5,6-dihydro-1,3-thiazine-2-yl, or 3,4,5,6-tetrahydropyrimidine-2-yl. Z² and Z³ are the same or different, are halogen, C₁₋₂alkoxy, phenoxy and so on.]

The compound (IV) can be manufactured by the known method (for example, Pharmazie, 48, (1993), H.6; J.Org. Chem., 47(3), 552(1982); J.Org.Chem., 57(2), 607(1992) and so on). In this reaction, phosgene (or diphosgene or triphosgene) represented by Z²-CO-Z³ (V), dimethyl carbonate, diethyl carbonate, diphenyl carbonate, chloro methyl formate, and so on are used usually about 0.4 to 20 times mol, or preferably about 0.6 to 10 times mol to the amount of the compound (IV). This reaction can be carried out in a solvent, which does not influence the reaction, and as a solvent, the same solvent, as shown in the manufacturing method 1 can be used. As the preferable base used in this reaction, the same base as described in the manufacturing method 1 can be used. The amount of the base is about 0.8 to 10 equivalents, or preferably about 1.0 to 5.0 equivalents to the compound (IV). The reaction temperature depends on the solvent or base, but is generally from about -10 to 150 °C, or preferably from about 10 to 100 °C. The reaction time varies depending on the reaction temperature, and is from about 10 minutes to 14 hours, or preferably from about 30 minutes to 8 hours. The reaction can be confirmed by thin-layer chromatography or high-speed chromatography. 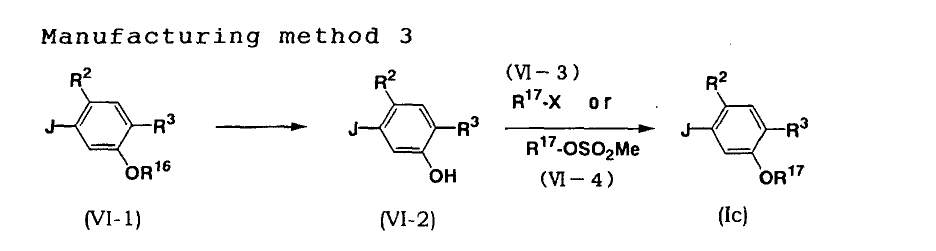 [Wherein each of J, R², R³ and X has the same meaning as described above, R¹⁶ is C₁₋₄alkyl group, R¹⁷ is a C₁₋₆alkyl group, a C₃₋₆alkenyl group, a C₃₋₆alkynyl group, a C₃₋₆cycloalkyl group, a C₁₋₆haloalkyl group, a C₃₋₇haloalkyl group, a C₇₋₁₂aralkyl group, C₆₋₁₀aryl group, a C₁₋₆alkoxyC₁₋₄alkyl group or the group represented by the formula; 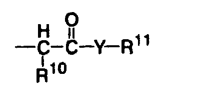 (wherein each of R¹⁰, R¹¹ and Y has the same meaning described above.).]

The compound (VI-I) can be manufactured by the manufacturing method 2. In this reaction, the compound (VI-I) is deprotected in hydrobromic acid, hydroiodic acid or acetic acid medium thereof. In the reaction, hydrobromic acid or hydroiodic acid is used usually from about 5 to 50 times mol, or preferably from about 10 to 30 times mol to the amount of compound (VI-I). The reaction temperature is generally from about 10 to 180 °C, or preferably from about 50 to 150 °C. The reaction time is different depending on the reaction temperature, is from about 10 minutes to 24 hours, or preferably from about 1 hour to 12 hours. Also, in this reaction, the compound (VI-I) can be deprotected by reacting with Lewis acid. As Lewis acid, boron tribromide, aluminium chloride, and so on can be used, and Lewis acid is used usually from about 1 to 10 times mol, or preferably from about 2 to 5 times mol to the amount of the compound (VI-I). This reaction can be carried out in the solvent, which does not influence the reaction. As the solvent, there may be mentioned, for example, aromatic hydrocarbons such as benzene, toluene, and so on, halogenated hydrocarbons such as chloroform, carbon tetrachloride, dichloromethane, and so on, ethers such as diethylether, diisopropylether, dioxane, tetrahydrofuran (THF) and so on. These solvents can be used by mixing two or more in appropriate ratio. The reaction temperature is generally about -10 to 150 °C, or preferably about 10 to 120 °C. The reaction time varies depending on the reaction temperature, but is about 10 minutes to 24 hours, or preferably about 1 hour to 12 hours. In order to produce the compound (Ic), the compound (VI-3) or the compound (VI-4) is used about 0.8 to 5 times mol, or preferably about 0.9 to 2.0 times mol to the amount of the compound (VI-2). This reaction can be carried out in a solvent, which does not influence the reaction. As the solvent, the same solvent showing the same reaction in the manufacturing method 1 can be used. As the preferable base of this reaction, the same base showing the same reaction in the manufacturing method 1 can be used. The amount of the base is about 0.8 to 4.0 equivalents, or preferably about 1.0 to 1.5 equivalents to the amount of the compound (VI-2). The reaction temperature varies depending on the solvent or the base, but is generally about -20 to 100 °C, or preferably about 0 to 50 °C. The reaction time varies depending on the reaction temperature, but is about 10 minutes to 10 hours, or preferably about 30 minutes to 3 hours. The reaction can be confirmed by thin-layer chromatography, high-speed liquid chromatography and so on. 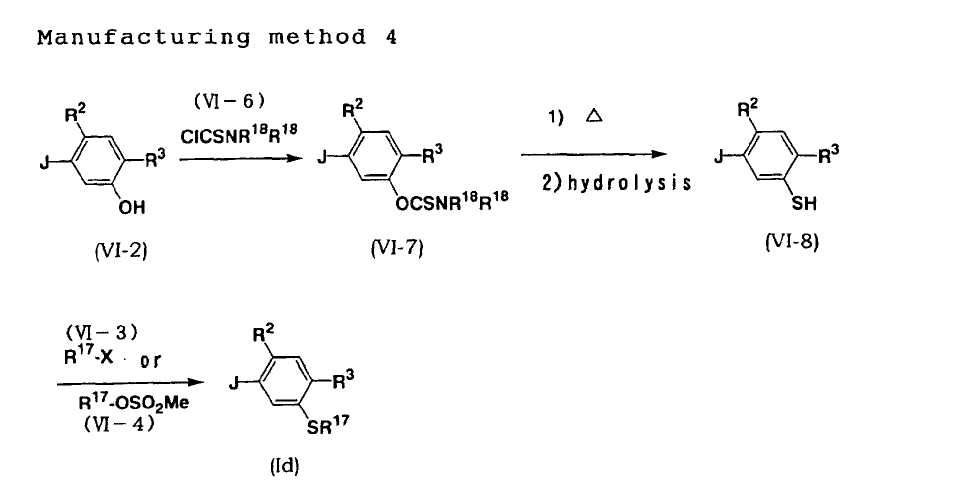 (wherein each of J, R²,R³,R¹⁷ and X has the same meaning as described above, R¹⁸ is a C₁₋₄alkyl group.)

The compound (VI-8) can be manufactured from the compound (VI-2) by the known method [for example, J.Org.Chem., 31, 3980 (1996) and so on]. In the reaction of producing the compound (VI-7) from the compound (VI-2), the compound (VI-6) is used usually from about 0.8 to 3.0 times mol, or preferably from about 0.9 to 1.5 times mol to the amount of compound (VI-2). This reaction can be carried out in a solvent, which does not influence the reaction. As the solvent, the same solvent described in the manufacturing method 1 can be used. As the preferable base in this reaction, the same base described in the manufacturing method 1 can be used. The amount of the base is from about 0.8 to 3 equivalents, or preferably from about 1.0 to 1.5 equivalents to the amount of the compound (VI-2). The reaction temperature is different depending on the solvent or base, is generally from about -20 to 150 °C, or preferably from about 0 to 100 °C. The reaction time is different depending on the reaction temperature, is from about 10 minutes to 10 hours, or preferably from about 30 minutes to 5 hours. Then, in the reaction for producing the compound (VI-8) from the compound (IV-7), the compound (IV-7) is heated without solvent, or in a solvent, which does not influence the reation. As the preferable solvent, there may be mentioned, for example, aromatic hydrocarobons such as benzene, toluene, p-dichlorobenzene, and so on, halogenated hydrocarbons such as chloroform, carbon tetrachloride, dichloromethane, and so on, ethers such as diethyl ether, diisopropyl ether, dioxane, tetrahydrofuran (THF), and so on, ketones such as acetone, methyl ethyl ketone, and so on, nitriles such as acetonitrile, and so on. The reaction temperature is different depending on the solvent or base, is generally from about 50 to 200 °C, or preferably from about 70 to 150 °C. The reaction time is different depending on the reaction temperature, is from about 10 minutes to 10 hours, or preferably from about 30 minutes to 5 hours. Then, mercapto compound (VI-8) can be obtained by hydrolysis reaction. As the condition of hydrolysis reaction, inorganic bases such as alkali metal hydroxides such as sodium hydroxide, potassium hydroxide, and so on, alkaline-earth metal hydroxides such as calcium hydroxide, and so on, alkali metal hydrogencarbonates such as sodium hydrogencarbonate, and so on, alkaline-earth metal carbonates such as calcium carbonate, and so on can be used. The amount of the base is from about 0.8 to 10 equivalents, or preferably from about 1.0 to 5 equivalents. The reaction temperature is different depending on the base, is generally from about -10 to 150 °C, or preferably from about 0 to 100 °C. The reaction time is different depending on the reaction temperature, but is from about 10 minutes to 5 hours, or preferably from about 30 minutes to 2 hours. To produce the compound (Id), the compound (VI-3) or the compound (VI-4) is used usually about 0.8 to 5 times mol, or preferably 0.9 to 2.0 times mol to the amount of the compound (VI-8). This reaction can be carried out in a solvent, which does not influence the reaction. The preferable base in this reaction are the same as described in the manufacturing method 1. The amount of the base is from about 0.8 to 4.0 equivalents, or preferably from about 1.0 to 1.5 equivalents to the amount of the compound (VI-8). The reaction temperature varies depending on the solvent or base, is usually from about -20 to 100 °C, or preferably from about 0 to 50 °C. The reaction time varies depending on the reaction temperature, is from about 10 minutes to 10 hours, or preferably from about 30 minutes to 3 hours. The reaction can be confirmed by thin-layer chromatography or high-speed liquid chromatography. 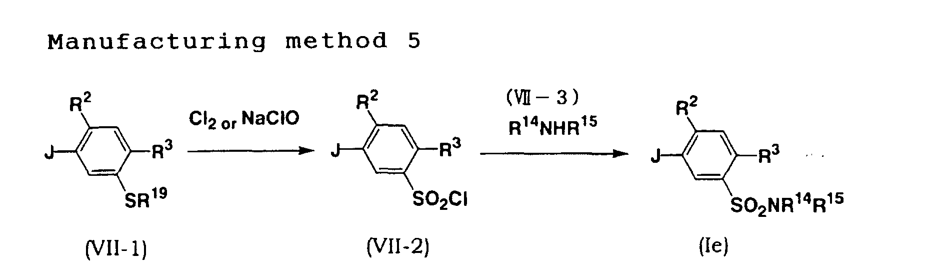 [Wherein each of J, R², R³, R¹⁴ and R¹⁵ has the same meaning described above, R¹⁹ is a benzyl group.]

In the reaction for producing the compound (VII-2) from the compound (VII-1), sulfonylchloride (VII-2) can be obtained by reacting the compound (VII-1) with the excessive amount, preferably from 5 to 10 times mol of chlorine gas or sodium hypochloride in the solvent, which does not influence the reaction. As the solvent, water, acetic acid, and halogenated hydrocarbons such as dichloromethane, chloroform, and so on can be used. These solvents can be mixed in appropriate ratio for use. The reaction temperature varies depending on the solvent, but is generally from about -50 to 60 °C, or preferably from about -20 to 30 °C. The reaction time varies depending on the reaction temperature, is from about 5 minutes to 2 hours, or preferably from about 10 minutes to 1 hour. Then, in order to produce the compound (Ie), the compound (VII-3) is used usually from about 0.8 to 3 times mol, or preferably from about 0.9 to 1.3 times mol to the amount of the compound (VII-2). The reaction can be carried out in a solvent, which does not influence the reaction. As the solvent, the same solvent as described in the manufacturing method 1 can be used. As the preferable base of this reaction, the same base described in the manufacturing method 1 can be used, or the compound (VII-3) can be used as base. The amount of the base is about 0.8 to 4.0 equivalents, or preferably about 1.0 to 1.5 equivalents to the amount of the compound (VII-3). In the cases where the compound (VII-3) is used as base, still more, it is necessary to add the compound (VII-3) about 1.0 to 1.5 equivalents. The reaction temperature varies depending on the solvent or base, but is generally about -20 to 100 °C, or preferably about 0 to 50 °C. The reaction time varies depending on the reaction temperature, but is about 10 minutes to 14 hours, or preferably about 30 minutes to 5 hours. The reaction can be confirmed by thin-layer chromatography or high-speed liquid chromatography and so on. 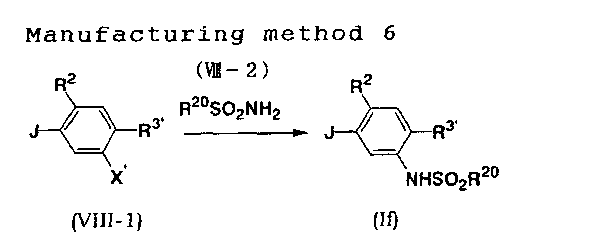 [Wherein each of J and R² has the same meaning described above, R³' is nitro group, cyano group, X' is fluorine, R²⁰ is a C₁₋₆alkyl group, a C₁₋₆haloalkyl group, a C₃₋₆alkenyl group, a C₃₋₆alkynyl group, C₆₋₁₀aryl group, or a C₇₋₁₂aralkyl group.]

The compound (VIII-1) can be manufactured by the manufacturing method 1. In this reaction, the compound (VIII-2) is used usually about 0.8 to 3 times mol, or preferably about 0.9 to 1.3 times mol to the amount of the compound (VIII-1). The reaction can be carried out in a solvent, which does not influence the reaction. As the solvent, the same solvent as described in the manufacturing method 1 is used. As the preferable base in this reaction, the same base as described in the manufacturing method 1, and so on is used. The amount of the base is about 0.8 to 4.0 equivalents, or preferably about 1.0 to 1.5 equivalents to the amount of the compound (VIII-1). The reaction temperature varies depending on the solvent or the base, but is generally about -20 to 200 °C, or preferably about 0 to 150 °C. The reaction time varies depending on the reaction temperature, but is about 10 minutes to 14 hours, or preferably about 30 minutes to 8 hours. The reaction can be confirmed by thin-layer chromatography or high-speed liquid chromatography and so on. 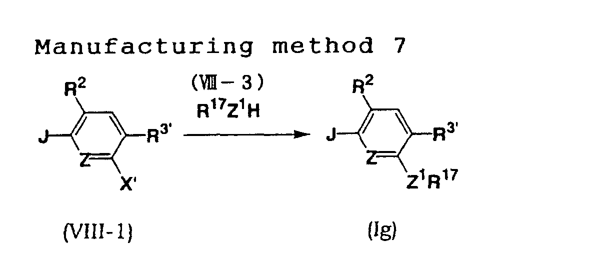 [Wherein each of J, R², R^{3'}, R¹⁷, X', Z and Z¹ has the same meaning described above.]

The compound (VIII-1) can be manufactured by the manufacturing method 1. In this reaction, the compound (VIII-3) is used usually about 0.8 to 3 times mol, or preferably about 0.9 to 1.3 times mol to the amount of the compound (VIII-1). The reaction can be carried out in a solvent, which does not influence the reaction. As the solvent, the same solvent as described in the manufacturing method 1 is used. As the preferable base in this reaction, the same base as described in the manufacturing method 1 is used, or the compund (VIII-3) can be used as base. The amount of the base is about 0.8 to 4.0 equivalents, or preferably about 1.0 to 1.5 equivalents to the amount of the compound (VIII-1). In the cases where the compund (VIII-3) is used as base, still more, it is necessary to add the compund (VIII-3) 1 equivalent. The reaction temperature varies depending on the solvent or the base, but is generally about -20 to 150 °C, or preferably about 0 to 80 °C. The reaction time varies depending on the reaction temperature, but is about 10 minutes to 14 hours, or preferably about 30 minutes to 8 hours. The reaction can be confirmed by thin-layer chromatography or high-speed liquid chromatography and so on. 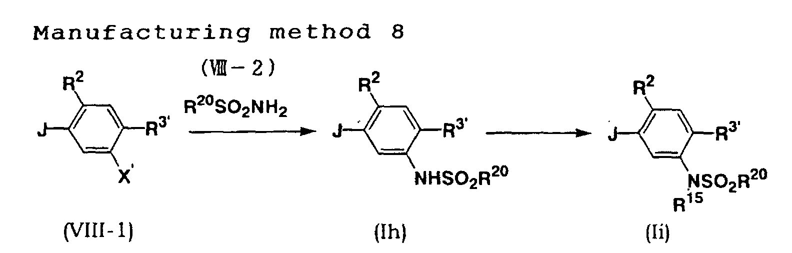 [wherein each of J, R², R^{3'}, R¹⁵ and X' has the same meaning described above]

The compound (VIII-2) is used usually about 0.8 to 3 times mol, or preferably about 0.9 to 1.3 times mol to the amount of the compound (VIII-1). The reaction can be carried out in a solvent, which does not influence on the reaction. As the solvent, the same solvent as described in the manufacturing method 1 is used. As the preferable base in this reaction, the same base as described in the manufacturing method 1 is used. The amount of the base is about 0.8 to 4.0 equivalents, or preferably about 1.0 to 1.5 equivalents to the amount of the compound (VIII-2). The reaction temperature varies depending on the solvent or the base, but is generally about 0 to 150 °C, or preferably about 20 to 100 °C. The reaction time varies depending on the reaction temperature, but is about 10 minutes to 14 hours, or preferably about 30 minutes to 5 hours. The compound (Ii) can be manufactured by alkylating or acylating the compound (Ih) in the presence of base. The alkylating and acylating agents(R⁶-X) is used usually about 0.8 to 3 times mol, or preferably about 0.9 to 2.0 times mol to the amount of the compound(Ih). The reaction can be carried out in a solvent, which does not influence the reaction. As the solvent, the same solvent described in the manufacturing method 1 and so on can be used. As the preferable base used in this reaction, the same base, shown in the manufacturing method 1 and so on can be used. The amount of the base is about 0.8 to 4.0 equivalents, or preferably about 1.0 to 1.5 equivalents to the amount of the compound (Ih). The reaction temperature varies depending on the solvent or the base, but is generally about -20 to 150 °C, or preferably about 0 to 80 °C. The reaction time varies depending on the reaction temperature, but is about 10 minutes to 14 hours, or preferably about 30 minutes to 8 hours. The reaction can be confirmed by thin-layer chromatography, high-speed liquid chromatography and so on. 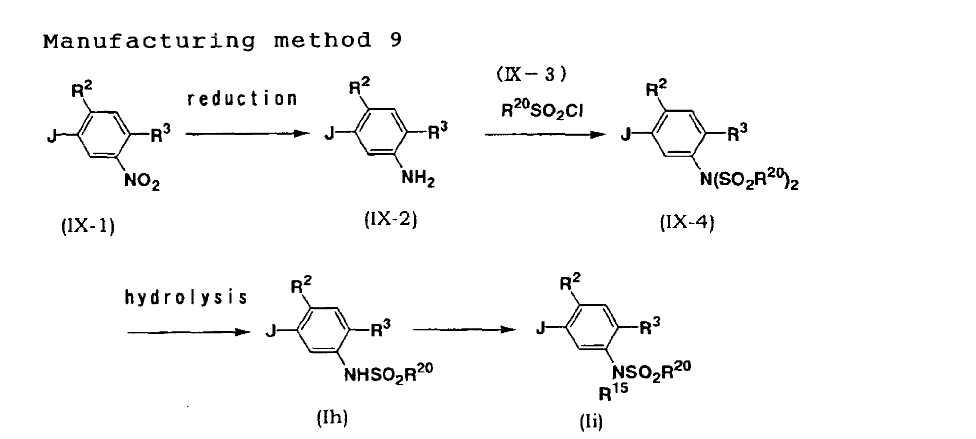 [Wherein each of J, R², R³, R¹⁵ and R²⁰ has the same meaning described above.]

The compound (IX-1) can be manufactured by the manufacturing method 2. The compound (IX-2) can be manufactual by the known method, that is, reacting the compound (IX-1) with iron or tin as a reducing agent under acid such as acetic acid, hydrochloric acid, and so on. As a solvent, there may be mentioned, aliphatic carboxylic acids such as acetic acid, and so on, alcohols such as methanol, ethanol, and so on, water, and so on. The reaction temperature varies depending on the solvent, but is generally about 0 to 100 °C, or preferably about 10 to 50 °C. The reaction time is about 30 minutes to 12 hours, or preferably about 1 hour to 6 hours. Also, the compound (IX-1) can be manufactured by contacting with palladium-carbon as a catalyst and adding hydrogen. As a solvent, there may be mentioned, aliphatic carboxylic acids such as acetic acid, and so on, aliphatic carbocylic esters such as ethyl acetate, and so on, alcohols such as methanol, ethanol, and so on. The reaction temperature is generally about 0 to 50 °C, or preferably about 10 to 25 °C, and the reaction will terminate at a point of time when theoretical amount of hydrogen is expenditured. In the reaction for producing the compound (IX-4) from the compound (IX-2), sulfonyl chloride (IX-3) is used usually about 1.5 to 4.5 times mol, or preferably about 1.8 to 3.0 times mol to the amount of the amino compound (IX-2). The reaction can be carried out in a solvent, which does not influence the reaction. As the solvent, the same solvent as described in the manufacturing method 1 is used. As the preferable base in this reaction, the same base as described in the manufacturing method 1 is used. The amount of the base is about 1.8 to 5.0 equivalents, or preferably about 2.0 to 3.5 equivalents to the amount of the compound (IX-2). The reaction temperature varies depending on the solvent or the base, but is generally about -20 to 150 °C, or preferably about 0 to 100 °C. The reaction time varies depending on the reaction temperature, but is about 10 minutes to 14 hours, or preferably about 30 minutes to 8 hours. The compound (Ii) can be manufactured by hydrolyzing the compound (IX-4) under basic condition. As a base, there may be mentioned, inorganic base such as alkali metal hydroxides such as sodium hydroxide, potassium hydroxide and so on, alkaline-earth metal hydroxides such as calcium hydroxide, and so on, alkali metal hydrogencarbonates such as sodium hydrogencarbonate, and so on, alkaline-earth metal carbonates such as calcium carbonate, and so on. The amount of base is about 0.8 to 3.0 equivalents, or preferably about 0.9 to 1.5 equivalents to the amount of the compound (IX-4). As a reaction solvent, there may be mentioned, water, ethers such as dioxane, tetrahydrofuran (THF), and so on, ketones such as acetone, methyl ethyl ketone, and so on, nitriles such as acetonitrile, and so on, aliphatic amides such as dimethylacetamide, and so on. These solvents can be mixed in appropriate ratio for use. The reaction temperature varies depending on the solvent or the base, but is generally about -10 to 100 °C, or preferably about 0 to 50 °C. The reaction time varies depending on the reaction temperature, but is about 10 minutes to 10 hours, or preferably about 30 minutes to 5 hours. The reaction can be confirmed by thin-layer chromatography or high-speed liquid chromatography and so on. 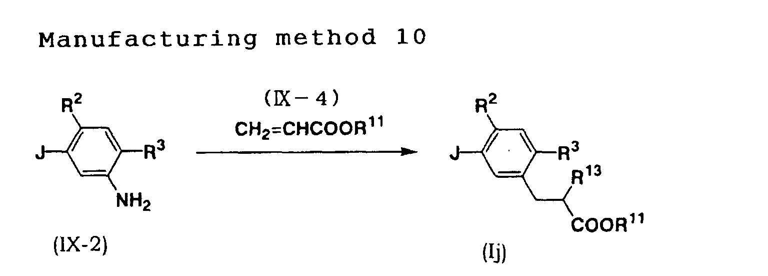 [wherein each of J, R², R³, R¹¹ and R¹³ has the same meaning described above.]

The compound (Ij) can be manufactured by reacting the compound (IX-2) with nitrite ester, and so on to produce a diazonium salt, and then reacting the said diazonium salt with acrylate ester (IX-4) under haloganated cupric. As a solvent of this reaction, there may be mentiond, for example, aromatic hydrocarobons such as benzene, toluene, and so on, ethers such as diethyl ether, diisopropyl ether, dioxane, tetrahydrofuran (THF), and so on, ketones such as acetone, methyl ethyl ketone, and so on, nitriles such as acetonitrile, and so on. As nitrite ester, it is used usually about 0.9 to 2.0 times mol, or preferably about 1.1 to 1.5 times mol. Also, as halogenated cupric, cupric chloride or cupric bromide is used about 0.9 to 2.0 equivalents, or preferably about 1.1 to 1.5 equivalens. The acrylate ester is used about 2.0 to 20 equivalents, or preferably about 5.0 to 15 equivalents. The reaction temperature varies depending on the solvent, but is generally about -10 to 50 °C, or preferably about 0 to 30 °C. The reaction time varies depending on the reaction temperature, but is about 1 to 48 hours, or preferably about 5 to 20 hours. The reaction can be confirmed by thin-layer chromatography or high-speed liquid chromatography and so on. 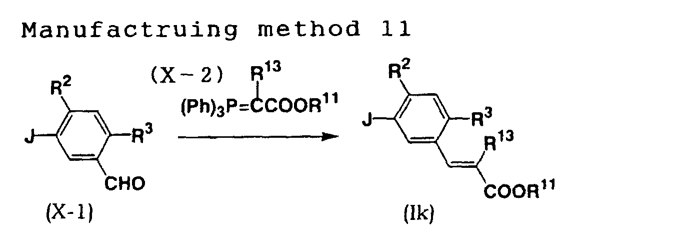 [Wherein each of J, R², R³', R¹¹ and R¹³ has the same meaning described above.]

In this reaction, the compound (X-2) is used usually about 0.8 to 2.0 times mol, or preferably about 0.9 to 1.5 times mol to the amount of the compound (X-1). The reaction can be carried out in a solvent, which does not influence the reaction. As the solvent, there may be mentioned, for example, aromatic hydrocarbons such as benzene, toluene, and so on, halogenated hydrocarbons such as chloroform, carbon tetrachloride, dichloromethane, and so on, ethers such as diethyl ether, diisopropyl ether, dioxane, tetrahydrofuran (THF) and so on. Generally this reaction is accelerated by adding base, but can progress without using base. As the base, there may be mentioned, for example, inorganic base such as alkali metal hydrogencarbonates such as sodium hydrogencarbonate, and so on, alkaline-earth metal carbonates such as calcium carbonate, and so on, metal hydroxides such as sodium hydroxide, potassium hydroxide, and so on. The amount of the base is about 0.8 to 2.5 equivalents, or preferably about 0.9 to 2.0 equivalents to the amount of the compound (X-1). The reaction temperature varies depending on the solvent or the base, but is generally about -20 to 100 °C, or preferably about 0 to 50 °C. The reaction time varies depending on the reaction temperature, but is about 10 minutes to 14 hours, or preferably about 30 minutes to 8 hours. The reaction can be confirmed by thin-layer chromatography, high-speed liquid chromatography and so on. 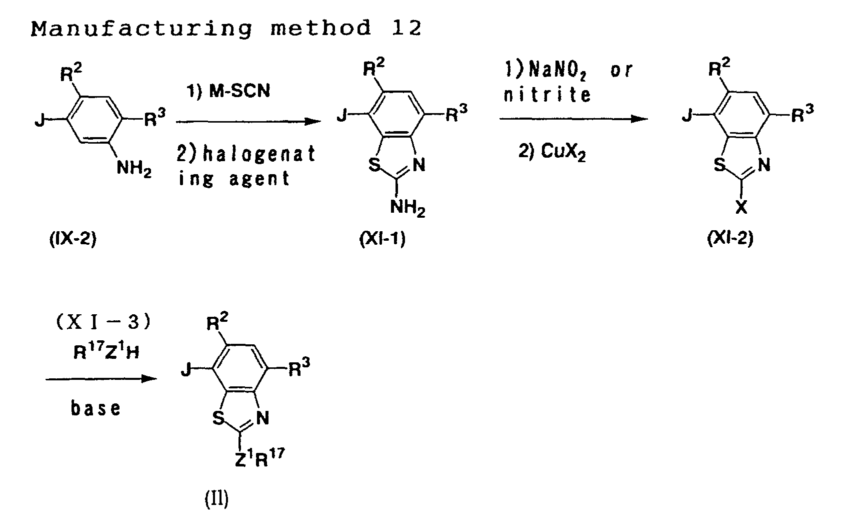 [wherein each of J, R², R³, R¹⁷ and Z¹ has the same meaning as mentioned above, X is halogen.]

In the reaction for producing the compound (XI-1) from the compound (IX-2), thiocyanate is about 0.8 to 3 times mol, or preferably about 0.9 to 1.3 times mol to the amount of the compound (IX-2). As thiocyanate, threre may be mentioned, for example, ammonium thiocyanate, sodium thiocyanate, potassium thiocyanate, and so on. This reaction is carried out under solvent, which does not influence the reaction. As the solvent, the same solvent, which shows the same reaction in the manufacturing method 1 can be used. The reaction temperature is -20 to 120 °C, or more preferably at 0 to 80 °C. And the reaction time varies depending on the reaction temperature, usually from about 10 minutes to 14 hours, or more preferably from about 30 minutes to 8 hours. Then, as a halogenaing agent, threre may be mentioned, for example, chlorine, bromine, thionyl bromide, thionyl chloride, hydrogen chloride, hydrogen bromide, and so on. The reaction temperature varies depending on the halogenating agent, is usually about -20 to 100 °C, or preferably about 0 to 80 °C. The reaction time varies depending on the reaction temperature, is about 10 minutes to 8 hours, or preferably about 30 minutes to 4 hours. In the reaction for producing the compound (XI-2) from the compound (XI-1), as a diazotizing agent, sodium nitrite, nitrite ester and so on can be used. The diazotizing agent is used usually about 0.8 to 2 times mol, or preferably about 0.9 to 1.2 times mol. This reaction is carried in a solvent, which does not influence the reaction. As preferable solvent, there may be mentioned, for example, water, acetic acid, sulfuric acid, hydrochloric acid, hydrobromic acid, acetonitrile and so on. As a halogenated copper, cupric chloride or cupric blomide and so on is used, and it is used usually about 0.8 to 2 times mol, or preferably about 0.9 to 1.2 times mol. The reaction temperature varies depending on a solvent, but is generally about -20 to 100 °C, or preferably about 0 to 80 °C. The reaction time varies depending on the reaction temperature, but is about 10 minutes to 8 hours, or preferably about 30 minutes to 4 hours. In manufacturing the compound (II), the compound (XI-3) is used generally about 0.8 to 3 times mol, or preferably about 0.9 to 2.0 times mol to the amount of the compound (XI-2). This reaction can be carried out in a solvent, which does not influence the reaction. As the solvent, the same solvent, which shows the same reaction in the manufacturing method 1 can be used. The preferable base, the same base, which shows the same reaction in the manufacturing method 1, can be used. The amount of the base is about 0.8 to 4.0 equivalents, or preferably about 1.0 to 1.5 equivalents to the amount of the compound (XI-2). The reaction temperature varies depending on the solvent or base, but is usually about -20 to 150 °C, or preferably about 0 to 80 °C. The reaction time varies depending on the reaction temperature, but is about 10 minutes to 14 hours, or preferably about 30 minutes to 8 hours. This reaction can be confirmed by thin-layer chromatography, high-speed liquid chromatography and so on. 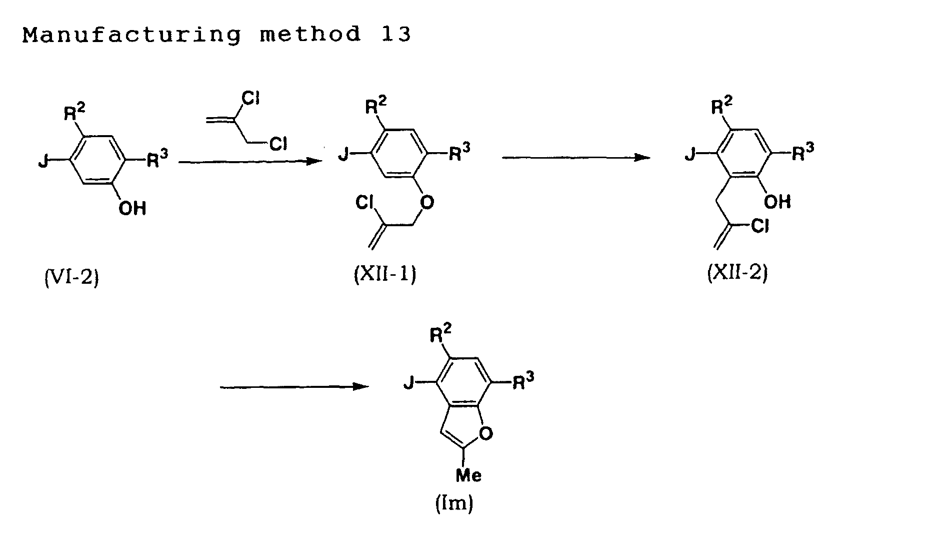 [wherein each of J, R² and R³ has the same meaning as mentioned above.]

The compound (XII-1) can be manufactured by reacting the compound (VI-2) with 2,3-dichloro-1-propene under a base.
2,3-Dichloro-1-propene is used generally about 0.8 to 3 times mol, or preferably about 0.9 to 1.5 times mol to the amount of the compound (VI-2). This reaction can be carried out in a solvent, which does not influence the reaction. As the solvent, the same solvent, which shows the same reaction in the manufacturing method 1, can be used. As the base, the same base, which shows the same reaction in the manufacturing method 1, can be used. The amount of the base is about 0.8 to 4 equivalents, or preferably about 1.0 to 1.5 equivalents to the amount of the compund (VI-2). The reaction temperature varies depending on the solvent or the base, but is generally about -20 to 150 °C, or preferably about 0 to 80 °C. The reaction time varies depending on the reaction temperature, but is generally about 10 minutes to 14 hours, or preferably about 30 to 8 hours. In the reaction for proceeding the compound (XII-2) from the compound (XII-1), the compound (XII-1) will be dissolved in aromatic hydrocarbons such as benzene, toluene, xylene, and so on, aliphatic amides such as dimethylformamide (DMF), dimethylacetamide, amines such as N,N-dimethylaniline and so on, on the condition that the reaction temperature is about 50 to 250 °C, or preferably about 100 to 200 °C, the reaction time varies depending on the reaction solvent, but is about 30 minutes to 20 hours, or preferably about 1 to 8 hours. The compound (XII-2) will be ring-closed by dissolving in organic acids such as methanesulfonic acid or trifluoromethanesulfonic acid and so on to produce the compound (Im). The reaction time is about 0 to 100 °C, or preferably about 20 to 50 °C, the reaction time varies depending on the reaction temperature, but is about 30 minutes to 10 hours, or preferably 1 to 5 hours. The reaction can be confirmed by thin-layer chromatography, high-speed liquid chromatography and so on. 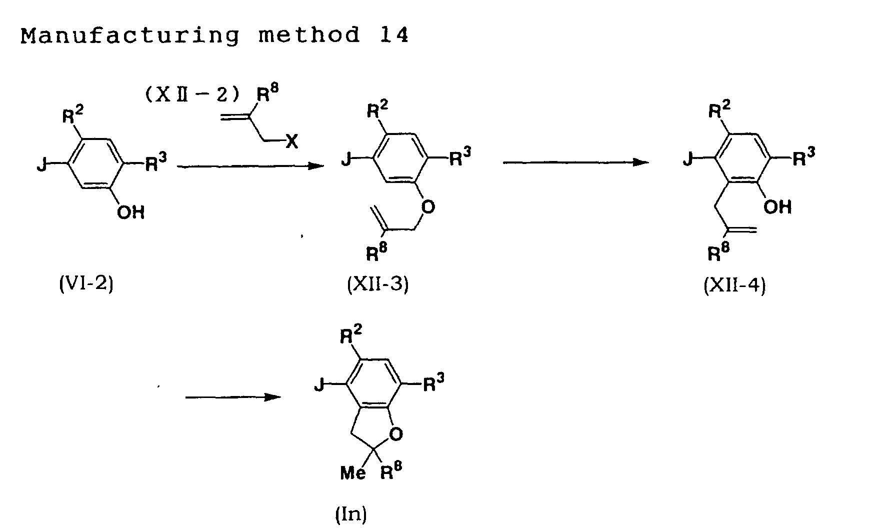 [wherein each of J, R², R⁸ and X has the same meaning as mentioned above.]

The compound (XII-3) can be manufactured by reacting the compound (VI-2) with the compound (XII-2) under a base. The compound (XII-2) is used generally about 0.8 to 3 times mol, or preferably about 0.9 to 2.0 times mol to the amount of the compound (VI-2). This reaction can be carried out in a solvent, which does not influence the reaction. As the solvent, the same solvent, which shows the same reaction in the manufacturing method 1, can be used. As the preferable base, the same base, which shows the same reaction in the manufacturing method 1, can be used. The amount of the base is about 0.8 to 4.0 equivalents, or preferably about 1.0 to 1.5 equivalents to the amount of the compound (VI-2). The reaction temperature varies depending on the solvent or the base, but is used generally about -20 to 150 °C, or more preferably about 0 to 80 °C. The reaction time varies depending on the reaction temperature, but is about 10 minutes to 14 hours, or preferably about 30 minutes to 8 hours. The compound (XII-3) will be heated in a solvent, which does not influence the reaction, to produce the compound (XII-4). As the preferable solvent, there may be mentioned, for example, aromatic hydrocarobons such as benzene, toluene, xylene, and so on, aliphatic amides such as dimethylformamide (DMF), dimethylacetamide, and so on, amines such as N,N-dimethyaniline,and so on. The reaction temperature is 50 to 250 °C, or preferably 80 to 200 °C, the reaction time varies depending on the reaction solvent, but is about 30 minutes to 20 hours, or preferably about 1 to 8 hours. The compound (In) can be manufactured by reacting the compound (XII-4) in a solvent, which does not influence the reaction under acidic condition. As the preferable solvent, aromatic hydrocarobons such as benzene, toluene, xylene, and so on, can be used. As the acid used in this reaction, there may be mentioned, for example, organic acid such as p-toluenesulfonic acid and so on, or inorganic acid such as sulfuric acid and so on, they can be used about 0.1 to 2 equivalents, or preferably about 0.2 to 0.5 equivalents to the amount of the compund (XII-4). The reaction temperature varies depending on the solvent, but is generally about 70 to 200 °C, or preferably about 80 to 130 °C. The reaction time varies depending on the reaction temperature, but is about 10 minutes to 14 hours, or preferably about 30 minutes to 8 hours. The reaction can be confirmed by thin-layer chromatography, high-speed liquid chromatography and so on. 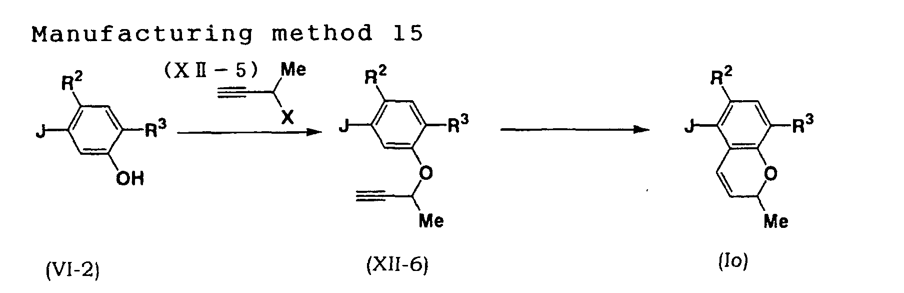 [wherein each of J, R², R³ and X has the same meaning as mentioned above.]

In producing the compound (XII-6), the compound (XII-5) is used generally about 0.8 to 3 times mol, or preferably about 0.9 to 2.0 times mol to the amount of the compound (VI-2). This reaction can be carried out in a solvent, which does not influence the reaction. As the solvent, the same solvent, which shows the same reaction in the manufacturing method 1, can be used. As the preferable base used in this reaction, the same base, which shows the same reaction in the manufacturing method 1, can be used. The amount of the base is about 0.8 to 4.0 equivalents, or preferably about 1.0 to 1.5 equivalents to the amount of the compound (VI-2). The reaction temperature varies depending on the solvent or the base, but is generally about -20 to 150 °C, or more preferably about 0 to 80 °C. The reaction time varies depending the reacton temperature, but is about 10 minutes to 14 hours, or preferably about 30 minutes to 8 hours. The compound (I₀) can be manufactured by ring-closing the compound (XII-6) under bases. This reaction can be carried in a solvent, which does not influence the reaction. As the preferable solvent, there may be mentioned, for example, aromatic hydrocarobons such as toluene, xylene, mesitylene and so on, halogenated hydrocarbons such as chloroform, carbon tetrachloride, and so on, amines such as N,N-dimethylaniline, N,N-diethylaniline, and so on, phosphoric amides such as hexamethyl phosphoric amide (HMPA) and so on, sulphones such as sulfolane and so on, polyalcohol such as diethyleneglycol and so on. These solvents can be mixed in appropriate ratio for use. As the preferable base used in this reaction, there may be mentioned, for example, sodium hydrogencarbonate, potassium carbonate, cesium fluoride, potassium fluoride, calcium fluoride, cesium chloride, and so on. The amount of the base is about 0.01 to 50 equivalents, or preferably about 0.1 to 20 equivalents to the amount of the compound (XII-6). The reaction temperature varies depending on the solvent or the base, but is generally about 60 to 220 °C, or preferably about 100 to 180 °C. The reaction time varies depending on the reaction temperature, but is about 30 minutes to 10 hours, or more preferably about 1 to 5 hours. The reaction can be confirmed by thin-layer chromatography, high-speed liquid chromatography and so on. 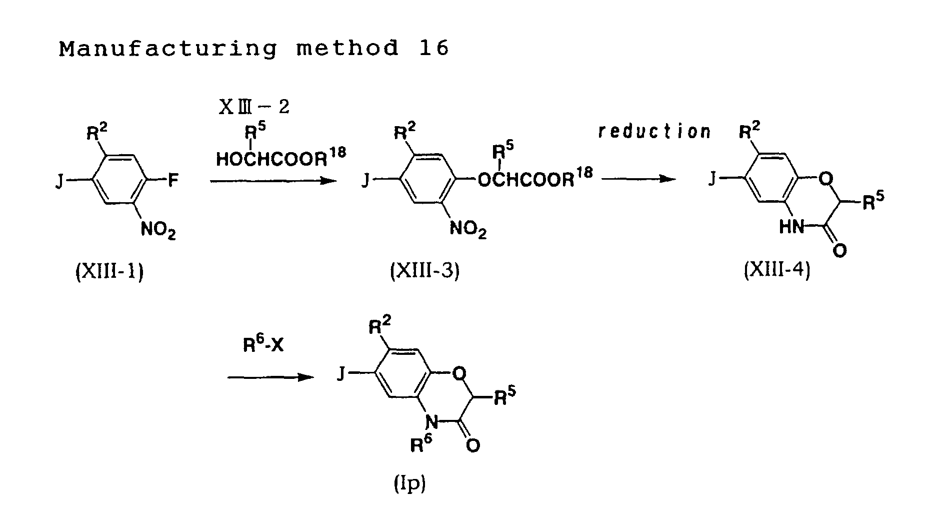 [wherein each of J, R², R⁵, R⁶, R¹⁸ and X has the same meaning as mentioned above.]

The compound (XIII-1) can be manufactured by the manufacturing method 2. The compound (XIII-3) can be manufactured by reacting the compound (XIII-1) with the compound (XIII-2) under a base. The compound (XIII-2) is used generally about 0.8 to 2.0 times mol, or preferably about 0.9 to 1.5 times mol to the amount of the compound (XIII-1). This reaction can be carried out in a solvent, which does not influence the reaction. As the solvent, the same solvent, which shows the same reaction in the manufacturing method 1, can be used. As the base used in this reaction, the same base, which shows the same reaction in the manufacturing method 1, can be used. The amount of the base is about 0.8 to 4.0 equivalents, or preferably about 1.0 to 1.5 equivalents to the amount of the compound (XIII-1). The reaction temperature varies depending on the solvent or the base, but is generally about -20 to 150 °C, or preferably about 0 to 80 °C. The reaction time varies depending on the reaction temperature, but is about 10 minutes to 14 hours, or preferably about 30 minutes to 8 hours. The compound (XIII-4) can be manufactured by reducing the compound (XIII-3) with a reduced iron under the condition of acid such as acetic acid, hydrochloric acid and so on. As the solvent, there may be mentioned, for example, aliphatic carboxylic acid ester such as ethyl acetate ester and so on, alcohols such as methanol, ethanol, and so on, water and so on. The reaction temperature varies depending on the solvent, but is generally about 0 to 100 °C, or preferably about 10 to 50 °C. The reaction time is about 30 minutes to 12 hours, or preferably about 1 to 6 hours. The compound (Ip) can be manufactured by alkylating or acylating the compound (XIII-4) under a base. The alkylating or acylating agent (R⁶-X) is used generally about 0.8 to 3 times mol, or preferably about 0.9 to 2.0 times mol to the amount of the compound (XIII-4). This reaction can be carried out in a solvent, which does not influence the reaction. As the solvent, the same solvent, which shows the same reaction in the manufacturing method 1, can be used. As the base, the same base, which shows the same reaction in the manufacturing method 1, can be used. The amount of the base is about 0.8 to 4.0 equivalents, or preferably about 1.0 to 1.5 equivalents to the amount of the compound(XIII-4). The reaction temperature varies depending on the solvent or base, but is generally about -20 to 150 °C, or preferably about 0 to 80 °C. The reaction time varies depending on the reaction temperature, but is about 10 minutes to 14 hours, or preferably about 30 minutes to 8 hours. The reaction can be confirmed by thin-layer chromatography, high-speed liquid chromatography and so on.

### EXAMPLES

Hereunder, the present invention is illustrated in more detail by reference to the following Reference Examples and Examples. However, the scope of the present invention is not to be considered to be restricted to the present embodiment. As the eluents used in the column chromatography in the Reference Examples and Examples, the eluents observed by means of TLC (Thin Layer Chromatography) were used. In the TLC-observation, the silicagel 60F₂₅₄ plates manufactured by Merck & Co., were used as the TLC-plate, and as the detection method, the UV-detector was adopted. As silica gel for the column chlomatography, silicagel 60(0.063-0.200 mm) manufactured by Merck & Co. was used. When a mixed solvent was used as eluent, the ratio shown in the ( ) indicates the volume to volume ratio of the solvents mixed. NMR(Nuclear Magnetic Resonance) spectrum means ¹H or ¹⁹F-NMR, and was measured by a Bruker AC-200P type (200MHz) spectrometer, using tetramethylsilane and fluorotrichloromethane as internal standard. All 6 values were in ppm. IR spectrum was measured by a Perkin Elmer Palagon 100 type FT-IR spectrmeter. The absorption band was shown by wave number (cm⁻¹).

Abbreviations used in the Reference Examples, Examples and Tables below have the meanings which follow; Me: methyl group, Et: ethyl group, n-Pr: normalpropyl group, i-Pr: isopropyl group, tert-Bu: tertiary-butyl group, Ph: phenyl group, s: singlet, br: broad, d: doublet, t: triplet, q: quartet, m: multiplet, dd: double doublet, J: coupling constant, Hz: Heltz, CDCl₃: deutero-chloroform(chloroform-d), DMSO-d₆: deutero-dimethyl sulfoxide, %: wt%, mp: melting point and dec: decomposition. In addition, room temperature means a temperatures within about 15-25 °C.

### Reference Examples

### Reference Example 1

### 6-Chloro-2-(4-cyano-2,5-difluorophenyl)-1,2,4-triazo lo[4,3-a]pyridin-3(2H)-one (Compound No. 1-1)

(1) 2,5-Dichloropyridine(2.0g, 14mmol) was added to hydrazine monohydrate(8ml ,0.165mol), and resulting mixture was stirred for 2 hours at 130 °C. After cooling, the reaction mixture was diluted with water, and the crystal was collected by filtration and dried to give 5-chloro-2-hydrazinopyridine (2.0g).
   ¹H-NMR(CDCl₃) δ :3.50(2H, br s), 5.92(1H, br s), 6.69(1H, d, J=8.8Hz), 7.43(1H, dd, J=8.8, 2.3Hz), 8.05(1H, d, J=2.3Hz).
(2) The mixture of the compound prepared in reference example 1-(1)(0.9g, 6.3mmol) and urea (0.68g, 11mmol) was stirred for 2 hours at 180 °C. After cooling, water was added to the reaction mixture, and the crystal was collected by filtration and dried to give 6-chloro-1,2,4-triazolo[4,3-a]pyridin-3(2H)-one(0.5g ). ¹H-NMR(DMSO-d₆ ) δ :7.18(1H, d, J=9.9Hz), 7.29(1H, d, 9.9Hz), 8.01(1H, s).
(3) The compound prepared in reference example 1-(2)(3.0g, 17.7mmol), 2,4,5-trifluorobenzonitrile (2.8g, 17.8mmol), and potassium carbonate(2.5g, 18.1mmol) were added to DMSO (15ml), and the resulting mixture was stirred for 4 hours at 60 °C. After cooling, icewater was added to the reaction mixture, and the crystal was collected by filtration, washed with diethyl ether and dried to give 6-chloro-2-(4-cyano-2,5-difluorophenyl)-1,2,4-triazolo[4,3-a]pyridin-3(2H)-one(4.2g).
   mp:>230 °C
   ¹H-NMR(CDCl₃) δ :7.15-7.17(2H, m), 7.51-7.58(1H, m), 7.66-7.72(1H, m), 7.86-7.88(1H, m).

### Reference Example 2

### 6-Cyano-2-(4-cyano-2,5-difluorophenyl)-1,2,4-triazolo[4,3-a]pyridin-3(2H)-one (Compound No. 1-2)

(1) To a solution of 6-chloronicotinamide (6.0g, 38.3mol) in pyridine (25ml), phosphorus oxychloride (7.6g, 49.7mmol) was added dropwise at room temperature, and the resulting mixture was stirred for 1 hour at same temperature. The reaction mixture was added to icewater, neutralized with dilute hydrochloric acid. Then the crystal was collected by filtration, washed with water and dried to give 6-chloro-3-cyanopyridine (5.3g).
   ¹H-NMR(CDCl₃) δ :7.49(1H, d, J=8.3Hz), 7.93(1H, d, J=8.3Hz), 8.70(1H, s).
(2) To a solution of the compound prepared in reference example 2-(1)(1.0g, 7.2mmol) in ethanol (15ml), hydrazine monohydrate (0.72g, 14.4mmol) and potassium carbonate(0.5g, 3.5mmol) were added and the resulting mixture was stirred for 12 hours at 50 °C. The reaction mixture was diluted with water, and the crystal was collected by filtration, washed with water and dried to give 5-cyano-2-hydrazinopyridine (0.53g).
   ¹H-NMR(DMSO-d₆) δ :6.75(1H, d, J=9.0Hz), 7.73(1H,dd, J=9.9, 2.1Hz), 8.35(1H, d, J=2.1Hz), 8.56(1H, s).
(3) The compound prepared in reference example 2-(2)(0.5g, 3.73mmol) and urea (0.5g, 8.33mmol) were added to DMF (1ml) and the resulting mixture was stirred at 120 °C for 2 hours, further at 140 °C for 6 hours. After cooling, the icewater was added to the reaction mixture, and the crystal was collected by filtration, washed with water and dried to give 6-cyano-1,2,4-triazolo[4,3-a]pyridin-3(2H)-one (0.42g).
   ¹H-NMR(DMSO-d₆) δ :7.25-8.37(2H, m), 8.73(1H, s), 12.85(1H, s).
(4) The compound prepared in reference example 2-(3)(0.4g, 2.5mmol), 2,4,5-trifluorobenzonitrile (0.4g, 2.5mmol) and potassium carbonate (0.35g, 2.5mmol) were added to DMSO (10ml), and the resulting mixture was stirred for 1 hour at 50 °C. After cooling, icewater was added to the reaction mixture, then the resulting mixture was neutralized with dilute hydrochloric acid, and extracted with ethyl acetate. The extract was dried and evaporated. Then diethyl ether was added to the residue, and the precipitated crystal was collected by filtration and dried to give 6-cyano-2-(4-cyano-2,5-difluorophenyl)-1,2,4-triazolo[4,3-a]pyridin-3(2H)-one(0.37g).
   ¹H-NMR(CDCl₃) δ :7.17-7.29(2H, m), 7.53-7.70(2H, m), 8.28(1H,m).

### Reference Example 3

### 6-Chloro-2-(4-cyano-2-fluoro-5-methoxyphenyl)-1,2,4-triazolo[4,3-a]pyridin-3(2H)-one (Compound No. 1-3)

(1) To a solution of 2,4,5-trifluorobenzoyl chloride (1.0g, 5.1mmol) in 1-methyl-2-pyrrolidone (10ml), sodium hydroxide (powder) (1.0g, 25mmol) was added, and the resulting mixture was stirred at 130 °C for 3 hours. After cooling, icewater was added to the reaction mixture, and the resulting mixture was neutralized with dilute hydrochloric acid and extracted with ethyl acetate. The extract was washed with water, dried and evaporated to give 4,5-difluoro-2-hydroxybenzoic acid (0.8g).
   ¹H-NMR(CDCl₃) δ :6.75-6.84(1H, m), 7.66-7.75(1H, m), 10.66(1H, br s).
(2) To a suspension of the compound prepared in reference example 3-(1) (2.0g, 11.5mmol) in water (20ml), sodium hydroxide (1.8g, 45mol) and dimethyl sulfate (2.9g) were added at 70 °C, and the resulting mixture was stirred at same temperature for 12 hours. Furthermore, sodium hydroxide and dimethyl sulfate were added to the reaction mixture every 12 hours, and each of them were added about 8 equivalents as the total amount, and then the reaction completed. After cooling, the reaction mixture was neutralized with dilute hydrochloric acid and extracted with ethyl acetate, and the extract was dried and evaporated to give 4,5-difluoro-2-methoxybenzoic acid(1.23g).
   ¹H-NMR(CDCl₃) δ :4.05(3H, s), 6.85-6.91(1H, m), 7.95-8.05(1H, m).
(3) To a solution of the compound prepared in reference example 3-(2) (1.2g, 6.4mmol) in diethyl ether (30ml), thionyl chloride (1.52g, 12.8mmol) and DMF (1drop) were added, and the resulting mixture was stirred at 45 °C for 2 hours. After the reaction mixture was evaporated, the residue was dissolved with acetonitrile (10ml), to the resulting mixture, aqueous ammonia (25%, 3ml) was added drpowise, and the resulting mixture was stirred at same temperature for 30 minutes. Acetonitrile was evaporated, water was added to the residue, and the resulting mixture was extracted with ethyl acetate. The extract was dried and evaporated. Diisopropyl ether was added to the residue, and the crystal separated was collected by filtration and dried to give 4,5-difluoro-2-methoxybenzamide (0.7g).
   ¹H-NMR(CDCl₃) δ :3.96(3H, s), 5.95(1H, br s), 6.78-6.87(1H, m), 7.60(1H, br s), 8.02-8.13(1H, m).
(4) To a solution of the compound prepared in reference example 3-(3) (0.3g, 1.6mmol) in pyridine (3ml), phosphorus oxychloride (0.37g, 2.4mmol) was added, and the resulting mixture was stirred at same temperature for 30 minutes. The reaction mixture was added to icewater, neutralized with dilute hydrochloric acid and extracted with diethyl ether, and the extract was dried and evaporated. Hexane was added to the residue, and the crystal separated was collected by filtration and dried to give 4,5-difluoro-2-methoxybenzonitrile (0.22g).
   ¹H-NMR(CDCl₃) δ :3.91(3H, s), 6.77-6.86(1H, m), 7.36-7.45(1H, m).
(5) The compound prepared in reference example 1-(2)(0.5g, 3.0mmol), the compound prepared in reference example 3-(4)(0.5g, 3.0mmol) and potassium carbonate (0.5g, 3.5mmol) were added to DMSO(15ml), and the resulting mixture was stirred for 3 hour at 75 °C . After cooling, icewater was added to the reaction mixture, and then the resulting mixture was neutralized with dilute hydrochloric acid. Ethyl acetate was added to the mixture, and the crystal separated was collected by filtration and dried to give 6-chloro-2-(4-cyano-2-fluoro-5-methoxyphenyl)-1,2,4-triazolo[4,3-a]pyridin-3(2H)-one(0.37g).
   ¹H-NMR(CDCl₃ ) δ :3.97(3H, s), 7.14-7.16(2H, m), 7.31(1H, d, J=5.7Hz), 7.48(1H, d, J=9.4Hz), 7.78(1H, s).

### Reference Example 4

### 6-Chloro-2-(2,5-difluoro-4-nitrophenyl)-1,2,4-triazolo[4,3-a]pyridin-3(2H)-one (Compound No. 1-4)

The compound prepared in reference example 1-(2)(3.0g, 17.7mmol), 2,4,5-trifluoronitrobenzene(3.1g. 17.7mmol) and potassium carbonate(2.5g, 18.1mmol) were added to DMSO(15ml), and the resulting mixture was stirred for 4 hours at 60 °C. After cooling, icewater was added to the reaction mixture, and the crystal separated was collected by filtration and dried. The crystal obtained was washed with diisopropyl ether, and then dried to give 6-chloro-2-(2,5-difluoro-4-nitrophenyl)-1,2,4-triazolo[4,3-a]pyridin-3(2H)-one (2.6g).
mp:184-187 °C
¹H-NMR(CDCl₃) δ :7.16-7.17(2H, m), 7.80-7.85(1H, m), 7.87(1H, s), 8.04-8.09(1H, m).

### Reference Example 5

### 6-chloro-2-(6-chloro-5-cyano-3-fluoropyridyl-2-yl)-1,2,4-triazolo[4,3-a]pyridin-3(2H)-one (Compound No. 1-5)

The compound prepared in reference example 1-(2)(0.44g, 2.6mmol), 2,6-dichloro-5-fluoronicotinonitrile (0.5g, 2.6mmol) and potassium carbonate (0.36g, 2.6mmol) were added to DMSO (10ml), and the resulting mixture was stirred for 4-hours at 50 °C. After cooling, icewater was added to the reaction mixture, and then the resulting mixture was neutralized with dilute hydrochloric acid. The crystal separated was collected by filtration, washed with acetonitrile, and then dried to give 6-chloro-2-(6-chloro-5-cyano-3-fluoropyridyl-2-yl)-1,2,4-triazolo[4,3-a]pyridin-3(2H)-one (0.31g).
¹H-NMR(CDCl₃) δ :7.17-7.18(2H, m), 7.85(1H, s), 7.97(1H, d, J=8.0Hz).

### Reference Example 6

### 2-(4-Cyano-2,5-difluorophenyl)-5-methylthiazolo[2,3-c][1,2,4]triazol-3(2H)-one (Compound No. 1-6)

(1)The mixture of 2-hydrazinothiazole hydrochloride(0.55g, 3.0mmol) and urea (0.36g, 6.0mmol) was stirred for 1 hour at 95 °C. Furthermore, DMF (3ml) was added to the reaction mixture, and the resulting mixture was stirred at 130 °C for 3 hours. After cooling, chloroform was added to the reaction mixture, and the insoluble part was filtered off, and the filtrate was evaporated. Then the residue was purified by silicagel column chromatography (ethyl acetate:hexane=2:1), to give 5-methylthiazolo[2,3-c][1,2,4]triazol-3(2H)-one(0.12g).
   ¹H-NMR(DMSO-d₆) δ :2.50(3H, s), 6.03(1H, s), 9.81(1H, br s).
(2) The compound prepared in reference example 6-(1)(70mg, 0.45mmol), 2,4,5-trifluorobenzonitrile (100mg, 0.64mmol) and potassium carbonate (70mg, 0.51mmol) were added to DMSO (5ml), and the resulting mixture was stirred for 4 hours at 45 °C. After cooling, icewater was added to the reaction mixture, and then the resulting mixture was neutralized with dilute hydrochloric acid. The crystal separated was collected by filtration, washed with water, and then dried to give 2-(4-cyano-2,5-difluorophenyl)-5-methylthiazolo[2,3-c][1,2,4]triazole-3(2H)-one (110mg).
   ¹H-NMR(CDCl₃) δ :2.53(3H, s, J=1.5Hz), 6.11(1H, q, J=1.5Hz), 7.47-7.54(1H, m), 7.58-7.65(1H, m).

### Reference Example 7

### 2-(4-Cyano-2,5-difluorophenyl)-1,2,4-triazolo[4,3-b] pyridazin-3(2H)-one (Compound No. 1-7)

(1) To a solution of 3,6-dichloropyridazine (4.5g, 30.2mmol) and semicarbazide hydrochloride (6.6g, 59.2mmol) in ethanol (30ml), concentrated hydrochloric acid (3 drops) was added, and the resulting mixture was stirred at 95 °C for 13 hours and further 110 °C for 6 hours. After cooling, the reaction mixture was evaporated. Then, cold water was added to the residue, and the crystal separated was collected by filtration, washed with water and diethyl ether repeatedly and dried to give 6-chloro-1,2,4-triazolo[4,3-b]pyridazin-3(2H)-one (1.6g).
   ¹H-NMR(DMSO-d₆) δ :6.98(1H, d, J=9.8Hz), 7.63(1H, d, J=9.8Hz), 12.82(1H, br s).
(2) To the solution of the compound prepared in reference example 7-(1) (0.6g, 3.52mmol) in methanol (44ml), aqueous ammonia (25%, 1.4ml) was added. Then, Pd-C (10%, 160mg) was added to the above-mentioned solution, hydrogenation reaction carried out at about 3 atoms. The reaction mixture was filtered on the celite, and the filtrate was evapolated. Cold water was added to the residue, and the crystal separated was collected by filtration and dried to give 1,2,4-triazolo[4,3-b]pyridazin-3(2H)-one(0.25g).
   ¹H-NMR(DMSO-d₆) δ :7.04-7.11(1H, m), 7.77(1H, d, J=9.6Hz), 8.22(1H, s), 12.66(1H, br s).
(3) The compound prepared in reference example 7-(2) (0.2g, 1.47mmol), 2,4,5-trifluorobenzonitrile (0.25g, 1.59mmol) and potassium carbonate (0.2g, 1.45mmol) were added to DMSO (5ml), and the resulting mixture was stirred for 14 hours at 40 °C. After cooling, icewater was added to the reaction mixture, and then the resulting mixture was neutralized with dilute hydrochloric acid and extracted with chloroform. The extract was washed with water, then dried, and evaporated. Diethyl ether was added to the residue, and the crystal separated was collected by filtration and dried to give 2-(4-cyano-2,5-difluorophenyl)-1,2,4-triazolo[4,3-b] pyridazin-3(2H)-one(0.2g).
   ¹H-NMR(CDCl₃) δ :7.05(1H, dd, J=9.7, 4.0Hz), 7.56-7.61(2H, m), 7.71-7.78(1H, m), 8.17-8.20(1H, m).
   IR(Nujol;cm⁻¹) 3200, 2200, 1718.

### Reference Example 8

### 2-(4-Cyano-2,5-difluorophenyl)-6-methoxy-1,2,4-triazolo[4,3-b] pyridazin-3(2H)-one (Compound No. 1-8)

(1) The compound prepared in reference example 7-(1)(0.4g, 2.35mmol), 2,4,5-trifluorobenzonitrile (0.4g, 2.55mmol) and potassium carbonate (0.35g, 2.54mmol) were added to DMSO(12ml), and the resulting mixture was stirred for 18 hours at 40 °C. After cooling, icewater was added to the reaction mixture, and then the resulting mixture was neutralized with dilute hydrochloric acid. The crystal separated was collected by filtration, washed with diethyl ether and dried to give 6-chloro-2-(4-cyano-2,5-difluorophenyl)-1,2,4-triazolo[4,3-b]pyridazin-3(2H)-one(0.55g).
   IR(Nujol;cm⁻¹) 3058, 2244, 1730.
   ¹H-NMR(CDCl₃, DMSO-d₆) δ :7.15(1H, d, J=9.8Hz), 7.55-7.75(3H, m).
(2) The compound prepared in reference example 8-(1) (0.2g, 0.65mmol), potassium carbonate (0.18g, 1.3mmol) and methanol (0.2ml) were added to DMSO (10ml), and the resulting mixture was stirred for 4 hours at 60 °C. After cooling, icewater was added to the reaction mixture, and then the resulting mixture was neutralized with dilute hydrochloric acid and extracted with ethyl acetate. The extract was dried and evaporated. Diisopropyl ether was added to the residue, and the crystal separated was collected by filtration and dried to give 2-(4-cyano-2,5-difluorophenyl)-6-methoxy-1,2,4-triazolo[4,3-b]pyridazin-3(2H)-one(0.14g).
   ¹H-NMR(CDCl₃) δ :4.08(3H, s), 6.81(1H, d, J=9.9Hz), 7.48(1H, d, J=9.9Hz), 7.53-7.58(1H, m), 7.70-7.77(1H, m).

### Reference Example 9

### 4,5-Difluoro-2-propargyloxybenzonitrile (Compound No. 1-9)

(1) To a solution of the compound prepared in reference example 3-(1) (0.26g, 1.5mmol) in DMF (8ml), potassium carbonate (0.62g, 4.5mmol) and propargylbromide(0.53g, 4.45mmol) were added, and the resulting mixture was stirred for 3 hours at 40 °C . The reaction mixture was neutralized with dilute hydrochloric acid, and extracted with ethyl acetate. The extract was dried and evaporated. Methanol (10ml) and water (5ml) were added to the residue, further sodium hydroxide (0.12g, 3.0mmol) was added, and the resulting mixture was stirred at 50 °C for 30 minutes. After cooling, the reaction mixture was neutralized with dilute hydrochloric acid and diluted with water, and the crystal separated was collected by filtration, washed with water and dried to give 4,5-difluoro-2-propargyloxybenzoic acid (0.24g).
   ¹H-NMR(CDCl₃) δ :2.70(1H, t, J=2.1Hz), 4.90(2H, d, J=2.1Hz), 7.00-7.09(1H, m), 7.96-8.95(1H, m).
(2) To a solution of the compound prepared in reference example 9-(1) (0.2g, 0.94mmol) in THF (5ml), thionyl chloride (0.22g, 1.85mmol) and DMF (ldrop) were added, and the resulting mixture was stirred at 60 °C for 2 hours. After the reaction mixture was evaporated, the residue was dissolved with acetonitrile (5ml), to the resulting mixture, aqueous ammonia (25%, 1ml) was added dropwise, and the resulting mixture was stirred at same temperature for 30 minutes. Acetonitrile was evaporated, water was added to the residue, and the resulting mixture was extracted with ethyl acetate. The extract was dried, and then evaporated. Diisopropyl ether was added to the residue, the crystal separated was collected by filtration and dried to give 4,5-difluoro-2-propargyloxybenzamide (0.2g).
   ¹H-NMR(CDCl₃) δ :2.64(1H, t, J=2.4Hz), 4.82(1H, d, J=2.4Hz), 5.85(1H, br s), 6.90-6.99(1H, m), 7.50(1H, br s), 8.03-8.13(1H,m).
(3) To a solution of the compound prepared in reference example 9-(2)(0.15g, 0.71mmol) in pyridine (2ml), phosphorus oxychloride(0.16g, 1.07mmol) was added dropwise at room temperature, and the resulting mixture was stirred for 30 minutes at same temperature. The reaction mixture was added to icewater, neutralized with dilute hydrochloric acid, and extracted with ethyl acetate. The extract was dried, and then evaporated. Hexane was added to the residue, and the crystal separated was collected by filtration and dried to give 4,5-difluoro-2-propargyloxybenzonitrile (0.1g).
   ¹H-NMR(CDCl₃) δ :2.63(1H, t, J=2.4Hz), 4.81(2H, d, J=2.4Hz), 6.98-7.07(1H, m), 7.39-7.47(1H, m).

Hereunder, the compounds obtained by the similar way to reference example 1 to reference example 9 are shown with ¹H-NMR spectral data and melting point (mp).

### Compound No. 1-10

6-bromo-1,2,4-triazolo[4,3-a]pyridin-3(2H)-one
¹H-NMR(DMSO-d₆) δ :7.22-7.29(2H, m), 8.07(1H, s), 12.6(1H, br s).

### Compound No. 1-11

6-trifluoromethyl-1,2,4-triazolo[4,3-a]pyridin-3(2H)-one
¹H-NMR(DMSO-d₆) δ :7.26-7.45(2H, m), 8.24(1H, s), 12.74(1H, br s).
mp:144-146 °C.

### Compound No. 1-12

6,8-dichloro-1,2,4-triazolo[4,3-a]pyridin-3(2H)-one
¹H-NMR(DMSO-d₆) δ :7.58(1H, d, J=1.54Hz), 8.08(1H, d, J=1.61Hz).
mp:not more than 143 °C.

### Compound No. 1-13

2-(4-cyano-2,5-difluorophenyl)-1,2,4-triazolo[4,3-a] pyridin-3(2H)-one
¹H-NMR(CDCl₃) δ :6.52-6.65(1H, m), 7.10-7.26(2H, m), 7.55(1H, dd), 7.72(1H, dd), 7.78-7.88(1H, m).
mp:174-177 °C.

### Compound No. 1-14

6-bromo-2-(4-cyano-2,5-difluorophenyl)-1,2,4-triazolo[4,3-a]pyridin-3(2H)-one
¹H-NMR(CDCl₃) δ :7.09(1H, d, J=10.9Hz), 7.24(1H, d, J=10.9Hz), 7.51-7.58(1H,m), 7.65-7.73(1H, m), 7.97(1H, s).
mp:218-220 °C.

### Compound No. 1-15

2-(4-cyano-2,5-difluorophenyl)-6-trifluoromethyl-1,2,4-triazolo[4,3-a]pyridin-3(2H)-one
¹H-NMR(CDCl₃) δ :7.28-7.30(2H, m), 7.52-7.71(2H, m), 8.21(1H, s).
mp:217-219 °C.

### Compound No. 1-16

8-chloro-2-(4-cyano-2,5-difluorophenyl)-1,2,4-triazolo[4,3-a]pyridin-3(2H)-one
¹H-NMR(CDCl₃) δ :6.53-6.60(1H, m), 7.31(1H, d, J=7.0Hz), 7.54-7.59(1H, m), 7.65-7.73(1H, m), 7.80(1H, d, J=7.0Hz).
mp:225-227 °C.

### Compound No. 1-17

2-(4-cyano-2,5-difluorophenyl)-5-methyl-1,2,4-triazolo[4,3-a]pyridin-3(2H)-one
¹H-NMR(CDCl₃) δ :2.81(3H, s), 6.10-6.15(1H, m), 6.90-7.05(2H, m), 7.50-7.55(1H, m), 7.65-7.70(1H, m).

### Compound No. 1-18

2-(4-cyano-2,5-difluorophenyl)-6-methyl-1,2,4-triazolo[4,3-a]pyridin-3(2H)-one ¹H-NMR(CDCl₃) δ :2.24(3H, s), 7.08-7.09(2H, m), 7.49-7.59(2H, m), 7.59-7.73(1H, m).

### Compound No. 1-19

2-(4-cyano-2,5-difluorophenyl)-7-methyl-1,2,4-triazolo[4,3-a]pyridin-3(2H)-one
¹H-NMR(CDCl₃) δ:2.34(3H, s), 6.41(1H, d, J=7.2Hz), 6.89(1H, m), 7.48-7.56(1H, m), 7.66-7.75(2H,m).

### Compound No. 1-20

2-(4-cyano-2,5-difluorophenyl)-8-methyl-1,2,4-triazolo[4,3-a]pyridin-3(2H)-one
¹H-NMR(CDCl₃) δ :2.37(3H, s), 6.48-7.00(1H, m), 6.96-7.00(1H, m), 7.50-7.57(1H, m), 7.66-7.75(2H, m).

### Reference Example 10

### 1-(4-Chloro-2-fluoro-5-methoxyphenyl)-2-(5-chloro-2-pyridyl)hydrazine (Compound No. 2-1)

(1) The solution of 2-aminopyridine (50.0g, 0.39mol) and dimethylsulfide (26.6g, 0.43mol) in dichloroethane(300ml) was cool down at -30 °C. NCS (52.0g, 0.39mol) was added dropwise to above-mentined solution, and the resulting mixture was stirred at -20 °C for 2 hours and further at room temperature for 1 hour. Sodium methoxide (28% methanol solution, 128g, 0.66mol) was added to the reaction mixture, the resulting mixture was stirred for 10 minutes, and then after addition of water (300ml), stirred for 4 hours. The organic layer was separated, and the water layer was extracted with chloroform. The organic layer combined was washed with water, dried and evaporated. Diisopropylether was added to the residue, the crystal separated was collected by filtration and dried to give S,S-dimethyl-N-2-(5-chloro-2-pyridyl)sulfilimine (45g).
   ¹H-NMR(CDCl₃) δ :2.72(6H, s), 6.60(1H, d, J=8.9Hz), 7.26(1H, d, J=8.9Hz), 7.92(1H, s).
(2) To a solution of mCPBA (70%, 45.0g, 0.18mol) in dichloromethane (300ml), the solution of the compound prepared in reference example 10-(1) (20.0g, 0.11mmol) in dichloromethane (200ml) was added dropwise at -50°C, and the resulting mixture was stirred at -20 °C for 1 hour, at room temperature for 1 hour. Further, dimethylsulfide (4ml) was added at room temperature, and the resulting mixture was stirred at same temperature for 1 hour. The reaction mixture was neutralized with sodium bicarbonate water, and the organic layer was separated, dried and evaporated. Diethy ether was added to the residue, the crystal separated was collected by filtration and dried to give 5-chloro-2-nitrosopyridine (11.0g).
   ¹H-NMR(CDCl₃) *δ* :7.28(1H, d, J=8.5Hz), 8.02(1H, d, J=8.5Hz), 8.77(1H, s).
(3) To a solution of the compound prepared in reference example 10-(2) (0.4g, 2.8mmol) and 4-chloro-2-fluoro-m-anisidine (0.4g, 2.3mmol) in dichloroethane (15ml), trifluoroacetic acid (5drops) was added at room temperature, and the resulting mixture was stirred at same temperature for 14 hours, and the mixture was evaporated. Diisopropyl ether was added to the residue, and the crystal separated was collected by filtration. The crystal obtained was purified by silicagel column chromatography (chloroform) to give 5-chloro-2-[(4-chloro-2-fluoro-5-methoxyphenyl)azo] pyridine (0.3g).
   ¹H-NMR(CDCl₃) δ :3.94(3H, s), 7.39(1H, d, J=9.6Hz), 7.48(1H, d, J=6.4Hz), 7.75-7.90(2H, m), 8.71(1H, s).
(4) To a solution of the compound prepared in reference example 10-(3)(0.15g, 0.5mmol) in toluene (10ml), tributyltin hydride(0.44mg, 1.5mmol) was added, and the resulting mixture was stirred at 70 °C for 1 hour. After cooling, the reaction mixture was evaporated, and the oil obtained was purified by silicagel column chromatography(chloroform→chloroform:ethyl acetate=1:1), to give 1-(4-chloro-2-fluoro-5-methoxyphenyl)-2-(5-chloro-2-pyridyl)hydrazine(0.08g).
   ¹H-NMR(CDCl₃) δ :3.76(3H, s), 6.05(1H, br s), 6.40(1H, br s), 6.60(1H, d, J=7.6Hz), 6.80(1H, d, J=9.1Hz), 7.09(1H, d, J=10.7Hz), 7.50-7.55(1H, m), 8.13(1H, s).

### Reference Example 11

### 1-(4-Chloro-2-fluoro-5-nitrophenyl)-2-(5-chloro-2-pyridyl)hydrazine (Compound No. 2-2)

(1) To a solution of the compound prepared in reference example 10-(2) (0.1g, 0.7mmol) and 4-chloro-2-fluoro-5-nitroaniline (0.13g, 0.68mmol) in dichloroethane (5ml), p-toluenesulfonic acid (cat.) was added, and the resultant was stirred at 50 °C for 12 hours. After the reaction mixture was evaporated, diisopropyl ether was added to the residue, and the crystal obtained was purified by silicagel column chromatography (chloroform), to give 5-chloro-2-[(4-chloro-2-fluoro-5-nitrophenyl)azo] pyridine(0.3g).
   ¹H-NMR(CDCl₃) δ :7.57(1H, d, J=9.3Hz), 7.85-7.95(2H, m), 8.51(1H, d, J=6.8Hz), 8.75(1H, d, J=2.3Hz).
(2) To a solution of the compound prepared in reference example 11-(1) (0.54g, 1.71mmol) in toluene (20ml), tributyltin hydride (1.49mg, 5.12mmol) was added, and the resulting mixture was stirred at 70 °C for 1 hour. After cooling, the reaction mixture was evaporated, and the oil obtained was purified by silicagel column chromatography (chloroform→chloroform:ethyl acetate=1:1), to give 1-(4-chloro-2-fluoro-5-nitrophenyl)-2-(5-chloro-2-pyridyl)hydrazine (0.35g).
   ¹H-NMR(CDCl₃) δ :6.25(1H, br s), 6.35(1H, br s), 6.72(1H, d, J=8.8Hz), 7.24(1H, d, J=8.8Hz), 7.50-7.55(1H, m), 7.62(1H, d, J=7.9Hz), 8.13(1H, s).

Hereunder, the compounds obtained by the similar way to reference example 10 and 11 are shown with ¹H-NMR spectral data.

### Compound No. 2-3

1-(4-chloro-5-ethoxycarbonyl-2-fluorophenyl)-2-(5-chloro-2-pyridyl)hydrazine
¹H-NMR(CDCl₃) δ :1.34(3H, t, J=7.2Hz), 4.33(2H, q, J=7.2Hz), 6.15(1H, br s), 6.45(1H, br s), 6.76(1H, d, J=8.9Hz), 7.16(1H, d, J=10.9Hz), 7.45(1H, d, J=9.1Hz), 7.50-7.55(1H, m), 8.11(1H, s).

### Compound No. 2-4

1-(4-chloro-2-fluoro-5-formylphenyl)-2-(5-chloro-2-pyridyl)hydrazine
¹H-NMR(CDCl₃) δ :6.16(1H, br s), 6.36(1H, br s), 6.70(1H, d, J=8.8Hz), 7.18(1H, d, J=10.7Hz), 7.49(1H, dd, J=8.8, 2.4Hz), 7.56(1H, d, J=9.1Hz), 8.11(1H, d, J=2.4Hz), 10.32(1H, s).

### Compound No. 2-5

1-(2,4-difluoro-5-nitrophenyl)-2-(5-chloro-2-pyridyl)hydrazine
¹H-NMR(CDCl₃) δ :6.22(1H, br s), 6.35(1H, br s), 6.73(1H, d, J=8.9Hz), 7.00-7.10(1H, m), 7.53(1H, dd, J=8.1, 1.8Hz), 7.72-7.80(1H, m), 8.13(1H, d, J=1.8Hz).

### Compound No. 2-6

1-(2,4-dichloro-5-methoxyphenyl)-2-(5-chloro-2-pyridyl)hydrazine
¹H-NMR(CDCl₃) δ :3.77(3H, s), 6.29(1H, br s), 6.38(1H, br s), 6.61(1H, s), 6.73(1H, d, J=8.8Hz), 7.30(1H, s), 7.51(1H, dd, J=8.8, 2.5Hz), 8.13(1H, d, J=2.5Hz).

### Compound No. 2-7

1-(4-chloro-5-ethoxycarbonyl-2-fluorophenyl)-2-(pyridazine-3-yl)hydrazine
¹H-NMR(CDCl₃) δ :1.22(3H, t, J=7.1Hz), 4.23(2H, q, J=7.1Hz), 6.96(1H, d, J=8.7Hz), 7.23(1H, d), 7.36-7.50(2H, m), 8.35(1H, br s), 8.60-8.65(1H, m), 8.95(1H, br s).

### Compound No. 2-8

1-(4-chloro-2-fluoro-5-isopropoxycarbonylphenyl)-2-( 5-chloro-2-pyridyl)hydrazine
¹H-NMR(CDCl₃) δ :1.32(6H, d, J=6.3Hz), 5.19(1H, m), 6.12(1H, br s), 6.33(1H, br s), 6.75(1H, d, J=8.8Hz), 7.14(1H, d, J=11.0Hz), 7.40(1H, d, J=9.0Hz), 7.50(1H, dd, J=8.8Hz), 8.11(1H, d, J=2.5Hz).

### Reference Example 12

### 1,3-Thiazolin-2-one(4-chloro-2-fluoro-5-propargyloxyphenyl)hydrazone hydrochloride (Compound No. 3-1)

2-Methylthio-2-thiazoline (0.26g, 2.0mmol) and 4-chloro-2-fluoro-5-propaygyloxyphenylhydrazine hydrochloride (0.5g, 2.0mmol) were dissolved with methanol(10ml), and the resulting mixture was refluxed for 1 hour. After cooling, acetone was added to the reaction mixture, and the crystal separated was collected by filtration and dried to give 1,3-thiazolin-2-one(4-chloro-2-fluoro-5-propargyloxyphenyl)hydrazone hydrochloride(0.31g).
¹H-NMR(CDCl₃) δ :2.50(1H, t, J=2.4Hz), 3.60-3.75(2H, m), 3.85-4.10(2H, m), 4.85(2H, d, J=2.4Hz), 6.75-6.90(1H, m), 7.45(1H, d, J=11.0Hz), 8.95(1H, br s).

### Reference Example 13

### 1-(7-Chloro-5-fluoro-2-methylbenzofuran-4-yl)-2-(5-chloro-2-pyridyl)hydrazine (Compound No. 3-2)

(1) 2-Chloro-4-fluoro-5-propargyloxyaniline (0.3g, 1.5mmol) and cesium fluoride (1.2g, 7.9mmol) were added to diethylaniline (4ml), and the resulting mixture was stirred at 200 °C for 4 hours. After cooling, the reaction mixture was purified by silicagel column chromatography(hexane:ethyl acetate=1:5→2:1) to give 4-amino-7-chloro-5-fluoro-2-methylbenzofuran(0.1g).
   ¹H-NMR(CDCl₃) δ :2.47(1H, d, J=1.1Hz), 3.80(2H,br s), 6.26(1H, d, J=1.1Hz),6.95(1H, d, J=10.9).
(2) A solution of the compound prepared in reference example 13-(1) (0.2g, 1.0mmol) and 5-chloro-2-nitrosopyridine prepared in reference example 10-(2) (0.2g, 1.4mmol) was dissolved in dichloroethane (8ml), and to the resulting mixture, trifluoroacetic acid (three drops) was added at room temperature, and then the resultant was stirred at the same temperature for 12 hours. To the reaction mixture, chloroform was added, and after washing with water and dring the resultant, the solvent was removed. To the residues, diisopropylether was added to obtain crystal.
   The obtained crystal was collected by filtration and dried to give 5-chloro-2-[(7-chloro-5-fluoro-2-methylbenzofuran-4-yl)azo]pyridine(0.2g).
   ¹H-NMR(CDCl₃) δ :2.45(3H,s), 7.20(1H, d, J=10.6Hz), 7.39(1H,s), 7.80-7.90(2H, m), 8.71(1H,s).
(3) The azo compound prepared in reference example 13 - (2) (0.2g, 0.62mmol) was dissolved in toluene (6ml), and tributyltin hydride (0.45g, 1.55mmol) was added to the resulting mixtue at room temperature, and then the resultant was stirred at 100 °C for 5 hours. After cooling. the reaction mixture was evaporated and purified by silicagel column chromatography(chloroform) to give 1-(7-chloro-5-fluoro-2-methylbenzofuran-4-yl)-2-(5-chloro-2-pyridyl)hydradine(0.11g).
   ¹H-NMR(CDCl₃) δ :2.45(3H,s), 7.20(1H, d, J=10.6Hz), 7.39(1H,s), 7.80-7.90(2H, m), 8.71(1H,s).

### Reference Example 14

### 1-(3,4-Dihydro-6-fluoro-1-propargyl-2(1H)-quinolinon-7-yl)-2-(5-chloro-2-pyridyl)hydrazine (Compound No. 3-3)

(1) 3-Fluoro-iodobenzene (1.0g, 4.5mmol), methyl acrylate (3.9g , 45.4mmol) and triethylamine (0.9g, 8.9mmol) were dissolved in acetonitrile (10ml), and to the resulting mixture, paradium acetate (catalyst quantity) was added at room temperature, and then the resultant was refluxed for 24 hours. After cooling, insoluble matter was removed by filtration and the filtrate was evaporated. To the obtained residue, ethyl acetate was added, and the solution was washed with diluted hydrochloric acid and dried, and then the solvent was removed. The obtained residue was purified by silicagel column chromatography (hexane: ethyl acetate=8:1) to give methyl 3-fluorocinnamate(0.7g).
   ¹H-NMR(CDCl₃) δ :3.81(3H,s), 6.43(1H, d, J=16.1Hz), 7.08-7.38(4H,m), 7.65(1H,d,J=16.1Hz).
(2) Methyl cinnamate (0.3g, 1.7mmol) prepared in reference example 14-(1) was dissolved in dried methanol (5ml), and the resultant was stirred at room temperature for 5 hours after adding magnesium (0.1g , 4.1mmol). After the reaction mixture was diluted with diluted hydrochloric acid, the resultant was extracted with ethyl acetate, and the extracted solution was washed with water and dried, and then the solvent was removed. As a result, methyl 3-fluoro-dihydrocinnamate (0.2g) was obtained.
   ¹H-NMR(CDCl₃) δ:2.63(2H,t, J=7.9Hz), 2.95 (2H, t, J=7.9Hz), 3.68(3H,s), 6.88-6.99(3H,m), 7.19-7.30(1H, m).
(3) Methyl dihydrocinnamate ester (0.7g, 3.8mmol) prepared in reference example 14-(2) was dissolved in concentrated sulfuric acid (5ml), and to the resulting mixture, nitric acid (65%, 0.8g, 8.3mmol) was added at room temperature, and then the resulting mixture was stirred at 40 °C for 1 hour. After cooling, the reaction mixture was added to water on ice, and then extracted with ether, the extract was washed with water and dried, and then the solvent was removed. As a result, methyl 3-fluoro-2,4-dinitro-dihydrocinnamate(0.5g) was obtained.
   ¹H-NMR(CDCl₃) δ :2.79(2H,t, J=7.1Hz), 3.34 (2H, t, J=7.1Hz), 3.71(3H,s), 7.45(1H,d,J=10.8Hz), 8.79(1H, d, J=8.0Hz).
(4) Methyl dinitrodihydrocinnamate (0.5g, crude) prepared in reference example 14-(3) was dissolved in a mixed solution of acetic acid (6ml) and water (1ml), and to the resulting mixture, reduced iron (0.5g ,8.9mmol) was added at room temperature, and then the mixture was stirred at 50 °C for 1 hour. After cooling, ethyl acetate and water were added to the reaction mixture, and insoluble material was removed by filtration. The organic layer was separated, washed with water and dried, and then the solvent was removed. Then ether was added to the obtained residue, and crystal was collected by filtration and dried to give 3,4-dihydro-7-amino-6-fluoro-2(1H)-quinolinone (0.15g).
   ¹H-NMR(CDCl₃) δ :2.58(2H,t, J=7.0Hz), 2.81 (2H, t, J=7.0Hz), 3.84(2H,br s), 6.30(1H,d,J=7.9Hz), 6.76(1H, d, J=10.8Hz),8.99(1H,br s).
(5)7-Aminoquinolinone prepared in reference example 14-(4) (0.2g, 1.1mol) and 5-chloro-2-nitrosopyridine prepared in reference example 10-(2) (0.2g, 1.4mmol) were dissolved in dichloroethane (8ml), and to the resulting mixture, trifluoro acetic acid(four drops) was added at room temperature, and then the mixture was stirred for 2 hours. The reaction mixture was evaporated, and the obtained residue was purified by silicagel column chromatography(hexane:ethyl acetate=1:10) to give 5-chloro-2-[(3,4-dihydro-7-amino-6-fluoro-2(1H)-quinolinon-7-yl)-azo]pyridine (0.13g).
   ¹H-NMR(CDCl₃) δ :2.68(2H,t, J=7.6Hz), 3.07 (2H, t, J=7.6Hz), 7.16(1H,d, J=10.1Hz), 7.30(1H,d,J=6.2Hz), 7.63(1H, br s),7.81-7.89(2H, m), 8.71(1H, d, J=2.4Hz).
(6) Azo compound prepared in reference example 14-(5) (0.13g, 0.43mmol) and potassium carbonate (0.24g, 1.74mmol) were suspended in acetonitrile (5ml), and to the resulting mixture, propargylbromide (0.22g, 1.85mmol) was added at room temperature, and then the mixture was stirred at 60 °C for 24 hours. After cooling, the reaction mixture was diluted with diluted hydrochloric acid and extracted with ethyl acetate. The extract was dried, and then the solvent was removed. The obtained residue was purified by silicagel column chromatography (chloroform:acetone=3:1) to give 5-chloro-2-[(3,4-dihydro-7-amino-6-fluoro-1-propargyl-2(1H)-quinolinon-7-yl)-azo]pyridine (0.065g).
   ¹H-NMR(CDCl₃) δ:2.22(1H,t, J=2.4Hz), 2.74 (2H, t, J=6.1Hz), 3.64(2H,t, J=6.1Hz), 4.75(1H,d,J=2.4Hz), 7.17(1H, d, J=9.9Hz),7.69(1H,d,J=6.1Hz),7.87-7.01(2H, m), 8.72(1H, s).
(7) Azo compound prepared in reference example 14-(6) (0.3g, 0.88mmol) was dissolved in toluene (20ml), and to the resulting mixture, tributyltin hydride (0.6g, 2.06mmol) was added at room temperature, and then the mixture was stirred at 80 °C for 1 hour. After cooling, the reaction mixture was evaporated, and hexane was added to the obtained residue, and then the precipitated crystal was collected by filtration and dried to give 1-(3,4-dihydro-6-fluoro-1-propargyl-2(1H)-quinolinon-7-yl)-2-(5-chloro-2-pyridyl)hydradine (0.28g).
   ¹H-NMR(CDCl₃) δ :1.97(1H,t, J=2.4Hz), 2.63 (2H, t, J=6.4Hz), 2.80(2H,t, J=6.4Hz), 4.55(2H,d,J=2.4Hz), 6.09(1H, br, s),6.34(1H, br s),6.79-6.90(3H, m), 7.47-7.51(1H, s), 8.12(1H, s).

### Reference Example 15

### 1,3-Thiazolin-2-one(2,5-difluoro-4-cyanophenyl) hydrazone (Compound No. 3-4)

2-Methylthio-2-thiazoline (1.0g, 7.52mmol), and 2,5-difluoro-4-cyanophenylhydrazine (1.27g, 7.51mmol) were dissolved in methanol (20ml), and to the resulting mixture, trifluoroacetic acid (four drops) was added at room temperature, and then the resultant was stirred at 55 °C for 48 hours. After cooling, the reaction mixture was evaporated, and the obtained residue was dissolved in ethyl acetate, washed with sodium bicarbonate solution and dried, and then the solvent was removed. The obtained residue was purified by silicagel column chromatography (chloroform:acetone=25:1) to give 1,3-thiazolin-2-one(2,5-difluoro-4-cyanophenyl) hydrazone(0.9g).
¹H-NMR(CDCl₃) δ :3.33(2H,t,J=6.6Hz), 3.70(2H, t, J=6.6Hz), 6.10(1H,br s), 6.60-6.78(1H,m), 7.09-7.17(1H, m).

### Reference Example 16

### 1,3-Thiazolin-2-one(7-fluoro-4-propargyl-3-oxo-1,4-benzoxazin-6-yl)hydrazone (Compound No. 3-5)

6-Amino-7-fluoro-4-propargyl-2H-1,4-benzoxazin-3(4H)-one (0.5g, 2.30mmol) was suspended in concentrated hydrochloric acid (6ml). To the solution, water solution(2ml) of sodium nitrite (0.2g, 2.9mmol) was dropped under cooling with ice and stirred at the same temperature for 30 minutes. On one hand, stannic chloride (1.5g, 7.9mmol) was dissolved in concentrated hydrochloric acid (6ml), and to the mixture, the above-mentioned solution was added dropwise by dropping pipet under cooling with ice. The resultant was stirred at the same temperature for 2 hours. The reaction mixturte was washed with ether, and the water-layer was concentrated to dryness. The residue was dissolved in methanol (10ml), and the solutions was refluxed for 3 hours after addition of 2-methylthiothiazoline (0.28g, 2.1mmol). After cooling, the reaction mixture was concentrated, and the residue was neutralized with sodium bicarbonate, and then the precipitated crystal was collected by filtration and dried to give 1,3-thiazolin-2-one(7-fluoro-4-propargyl-3-oxo-1,4-b enzoxazin-6-yl)hydrazone(0.4g).
¹H-NMR(CDCl₃) δ :2.26(1H,t,J=2.4Hz), 3.28(2H, t, J=6.5Hz), 3.68(2H, t, J=6.5Hz), 4.55(2H,d), 4.67(2H, d, J=2.4Hz), 6.25(1H, br s), 6.73(1H, d, J=11.1Hz), 6.97(1H, d, J=8.0Hz).

### Reference Example 17

### 2H-Tetrahydro-1,3-thiazin-2-one(2,4-difluoro-5-cyanophenyl)hydrazone (Compound No. 3-6)

(1) 3-Amino-1-propanol (10.0g, 0.13mol) and triethylamine (19g, 0.19mol) were dissolved in pyridine (80ml). To the mixture, carbon disulfide (21.0g, 0.28mol) was dropped under cooling with ice, and the resultant was stirred at the same temperature for 90 minutes. Then methyl iodide (20.0g, 0.14mol) was added dropwise under cooling with ice and the mixture was stirred at room temperature for 2 hours. The reaction mixture was added to icewater, and the resultant was neutralized with hydrochloric acid, and then extracted with ether. The combined ether extract was dried, and then the solvent was removed to give methyl N-(3-hydroxypropyl)dithiocarbamate(11.0g) was obtained.
   ¹H-NMR(CDCl₃) δ :1.80-1.95(2H, m), 2.62(3H,s), 3.75-3.90(2H,m), 3.90-4.00(2H,m), 7.70(1H,br s).
(2)Methyl dithiocarbamate(11.0g, 66.7mmol) prepared in referance example 17-(1) was dissolved in ether(20ml). To the mixture, thionyl chloride (31.0g, 0.26mol) in ether(50ml) solution was added dropwise, and the mixture was stirred at temperature for 12 hours. The reaction mixture was added to icewater and then neutralized with sodium bicarbonate, and the resultant was extracted with diethyl ether. The ether extract was dried, and the solvent was removed (6.5g). The obtained residue (2.0g) was dissolved in 2-propanol (20ml), to the mixture, 5-cyano-2,4-difluorophenylhydrazine (1.0g, 5.9mmol) was added at room temperature and then stirred at 80 °C for 12 hours. After cooling, the reaction mixture was evaporated, and ether was added to the residue. The resultant solution was washed with sodium bicarbonate and dried, and then the solvent was removed. The obtained residue was purified by silicagel column chromatography (hexane: ethyl acetate=1:1) to give 2H-tetrahydro-1,3-thiazin-2-one(2,4-difluoro-5-cyanophenyl)hydrazone(0.6g).
   ¹H-NMR(CDCl₃) δ :2.10-2.23(2H, m), 3.03(2H,t, J=6.2Hz), 3.39(2H,t, 5.9Hz), 6.60(1H,br s), 6.75-6.90(1H, m).7.07-7.15(1H, m).

### Examples

### Example 1

### 2-(4-Cyano-5-ethanesulfonylamino-2-fluorophenyl)-1,2, 4-triazolo[4,3-a]pyridin-3(2H)-one (Compound No. A-1)

To a suspension of Compound No.1-10(1.0g, 3.6mmol) and ethanesulfonamide(0.44g, 4.0mmol) in DMSO(10ml), potassium carbonate(0.55g, 4.0mmol) was added, and the resulting mixture was stirred at 60 °C for 6 hours and at 100 °C for 16 hours. After cooling, the reaction mixture was added to icewater and neutralized with dilute hydrochloric acid, and the crystal separated was collected by filtration. The crystal obtained was dissolved with ethyl acetate, and the solution was washed with water and evaporated. The mixture of hexane and ethyl acetate(1:1) was added to the residue, and the crystal separated was collected by filtration, to give 2-(4-cyano-5-ethanesulfonylamino-2-fluorophenyl)-1,2 ,4-triazolo[4,3-a]pyridin-3(2H)-one (0.23g).
¹H-NMR(CDCl₃) δ 1.46(3H, t, J=7.43Hz), 3.26(2H, q, J=7. 31Hz), 6.57(1H, m), 7.12-7.30(2H, m), 7.56(1H, q, J=9. 26Hz), 7.81(1H, d, J=7.15Hz), 8.14(1H, d, J=6.44Hz).
mp:not more than 215 °C.

### Example 2

### 6-Chloro-2-(4-cyano-5-ethanesulfonylamino-2-fluorophenyl)-1,2,4-triazolo[4,3-a]pyridin-3(2H)-one (Compound No. A-3)

To a solution of Compound No.1-1(40g, 0.13mol) and ethanesulfonamide(40g, 0.367mol) in DMF(450ml), potassium fluoride(40g, 0.69mol), almina(50g) and 18-crown-6(4g, 15mmol) were added, and the resulting mixture was stirred at 95 °C for 48 hours. After cooling, the reaction mixture was added to icewater, neutralized with dilute hydrochloric acid and extracted with ethyl acetate. The extract was washed with water and evaporated. The residue was purified by silicagel column chromatography(twice)(chloroform:acetone:hexane=3:1: 5), to give 6-chloro-2-(4-cyano-5-ethanesulfonylamino-2-fluorophenyl)-1,2,4-triazolo[4,3-a]pyridin-3(2H)-one (5.5g).
¹H-NMR(CDCl₃) δ 1.46(3H, t, J=7.34Hz), 3.26(2H, q, J=7.42Hz), 7.12-7.20(2H, m), 7.53(1H, d, J=9.14Hz), 7.85(1H, s), 8.12(1H, d).

### Example 3

### 6-Chloro-2-[4-cyano-5-(N-ethanesulfonyl-N-propargylamino)-2-fluorophenyl]-1,2,4-triazolo[4,3-a]pyridin-3(2H)-one (Compound No. A-5)

To a suspension of Compound No.A-3(0.25g, 0.63mol) and potassium carbonate(0.11g, 0.92mmol) in DMF(10ml), propargylbromide(0.11g, 0.92mmol) was added, and the resulting mixture was stirred at room temperature for 24 hours. The reaction mixture was added to icewater, the crystal separated was collected by filtration, washed with dilute hydrochloric acid and water and dried to give 6-chloro-2-[4-cyano-5-(N-ethanesulfonyl-N-propargylamino)-2-fluorophenyl]-1,2,4-triazolo[4,3-a]pyridin-3(2H)-one (0.21g).
¹H-NMR(CDCl₃) δ 1.52(3H, t, J=7.4Hz), 2.44(2H, q, J=7.4Hz), 3.31(2H, q, J=7.4Hz), 4.52(2H, d, J=2.4Hz), 7.10-7.17(2H, m), 7.63(1H, d, J=9.4Hz), 8.11(1H, d, J=6.5Hz).
mp:176-178 °C.
IR(Nujol;cm⁻¹) 3292, 2233, 1721.

### Example 4

### 2-[5-(N-acetyl-N-ethanesulfonylamino)-4-cyano-2-fluorophenyl]-6-chloro-1,2,4-triazolo[4,3-a]pyridin-3(2H)-one (Compound No. A-6)

To a solution of Compound No.1-1(0.25g, 0.63mmol) and triethylamine(0.14g, 1.38mmol) in acetonitrile, acetyl chloride(80mg, 1.02mmol) was added, and the resulting mixture was stirred at room temperature for 24 hours. After the reaction mixture was evaporated, water was added to the residue, and the crystal separated was collected by filtration, washed with water and dried to give 2-[5-(N-acetyl-N-ethanesulfonylamino)-4-cyano-2-fluorophenyl]-6-chloro-1,2,4-triazolo[4,3-a]pyridin-3(2H)-one (0.26g).
¹H-NMR(CDCl₃) δ 1.56(3H, t, J=7.5Hz), 2.15(3H, s), 3.60-3.95(2H, m), 7.13-7.17(2H, m), 7.69(1H, d, J=9.3Hz), 7.86(1H, s), 7.99(1H, d, J=6.4Hz).
mp:220-222 °C.
IR(Nujol;cm⁻¹) 2221, 1737.

### Example 5

### 2-(5-Allyloxy-4-cyano-2-fluorophenyl)-6-chloro-1,2,4-triazolo[4,3-a]pyridin-3(2H)-one (Compound No. A-13)

(1)To a solution of compound No.1-3(0.1g, 0.31mmol) in dichloromethane(3ml), boron tribromide(dichloromethane solution, 1.0M, 0.4ml) was added at room temperature, and the resulting mixture was stired at same temperature for 1.5 hours. Since unreacting material was found, further boron tribromide(dichloromethane solution, 0.4ml) was added, and the resulting mixture was stirred at room temperature for 14 hours. The reaction mixture was added to icewater, and resulting mixture was stirred for 30 minutes. The crystal separated was collected by filtration, washed with water and dried to give 6-chloro-2-(4-cyano-2-fluoro-5-hydroxyphenyl)--1,2,4-triazolo[4,3-a]pyridin-3(2H)-one(60mg).
   ¹H-NMR(DMSO-d₆) δ 7.30-7.40(3H, m), 7.91(1H, d, J=10.5Hz), 7.21(1H, s), 11.56(1H, s).
   mp:>260 °C.
(2) To a suspension of the compound prepared in example 5-(1)(0.15g, 0.49mmol) and potassium carbonate (81mg, 0.59mmol) in DMF(10ml), allylbromide(90mg, 0.74mmol) was added, and the resulting mixture was stirred at 50 °C for 2 hours. After cooling, the reaction mixture was neutralized with dilute hydrochloric acid, and the crystal separated was collected by filtration, washed with water and dried to give 2-(5-allyloxy-4-cyano-2-fluorophenyl)-6-chloro-1,2,4 -triazolo[4,3-a]pyridin-3(2H)-one(0.11g).
   ¹H-NMR(CDCl₃) δ 4.68(2H, d, J=5.2Hz), 5.35-5.54(2H, m), 5.91-6.10(1H, m), 7.31(1H, d, J=5.7Hz), 7.48(1H, d, J=9.4Hz), 7.87(1H, s).
   mp:183-185 °C.
   IR(Nujol;cm⁻¹) 2233, 1716.

### Example 6

### 6-Chloro-2-(4-cyano-2-fluoro-5-propargyloxyphenyl)-1,2,4-triazolo[4,3-a]pyridin-3(2H)-one (Compound No. A-14)

To a solution of the propargylalchol (55mg, 0.98mmol) in acetonitrile (5ml), sodium hydride (60% in oil, 40mg, 1.0mmol) was added at room temperature, and the resulting mixture was stirred at same temperature for 30 mimutes. Then, compound No.1-1 (0.2g, 0.65mmol) was added to above-mentioned solution, and the resulting mixture was stirred at room temperature for 20 hours. Dilute hydrochloric acid was added to the reaction mixture, and the crystal separated was collected by filtration and dried, and then the obtained crystal was purified by silicagel column chromatography (chloroform) to give 6-chloro-2-(4-cyano-2-fluoro-5-propargyloxyphenyl)-1 ,2,4-triazolo[4,3-a]pyridin-3(2H)-one(50mg).
¹H-NMR(CDCl₃) δ 2.61(1H, t, J=2.4Hz), 4.86(2H, d, J=2.4Hz), 7.15-7.16(2H, m), 7.48-7.55(2H, m), 7.78(1H, s).
mp:209-211 °C
IR(Nujol;cm⁻¹ ) 2236, 1724.

### Example 7

### 6-Chloro-2-(4-cyano-2-fluoro-5-isopropylthiophenyl)-1,2,4-triazolo[4,3-a]pyridin-3(2H)-one (Compound No. A-18)

To a suspension of the compound No.1-1 (0.2g, 0.65mmol) and potassium carbonate (0.27g, 1.95mmol) in DMSO (10ml), 2-propanethiol (75mg, 0.98mmol) was added, and the resulting mixture was stirred at 50 °C for 4 hours. After cooling, the reaction mixture was neutralized with dilute hydrochloric acid, and extracted with ethyl acetate. The extract was dried and evaporated. Hexane was added to the residue, and the crystal separated was collected by filtration and dried to give 6-chloro-2-(4-cyano-2-fluoro-5-isopropylthiophenyl)-1,2,4-triazolo[4,3-a]pyridin-3(2H)-one(0.13g).
¹H-NMR(CDCl₃) δ 1.37(6H, d, J=6.7Hz), 3.45-3.65(1H, m), 7.14-7.16(2H, m), 7.56(1H, d, J=9.6Hz), 7.85-7.89(2H, m).
mp:148-150 °C.
IR(Nujol;cm⁻¹) 2227, 1718.

### Example 8

### 6-Chloro-2-(4-chloro-2-fluoro-5-methoxyphenyl)-1,2,4-triazolo[4,3-a]pyridin-3(2H)-one (Compound No. A-23)

(1) To a solution of cyanoethanol (1.1g, 16mmol) in acetonitrile (15ml), sodium hydride(60% in oil, 0.64g, 9.6mmol) was added at room temperature, and the resulting mixture was stirred at same temperature for 30 mimutes. Then, compound No.1-4 (1.0g, 3.2mmol) was added to the above-mentioned solution, and the resulting mixture was stirred at room temperature for 30 minutes. The reaction mixture was neutralized with dilute hydrochloric acid. The crystal separated was collected by filtration, and then dissolved in chloroform, and evaporated after dryness. Diisopropyl ether was added to the residue, and the crystal separated was collected by filtration and dried to give 6-chloro-2-(2-fluoro-5-hydroxy-4-nitrophenyl)-1,2,4-triazolo[4,3-a]pyridin-3(2H)-one (600mg).
   ¹H-NMR(CDCl₃) δ 7.15-7.16(2H, m), 7.60(1H, d, J=6.2Hz), 7.87(1H, s), 8.04(1H, d, J=10.1Hz), 10.44(1H, s).
   mp:207-209 °C.
   IR(Nujol;cm⁻¹) 3100, 1732.
(2)To a solution of the compound prepared in example 8-(1)(0.35g, 1.2mmol) in DMF(20ml), sodium hydride(60% in oil, 96mg, 2.4mmol) was added at room temperature, and the resulting mixture was stirred at same temperature for 30 mimutes. Then, iodomethane (3.4g, 23.9mmol) was added to the above-mentioned solution, and the resulting mixture was stirred at room temperature for 36 hours. The reaction mixture was neutralized with dilute hydrochloric acid, and the crystal separated was collected by filtration. The obtained crystal was dissolved in ethyl acetate, and the solution was concentrated after dryness. To the residue, Hexane was added to make crystallization, and the crystal was collected by filtration and dried to give 6-chloro-2-(2-fluoro-5-methoxy-4-nitrophenyl)-1,2,4-triazolo[4,3-a]pyridin-3(2H)-one(0.2g).
   ¹H-NMR(CDCl₃) δ 4.00(3H, s), 7.15-7.16(2H, m), 7.47(1H, d, J=5.9Hz), 7.85-7.90(2H, m).
   mp:164-166 °C.
   IR(Nujol;cm⁻¹) 1722.
(3)The compound prepared in example 8-(2)(0.6g, 1.8mmol) was added to the mixed solution of acetic acid (3ml) and water(1ml), and to this mixture at room temperature, reduced iron(0.3g, 5.4mmol) was added gradually. The resulting mixture was stirred at 70 °C for 40 minutes. After cooling, the reaction mixture was neutralized with sodium bicarbonate water and extracted with ethyl acetate. After insoluble part was filtered off on celite, the extract was dried and concentrated. Hexane was added to the residue, and the crystal was collected by filtration and dried to give 2-(4-amino-2-fluoro-5-methoxyphenyl)-6-chloro-1,2,4-triazolo[4,3-a]pyridin-3(2H)-one(0.4g).
   ¹H-NMR(CDCl₃) δ 3.85(3H, s), 6.60(1H, d, J=9.5Hz), 6.87(1H, d, J=6.6Hz), 7.05-7.15(2H, m), 7.88(1H, m).
   mp:138-140 °C.
(4) To a solution of the compound prepared in example 8-(3)(0.2g, 0.64mmol) in concentrated hydrochloric acid (10ml), cuprous chloride (20mg) was added, and then an aqueous solution of sodium nitrite (0.1g. 1.45mmol) was added dropwise under cooling with ice. The resulting mixture was stirred at same temperature for 30 minutes and at 90 °C for 1 hour. After cooling, the reaction mixture was diluted with water and extracted with ethyl acetate. The extract was dried and evaporated. Hexane was added to the residue, and the crystal was collected by filtration and dried to give 6-chloro-2-(4-chloro-2-fluoro-5-methoxyphenyl)-1,2,4-triazolo[4,3-a]pyridin-3(2H)-one(0.2g).
   ¹H-NMR(CDCl₃) δ 3.92(3H, s), 7.12-7.16(3H, m), 7.34(1H, d, J=9.4Hz), 7.88(1H, m).
   mp:140-142 °C
   IR(Nujol;cm⁻¹) 1725.

### Example 9

### 6-Chloro-2-(4-chloro-2-fluoro-5-methoxyphenyl)-1,2,4-triazolo[4,3-a]pyridin-3(2H)-one (Compound No. A-23)

To a solution of triphosgene (98mg, 0.33mmol) in THF (10ml), pyridine(79mg, 1.0mmol) was added dropwise, and the resulting mixture was stirred for 30 minutes. While, hydrazine of compound No.2-1 (0.1g, 0.33mmol) and triethylamine (0.1g, 1.0mmol) are dissolved in THF (5ml), this solution was added dropwise to the above-mentioned phosgene solution at room temperature, and the resulting mixture was stirred for 30 minutes. Furthermore at room temperature, triethylamine (0.24g, 2.0mmol) was added to the reaction mixture, THF (10ml) solution concluding triphosgene(0.2g, 0.66mmol) and pyridine(0.16g, 3.0mmol) was further added to the reaction mixture, and the resulting mixture was stirred for 30 minutes. Dilute hydrochloric acid was added to the reaction mixture, and the resulting mixture was extracted with ethyl acetate, and the extract was dried and concentrated. Diisopropyl ether was added to the residue, and the crystal separated was collected by filtration and dried to give 6-chloro-2-(4-chloro-2-fluoro-5-methoxyphenyl)-1,2,4-triazolo[4,3-a]pyridin-3(2H)-one(33mg).
¹H-NMR(CDCl₃), mp and IR are the same as example 8.

### Example 10

### 6-Chloro-2-(4-chloro-2-fluoro-5-propargyloxyphenyl)-1,2,4-triazolo[4,3-a]pyridin-3(2H)-one (Compound No. A-25)

(1) To a solution of the compound prepared in example 8-(4)(0.35g, 1.07mmol) in dichloromethane(15ml), boron tribromide(dichloromethane solution, 1.0M, 2.1ml, 2.1mmol) was added dropwise at room temperature, and the resulting mixture was stired at same temperature for 4 hours. The reaction mixture was added to icewater, and resulting mixture was stirred for 30 minutes, neutralized with sodium bicarbonate water and filtered on celite. The organic layer in filtrate was separated, and the aqueous layer was extracted with chloroform. The organic layer and chloroform extract was combined, dried, and then concentrated. Diisopropyl ether was added to the residue, and the crystal separated was collected by filtration and dried to give 6-chloro-2-(4-chloro-2-fluoro-5-hydroxyphenyl)-1,2,4-triazolo[4,3-a]pyridin-3(2H)-one(0.18g).
   ¹H-NMR(CDCl₃) δ 5.80(1H,br s), 7.05-7.20(3H, m), 7.30-7.35(1H, m), 7.87(1H, s).
   mp:>210 °C
(2) To a suspension of the compound prepared in example 10-(1)(0.1g, 0.32mmol) and potassium carbonate(60mg, 0.43mmol) in DMF(5ml), propargylbromide(57mg, 0.48mmol) was added, and the resulting mixture was stirred at 60 °C for 2 hours. After cooling, the reaction mixture was neutralized with dilute hydrochloric acid and extracted with ethyl acetate, and the extract was dried and concentrated. The residue was purified by silicagel column chromatography (hexane:ethyl acetate=1:2), to give 6-chloro-2-(4-chloro-2-fluoro-5-propargyloxyphenyl)-1,2,4-triazolo[4,3-a]pyridin-3(2H)-one(50mg).
   ¹H-NMR(CDCl₃) δ 2.58(1H, t, J=2.4Hz), 4.79(2H, d, J=2.4Hz), 7.12-7.14(2H, m), 7.34(1H, d, J=6.3Hz), 7.35(1H, d, J=9.5Hz), 7.87(1H, s).
   mp:145-148 °C
   IR(Nujol;cm⁻¹) 1720.

### Example 11

### Isopropyl 2-chloro-4-fluoro-5-(6-chloro-2,3-dihydro-3-oxo-1,2,4-triazolo[4,3-a]pyridin-2-yl)benzoate (Compound No. A-33)

Compound no.2-8 (0.8g, 2.23mmol) and triethylamine(2.7g, 26.6mmol) were dissolved in THF(15ml). While, to a solution of triphosgene (2.65g, 2.23mmol) in THF(15ml), pyridine (2.11g, 26.7mmol) was added dropwise at room temperature, and the resulting mixture was stirred for 30 minutes. This suspension was added dropwise to above-mentioned hydrazine solution by dropping pipet. After the reaction mixture was stirred at room temperature for 12 hours, the reaction mixture was added to ice water, and THF was evaporated. The crystal separated was collected by filtration, washed with water and dried to give isopropyl 2-chloro-4-fluoro-5-(6-chloro-2,3-dihydro-3-oxo-1,2,4-triazolo[4,3-a]pyridin-2-yl)benzoate(0.63g).
¹H-NMR(CDCl₃) δ 1.38(6H, d, J=6.2Hz), 5.27(1H, m), 7.40(1H, d, J=9.7Hz), 7.88(1H, s), 8.13(1H, d, J=7.8Hz).
mp:120-123 °C
IR(Nujol;cm⁻¹) 1735, 1706.

### Example 12

### Ethoxycarbonylmethyl 2-chloro-4-fluoro-5-(6-chloro-2,3-dihydro-3-oxo-1,2,4-triazolo[4,3-a]pyridin-2-yl)benzoate (Compound No. A-34)

(1) To a solution of the compound prepared in example 11(50mg, 0.13mmol) in dichloromethane (3ml), boron tribromide(dichloromethane solution, 1.0M, 0.39ml) was added at room temperature, and the resulting mixture was stired for 24 hours. Water was added to the reaction mixture, and dichloromethane was evaporated. The crystal separated was collected by filtration, washed with water and diethyl ether, and dried to give 2-chloro-4-fluoro-5-(6-chloro-2,3-dihydro-3-oxo-1,2,4-triazolo[4,3-a]pyridin-2-yl)benzoic acid(20mg).
   ¹H-NMR(CDCl₃) δ 7.36-7.44(2H, m), 7.89(1H, d, J=10.3Hz), 8.14-8.21(2H, m).
(2) Diethyl azodicarboxylate(40%toluene solution, 290mg, 0.66mmol) and triphenylphosphine(0.173g, 0.66mmol) were dissolved in toluene. Then, the carboxylic acid prepared in example 12-(1)(0.15g, 0.44mmol) and ethyl glycolate(0.92mg, 0.88mmol) were added to above-mentioned solution at room temperature, and the resulting mixture was stirred for 3 hours. Furthermore, diethyl azodicarboxylate (40% toluene solution, 430mg) and triphenylphosphine (260mg) were added to the reaction mixture at room temperature, and the resulting mixture was stirred for 1 hour. The reaction mixture was concentrated, and the residue was purified by silicagel column chromatography (hexane:ethyl acetate=2:1) to give ethoxycarbonylmethyl 2-chloro-4-fluoro-5-(6-chloro-2,3-dihydro-3-oxo-1,2,4-triazolo[4,3-a]pyridin-2-yl)benzoate(80mg).
   ¹H-NMR(CDCl₃) δ 1.30(3H, t, J=7.0Hz), 4.26(2H, q, J=7.0Hz), 4.85(2H, s), 7.10-7.15(2H, m), 7.44(1H, d, J=9.6Hz), 7.87(1H, s), 8.33(1H, d, J=7.8Hz).
   mp:62-65 °C
   IR(Nujol;cm⁻¹) 1734.

### Example 13

### 6-Chloro-2-(4-chloro-5-ethanesulfonylamino-2-fluorophenyl)-1,2,4-triazolo[4,3-a]pyridin-3(2H)-one (Compound No. A-35)

(1) To a solution of the compound No.2-2(0.6g, 1.89mmol) in THF(20ml), triethylamine(2.87g, 28.4mmol) was added. While, triphosgene (2.8g, 9.46mmol) was dissolved in THF (15ml), to this solution at room temperature, pyridine(2.24g, 28.4mmol) was added dropwise, and the resulting mixture was stirred 30 minutes. This suspension was added dropwise to above-mentioned hydrazine solution by dropping pipet. After the reaction mixture was stirred at room temperature for 12 hours, the reaction mixture was added to ice water, and the crystal separated was collected by filtration, washed with water and dried to give 6-chloro-2-(4-chloro-2-fluoro-5-nitrophenyl)-1,2,4-triazolo[4,3-a]pyridin-3(2H)-one(0.6g).
   ¹H-NMR(CDCl₃) δ 7.14-7.15(2H, m), 7.51(1H, d, J=9.4Hz), 7.87(1H, s), 8.36(1H, d, J=6.9Hz).
   mp:210-212 °C
(2) The compound prepared in example 13-(1)(0.3g, 0.87mmol) was added to the mixed solution of acetic acid(5ml) and water(0.5ml), and reduced iron (0.25g, 4.46mmol) was added to the solution. The resulting mixture was stirred at 80 °C for 3 hours. After cooling, the reaction mixture was diluted with ethyl acetate, neutralized with sodium bicarbonate water and filtered on celite. The organic layer in filtrate was separated, dried and concentrated. Hexane was added to the residue, and the crystal separated was collected by filtration and dried to give 2-(5-amino-4-chloro-2-fluorophenyl)-6-chloro-1,2,4-t riazolo[4,3-a]pyridin-3(2H)-one(0.12g).
   ¹H-NMR(CDCl₃) δ 4.10(2H, br s), 6.99(1H, d, J=6.7Hz), 7.10-7.15(2H, m), 7.23(1H, d, J=9.4Hz), 7.87(1H, s).
   mp:247-250 °C
(3) To a solution of the compound prepared in example 13-(2)(0.18g, 0.58mmol) in pyridine(5ml), ethanesulfonylchloride (0.23g, 1.79mmol) was added, and the resulting mixture was stirred for 24 hours. The reaction mixture was diluted with water, and extracted with ethyl acetate. The extract was washed with water, dried, and then concentrated. The residue was purified by silicagel column chromatography(hexane:ethyl acetate=1:2→1:1), to give 6-chloro-2-(4-chloro-5-ethanesulfonylamino-2-fluorophenyl)-1,2,4-triazolo[4,3-a]pyridin-3(2H)-one (0.1g).
   ¹H-NMR(CDCl₃) δ 1.40(3H, s), 3.17(2H, q, J=7.4Hz), 6.74(1H, br s), 7.12-7.14(2H, m), 7.38(1H, d, J=9.1Hz), 6.86(1H, s), 7.98(1H, d, J=7.0Hz).
   mp:120-122 °C
   IR(Nujol;cm⁻¹) 3100, 1711.

### Example 14

### 2-Chloro-4-fluoro-5-(6-chloro-2,3-dihydro-3-oxo-1,2,4-triazolo[4,3-a]pyridin-2-yl)propionanilide (Compound No. A-36)

The compound prepared in example 13-(2)(0.18g, 0.58mmol) and triethylamine(59mg, 0.58mmol) were dissolved in THF(20ml), propionyl chloride(54mg, 0.58mmol) was added to above-mentioned solution at room temperature, and the resulting mixture was stirred at same temperature for 1 hour. The reaction mixture was neutralized with dilute hydrochloric acid, and the crystal separated was collected by filtration, washed with water and dried to give 2-chloro-4-fluoro-5-(6-chloro-2,3-dihydro-3-oxo-1,2,4-triazolo[4,3-a]pyridin-2-yl)propionanilide (0.15g).
¹H-NMR(CDCl₃) δ 1.26(3H, t, J=7.6Hz), 2.47(2H, q, J=7.5Hz), 7.09-7.12(2H, m), 7.33(1H, d, J=9.3Hz), 7.55(1H, br s), 7.86(1H, s), 8.75(1H, d, J=7.6Hz).
mp:210-213 °C
IR(Nujol;cm⁻¹) 3524, 1720, 1661.

### Example 15

### 5-(6-Chloro-2,3-dihydro-3-oxo-1,2,4-triazolo[4,3-a] pyridin-2-yl)-2-cyano-4-fluoro-N-propionylbenzenesul fonamide (Compound No. A-20)

(1)Compound No.1-1(0.5g, 1.63mmol) and potassium carbonate(0.338g, 2.45mmol) were dissolved in dioxane (25ml), to this solution, benzylmercaptane (0.3g, 2.45mmol) was added, and the resulting mixture was stirred at 80 °C. Potassium carbonate (0.113g, 0.82mmol) and benzylmercaptane (0.101g, 0.81mmol) were added to the reaction mixture 3 times every 12 hours. After cooling, the reaction mixture was concentrated, water was added to the residue, and this solution was extracted with chloroform. The extract was dried and then concentrated. The residue was crystallized by diisopropyl ether, and the separated crystal was collected by filtration, washed with water and dried to give 2-(5-benzylthio-4-cyano-2-fluorophenyl)-6-chloro-1,2,4-triazolo[4,3-a]pyridine-3(2H)-one(0.4g).
   ¹H-NMR(CDCl₃) δ 4.23(2H, s), 7.13-7.15(2H, m), 7.26-7.35(5H, m), 7.50(1H, d, J=9.9Hz), 7.80(1H, d), 7.86(1H, s).
(2) The compound prepared in example 15-(1)(0.5g, 1.22mmol) was suspended to the mixed solution of acetic acid(6ml) and water(6ml). After bubbling chlorine gas to above suspension under -10 °C and confirming consumption of benzylthio compound on TLC, the resulting mixture was diluted in water. The crystal separated was collected by filtration and dissolved in dichloromethane (10ml), and the solution was dried. This dichloromethane solution was added dropwise to ammonia-THF solution (5%, 5ml) prepared separately, and the resulting mixture was stirred at room temperature for 30 minutes. After the reaction mixture was evaporated, water and diisopropyl ether were added to the residue, and the crystal separated was collected by filtration and dried to give 5-(6-chloro-2,3-dihydro-3-oxo-1,2,4-triazolo[4,3-a] pyridin-2-yl)-2-cyano-4-fluorobenzenesulfonamide (0.16g).
   ¹H-NMR(acetone-d₆) δ 7.25(2H, br s), 7.34-7.35(2H, m), 8.01(1H, s), 8.15(1H, d, J=10.2Hz), 8.15(1H, d).
(3) The compound prepared in example 15-(2)(0.2g, 0.54mmol) and triethylamine(0.11g, 1.10mmol) were suspended to dichloromethane(10ml). To this suspention at room temperature, propionyl chloride(75mg, 0.81mmol) was added, and the resulting mixture was stirred at same temperature for 30 minutes. The reaction mixture was added to water, neutralized with dilute hydrochloric acid, and the organic layer was separated, washed with water, dried and then concentrated. The residue was purified by silicagel column chromatography(hexane:ethyl acetate=1:2), to give 5-(6-chloro-2,3-dihydro-3-oxo-1,2,4-triazolo[4,3-a] pyridin-2-yl)-2-cyano-4-fluoro-N-propionylbenzenesulfonamide(60mg).
   ¹H-NMR(CDCl₃) δ 1.03(3H, t, J=7.3Hz), 2.31(1H, q, J=7.3Hz), 7.10-7.15(2H, m), 7.68(1H, d, J=9.4Hz), 7.87(1H, s), 8.62(1H, d, J=6.6Hz).
   mp:190-194 °C
   IR(Nujol;cm⁻¹) 3200-3100, 2100, 1725, 1713.

### Example 16

### 5-(6-Chloro-2,3-dihydro-3-oxo-1,2,4-triazolo[4,3-a] pyridin-2-yl)-2-cyano-4-fluoro-N-methylbenzenesulfonamide (Compound No. A-21)

The compound prepared in example 15-(1)(0.5g, 1.22mmol) was suspended to the mixed solution of acetic acid (6ml) and water (6ml). After bubbling chlorine gas under -10 °C and confirming consumption of benzylthio compound on TLC, the resulting mixture was diluted in water, the crystal separated was collected by filtration, the obtained crystal was dissolved in dichloromethane (10ml), and the solution was dried. Separately to a suspension of methylamine hydrochloride (0.33g, 4.89mmol) in dichloromethane, triethylamine (0.49g, 4.9mmol) was added under cooling with ice, and the resulting mixture was stirred at room temperature for 30 minutes. Above-obtained sulfonylchloride solution was added dropwise to this methylamine solution at room temperature, and the resulting mixture was stirred for 1 hour. After the reaction mixture was evaporated, the residue was purified by silicagel column chromatography(chloroform:methanol=10:1), to give 5-(6-chloro-2,3-dihydro-3-oxo-1,2,4-triazolo[4,3-a] pyridin-2-yl)-2-cyano-4-fluoro-N-methylbenzenesulfonamide (80mg).
¹H-NMR(CDCl₃) δ 3.18(3H, s), 7.35-7.50(3H, m), 8.28(1H, s), 8.53(1H, d, J=7.0Hz).
mp:154-157 °C
IR(Nujol;cm⁻¹) 3200, 2200, 1712.

### Example 17

### 6-Chloro-2-(7-fluoro-3-oxo-4-propargyl-2H-1,4-benzox azin-6-yl)-1,2,4-triazolo[4,3-a]pyridine-3(2H)-one (Compound No. A-57)

(1) Compound No.2-5(1.5g, 5.3mmol) and triethylamine(8.0g, 79.1mmol) were dissolved in THF(70ml). While, to a solution of triphosgene (7.8g, 26.3mmol) in THF (50ml), pyridine (6.3g, 79.6mmol) was added dropwise at room temperature. This phosgene solution was added to above-abtained hydrazine solution at room temperature by dropping pipet, and the resulting mixture was stirred at same temperatrure for 2 days. The reaction mixture was diluted with dilute hydrochloric acid, and THF was evaporated. The crystal separated was colleceted by filtration, washed with water, dried and then washed with diethyl ether, to give 6-chloro-2-(2,4-difluoro-5-nitrophenyl)-1,2,4-triazolo[4,3-a]pyridin-3(2H)-one(1.6g).
   ¹H-NMR(CDCl₃) δ 7.15-7.17(2H, m), 7.21-7.32(2H, m), 7.88(1H, s), 8.45-8.53(1H, m).
(2) To a solution of the compound prepared in example 17-(1)(0.5g, 1.53mol) in dioxane(20ml), methyl glycolate ester (1.26g, 12.1mmol) and potassium fluoride(1.08g, 18.6mmol) were added, and the resulting mixture was stirred at 90-110°C for 24 hours. After cooling, the reaction mixture was diluted in water, dioxane was evaporated. The crystal separated was collected by filtration washed with water, furthermore washed with diethyl ether and dried to give ethyl 5-fluoro-2-nitro-4-(6-chloro-2,3-dihydro-3-oxo-1,2,4-triazolo[4,3-a]pyridin-2-yl)phenoxyacetate(0.6 g).
   ¹H-NMR(CDCl₃) δ 1.32(3H, t, J=7.2Hz), 4.30(2H, q, J=7.2Hz), 4.82(2H, s), 6.89(1H, d, J=11.0Hz), 7.13-7.15(2H, s), 7.87(1H, s), 8.29(1H, d, J=7.5Hz).
(3)To a suspension of reduced iron (0.5g, 19.0mmol) in the mixed solution of acetic acid (2ml) and water (10ml), the compound prepared in example 17-(2) (0.5g, 19.0mmol) was added gradually at 50 °C, and the resulting mixture was stirred at 60 °C for 1 hour. After cooling, the insoluble material was collected by filtration, and dried and dissolved in DMF, and then further the insoluble of the solution was filtered off. The filtrate was diluted with dilite hydrochloric acid, and the crystal separated was collected by filtration, washed with water and dried to give 6-chloro-2-(7-fluoro-3-oxo-2H-1,4-benzoxazin-6-yl)-1,2,4-triazolo[4,3-a]pyridin-3(2H)-one(0.28g).
   ¹H-NMR(CDCl₃) δ 4.69(2H, s), 7.10(1H, d, J=7.3Hz),7.17(1H, d, J=11.0Hz), 7.33-7.37(2H, s), 8.17(1H, s), 10.91(1H, br s).
(4)To a solution of the compound prepared in example 17-(3)(0.18g, 0.54mmol) and propargylbromide (96mg, 0.81mmol) in DMF(5ml), potassium carbonate(90mg, 0.65mmol) was added at room temperature, and the resulting mixture was stirred at same temperature for 12 hours. The reaction mixture was added to icewater and neutralized with dilute hydrochloric acid, and the crystal separated was collected by filtration, washed with water, further washed with diethyl ether and dried to give 6-chloro-2-(7-fluoro-3-oxo-4-propargyl-2H-1,4-benzoxazin-6-yl)-1,2,4-triazolo[4,3-a]pyridin-3(2H)-one(0.17g).
   ¹H-NMR(CDCl₃) δ 2.89(1H, t, J=2.4Hz), 4.70(4H, m), 6.95(1H, d, J=10.1Hz), 7.13-7.15(2H, m), 7.39(1H, d, J=6.8Hz), 7.89(1H, s).
   mp:196-198 °C
   IR(Nujol;cm⁻¹) 3268, 1730, 1694.

### Example 18

### Ethyl 2-chloro-4-fluoro-5-(6-chloro-2,3-dihydro-3-oxo-1,2,4-triazolo[4,3-a]pyridin-2-yl)cinnamate (Comp ound No. A-42)

(1)Compound No.2-4(0.3g, 1.0mmol) and triethylamine(1.0g, 9.88mmol) were dissolved in THF(10ml). While, to a solution of triphosgene(0.99g, 3.33mmol) in THF, pyridine(0.79g, 10.0mmol) was added dropwise under cooling with ice, and the resulting mixture was stirred for 30 minutes. This solution was added dropwise to above-mentioned hydrazine solution at room temperature by dropping pipet, and the resulting mixture was stirred at same temperature for 2 hours. After the reaction mixture was diluted with dilute hydrochloric acid, THF was evaporated and extracted with ethyl acetate, and the extract was dried and then concentrated. Diisopropyl ether was added to the residue, and the crystal separated was collected by filtration and dried to give 2-chloro-4-fluoro-5-(6-chloro-2,3-dihydro-3-oxo-1,2,4-triazolo[4,3-a]pyridin-2-yl)benzaldehyde (0.17g).
   ¹H-NMR(CDCl₃) δ 7.13-7.15(2H, m), 7.42(1H, d, J=9.4Hz), 7.87(1H, s), 8.22(1H, d, J=7.9Hz), 10.41(1H,s).
(2) The compound prepared in example 18-(1)(0.15g, 0.46mmol) and ethoxycarbonylmethylenetriphenylphosphorane(0.16g, 0.46mmol) were dissolved in toluene(8ml), and the resulting mixture was stirred at 75 °C for 12 hours. Furthermore, to this solution, ethoxycarbonylmethylenetriphenylphosphorane(40mg, 0.11mmol) was added, and the resulting mixture was stirred at 75 °C for 5 hours. After cooling, the reaction mixture was evaporated, and the residue was purified by silicagel column chromatography(chloroform:acetone=50:1), to give ethyl 2-chloro-4-fluoro-5-(6-chloro-2,3-dihydro-3-oxo-1,2, 4-triazolo[4,3-a]pyridin-2-yl)cinnamate(80mg).
   ¹H-NMR(CDCl₃) δ 1.34(3H, t, J=7.1Hz), 4.28(2H, q, J=7.1Hz), 6.42(1H, d, J=16.01Hz), 7.13-7.15(2H, m), 7.38(1H, d, J=9.6Hz), 7.88(1H, s), 7.89(1H, d, J=7.3Hz), 7.99(1H, d, J=16.01Hz).
   mp:162-165 °C
   IR(Nujol;cm⁻¹) 1722.

### Example 19

### Methyl 2-chloro-3-[2-chloro-4-fluoro-5-(6-chloro-2,3-dihydro-3-oxo-1,2,4-triazolo[4,3-a]pyridin-2-yl)phenyl]propionate (Compound No. A-43)

To a mixed solution of methyl acrylate (6ml) and acetonitrile(5ml), t-butyl nitrite(0.13g, 1.26mmol) and cuprous chloride(0.16g, 1.6mmol) were added, the resulting mixture was cooled at -20 °C. A suspension of the compound prepared in example 13-(2)(0.25g, 0.8mmol) in acetonitrile(5ml) was added to the above-mentioned solution by dropping pipet at keeping -20 °C. Then, the temperature was increased gradually up to room temperature, and the resulting mixture was stirred for 12 hours. The insoluble in the reaction mixture was filtered off, and the filtrate was concentrated. The residue was purified by silicagel column chromatography(chloroform:acetone=50:1), to give methyl 2-chloro-3-[2-chloro-4-fluoro-5-(6-chloro-2,3-dihydro-3-oxo-1,2,4-triazolo[4,3-a]pyridin-2-yl)phenyl]propionate (0.16g, amorphous).
¹H-NMR(CDCl₃) δ 3.29(1H, m), 3.53(1H, m), 3.79(3H, s), 7.58(1H, dd, J=8.1, 6.7Hz), 7.13-7.15(2H, m), 7.35(1H, d, J=9.7Hz), 7.57(1H, d, J=7.6Hz), 7.87(1H, s).
IR(Nujol;cm⁻¹) 1730.

### Example 20

### 6-Chloro-2-(5-cyano-3-fluoro-6-propargyloxypyridin-2 -yl)-1,2,4-triazolo[4,3-a]pyridin-3(2H)-one (Compound No. A-14)

Compound No. 1-5 (0.5g, 1.54mmol) and propargylalcohol (96mg, 1.71mmol) were dissolved in DMF (10ml), then to this solution, potassium carbonate was added, and the resulting mixture was stirred at 70 °C for 17 hours. After cooling, the reaction mixture was added to icewater, neutralized with dilute hydrochloric acid and extracted with ethyl acetate. The extract was washed with water, dried and then concentrated. The resudue was purified by silicagel column chromatography(chloroform:acetone:hexane=3:1:6), to give 6-chloro-2-(5-cyano-3-fluoro-6-propargyloxypyridin-2-yl)-1,2,4-triazolo[4,3-a]pyridin-3(2H)-one(0.13g).
¹H-NMR(CDCl₃) δ 2.53(1H, t, J=2.4Hz), 5.10(2H, d, J=2.4Hz), 7.15-7.17(2H, m), 7.86(1H, s), 7.89(1H, d, J=8.2Hz).
mp:207-209 °C
IR(Nujol;cm⁻¹) 3260, 2232, 2132, 1746.

### Example 21

### 6,8-Dichloro-2-(4-cyano-2-fluoro-5-propargyloxyphenyl)-1,2,4-triazolo[4,3-a]pyridin-3(2H)-one (Compound No. A-49)

Compound No. 1-12 (0.18g, 0.88mmol) and Compound No. 1-92 (0.15g, 0.88mmol) were dissolved in DMF (7ml), and then to this solution, potassium carbonate (0.12g, 0.88mmol) was added. The resulting mixture was stirred at 70 °C for 3 hours. After cooling, the reaction mixture was added to icewater and neutralized with dilute hydrochloric acid, and then the crystal separated was collected by filtration. The obtained crystal was dissolved in ethyl acetate, and the solution was dried and then evaporated. Diethyl ether was added to the residue, and the crystal precipitated was collected by filtration, to give 6,8-dichloro-2-(4-cyano-2-fluoro-5-propargyloxyphenyl)-1,2,4-triazolo[4,3-a]pyridin-3(2H)-one(40mg).
¹H-NMR(CDCl₃) δ 2.62(1H, t, J=2.4Hz), 4.87(2H, d, J=2.4Hz), 7.26(1H, s), 7.45-7.58(2H, m), 7.86(1H, s).
mp:145-147°C
IR(Nujol;cm⁻¹) 3400, 2200, 1738.

### Example 22

### 6-Chloro-2-[4-cyano-2-fluoro-5-(1-pyrrolidino) phenyl]-1,2,4-triazolo[4,3-a]pyridin-3(2H)-one (Compound No. A-22)

Compound No.1-1(0.10g, 0.33mmol), pyrrolidine(35mg, 0.49mmol) and potassium carbonate (55mg, 0.40mmol) were added to DMSO(3ml), and the resulting mixture was stirred at 70 °C for 3 hours. After cooling, icewater was added to the reaction mixture, the resulting mixture was extracted with ethyl acetate, and the extract was washed with water, dried and then evaporated. Diethyl ether was added to the resudue, and the crystal separated was collected by filtration and dried to give 6-chloro-2-[4-cyano-2-fluoro-5-(1-pyrrolidino) phenyl]-1,2,4-triazolo[4,3-a]pyridin-3(2H)-one (70mg).
¹H-NMR(CDCl₃) δ 1.95-2.05(2H, m), 3.55-3.65(4H, m), 6.91(1H, d, J=6.1Hz), 7.21-7.14(2H, m), 7.34(1H, s, J=9.9Hz), 7.87(1H, s).
mp:208-210 °C
IR(Nujol;cm⁻¹) 2210, 1720.

### Example 23

### 2-(4-Chloro-2-fluoro-5-propargyloxyphenyl-5,6-dihydrothiazolo[2,3-c][1,2,4]-triazol-3-yl-3-(2H)-on e (Compound No. C-1)

Compound No.3-1 (0.27g, 0.8mmol) and triethylamine(0.89g, 8.8mmol) were dissolved in THF(10ml). While, To a solution of triphosgene (0.79g, 2.67mmol) in THF(10ml), pyridine(0.63g, 8.0mmol) was added dropwise under cooling with ice, and the resulting mixture was stirred for 30 minutes. This solution was added dropwise to the above-mentioned hydrazine solution by dropping pipet at room temperature, the resulting mixture was stirred for 2 hours at same temperature. After the reaction mixture was diluted with dilute hydrochloric acid, THF was evaporated, the resulting solution was extracted with ethyl acetate, and the extract was dried and then concentrated. Diisopropyl ether was added to the residue, and the crystal separated was collected by filtration and dried to give 2-(4-chloro-2-fluoro-5-propargyloxyphenyl-5,6-dihydrothiazolo[2,3-c][1,2,4]-triazol-3-yl-3-(2H)-on e(0.17g).
¹H-NMR(CDCl₃) δ 2.58(1H, t, J=2.4Hz), 3.84(2H, t, J=6.3Hz), 4.08(2H, t, J=6.3Hz), 4.76(2H, d, J=2.4Hz), 7.23-7.31(2H, m).
mp:173-176 °C
IR(Nujol;cm⁻¹) 3240, 1719.

### Example 24

### 6-Chloro-2-(7-chloro-5-fluoro-2-methylbenzofuran-4-y 1)-1,2,4-triazolo[4,3-a]pyridin-3(2H)-one (Compound No. A-62)

Hydrazine of the Compound No.3-2 (0.4g, 1.22mmol) and triethylamine (1.23g, 12.2mmol) were dissolved in THF (10ml). On the one hand, triphosgene (1.2g, 4.1mmol) was dissolved in THF (25ml), and pyridine(0.97g, 12.2mmol) was added dropwise to it at room temperature, and then the mixture was stirred for 30 minutes. The later solution was added dropwise to the solution of hydrazine at room temperature, and the resulting mixture was stirred for 15 hours at the same temperature. Futhermore, the same quantity of the above-mentioned THF solution of triphosgene and pyridine was prepared and added to the said resultant, and the mixture was stirred for 24 hours. After the reaction mixture was diluted with diluted hydrochloric acid, THF was evaporated, the resulting solution was extracted with ethyl acetate, and the extract was dried and concentrated. To the obtained residue, diethylether was added, and the crystal precipitated was collected by filtration and dried to give 6-chloro-2-(7-chloro-5-fluoro-2-methylbenzofuran-4-yl)-1,2,4-triazolo[4,3-a]pyridin-3(2H)-one (0.12g).
¹H-NMR(CDCl₃) δ 2.50(3H, s), 6.48(1H, s), 7.14-7.15(2H, m), 7.18(1H, d, J=11.0Hz), 7.91(1H, s).
mp:205-207 °C
IR(Nujol;cm⁻¹) 1712.

### Example 25

### 6-Chloro-2-(3,4-dihydro-6-fluoro-1-propargyl-2(1H)-quinolinon-7-yl)-1,2,4-triazolo[4,3-a]pyridin-3(2H)-one (Compound No. A-63)

Hydrazine of the Compound No. 3-3 (0.25g, 0.73mmol) and triethylamine (1.1g, 9.9mmol) were dissolved in THF (20ml). On the one hand, triphosgene (1.09g, 3.67mmol) was dissolved in THF (20ml), and pyridine (0.87g, 11mmol) was added dropwise to it at room temperature, and then the mixture was stirred for 10 minutes. The later solution was added dropwise to the above solution of hydrazine at room temperature, and the resultant mixture was stirred for 3 days at the same temperature. The reaction mixture was diluted with diluted hydrochloric acid, THF was evaporated, the resulting solution was extracted with ethyl acetate, and the extract was dried and evaporated. The obtained residue was purified with silicagel column chromatography(hexane:ethyl acetate=1:1) to give 6-chloro-2-(3,4-dihydro-6-fluoro-1-propargyl-2(1H)-quinolinon-7-yl)-1,2,4-triazolo[4,3-a]pyridin-3(2H)-one(0.09g)
¹H-NMR(CDCl₃) δ 2.24(1H, t, J=2.4Hz), 2.72(2H, t, J=6.6Hz), 2.98(2H, t, J=6.6Hz), 4.70(2H, d, J=2.4Hz), 7.10-7.18(3H, m), 7.41(1H, d, J=6.2Hz), 7.89(1H, s).
mp:200-202 °C
IR(Nujol;cm⁻¹) 3200,1714,1681.

### Example 26

### 5,6-Dihydro-2-(4-cyano-5-ethylsulfonylamino-2-fluorophenyl)thiazolo[2,3-c][1,2,4]triazol-3(2H)-one (Compound No. C-2)

(1) Phenylhydrazone of the Compound No.3-4 (0.6g, 2.36mmol) and triethylamine (2.4g, 23.7mmol) were dissolved in THF (20ml). On the one hand, triphosgene (2.3g, 7.75mmol) was dissolved in THF (50ml), and pyridine (1.9g, 24mmol) was added dropwise to it at room temperature, and then the mixture was stirred for 30 minutes. The later solution was added dropwise to the above solution of hydrazone at room temperature, and the resulting mixture was stirred for 14 hours at the same temperature. Icewater was added to the reaction mixture, neutralized with hydrochloric acid, and THF was evaporated. The obtained residue was extracted with ethyl acetate, and the extract was dried and evaporated. The residue was purified by silicagel column chromatography (hexane:ethyl acetate=1:1) to give 5,6-dihydro-2-(4-cyano-2,5-difluorophenyl)thiazolo [2,3-c][1,2,4]triazol-3(2H)-one(0.5g)
   ¹H-NMR(CDCl₃) δ 3.85(2H, t, J=6.9Hz), 4.08(2H, t, J=6.9Hz), 7.41-7.49(1H, m), 7.54-7.61(1H, m).
(2) The solution of the compound prepared in example 26-(1) (0.21g, 0.75mmol), ethanesulfonamide (0.123g, 1.13mmol) and potassium carbonate(0.207g, 1.5mmol) in DMSO (10ml) was stirred at 80 °C for 12 hours. After cooling, the reaction mixture was added to icewater, neutralized with concentrated hydrochloric acid and extracted with ethyl acetate, and then the extract was dried and removed the solvent. The obtained residue was purified by silicagel column chromatography (hexane:ethyl acetate=1:1) to give 5,6-dihydro-2-(2-fluoro-4-cyano-5-ethylsulfonylaminophenyl)thiazolo[2,3-c][1,2,4] triazol-3(2H)-one(0.06g).
   ¹H-NMR(CDCl₃) δ 1.45(3H, t, J=7.4Hz), 3.24(2H, q, J=7.4Hz), 3.86(2H, t, J=7.4Hz), 4.09(2H, t, J=7.4Hz), 6.91(1H, br s), 7.45(1H, d, J=9.3Hz), 8.01(1H, d, J=6.4Hz).
   mp:209-211 °C
   IR(Nujol;cm⁻¹) 3100,2236,1730.

### Example 27

### 5,6-Dihydro-2-(7-fluoro-4-propargyl-3-oxo-1,4-benzox azin-6-yl)thiazolo[2,3-c][1,2,4]triazol-3(2H)-one (Compound No. C-3)

Phenylhydrazone of the Compound No. 3-5 (0.2g, 0.63mmol) and triethylamine (0.64mg, 6.3mmol) were dissolved in THF (5ml). On the one hand, triphosgene (0.62g, 2.1mmol) was dissolved in THF (15ml), and pyridine(0.5g, 6.3mmol) was added dropwise to it at room temperature, and then the mixture was stirred for 15 minutes. The later solution was added dropwise to the above solution of hydrazone at room temperature, and the resultant mixture was stirred for 12 hours at the same temperature. Icewater was added to the reaction mixture, and the mixture was neutralized with hydrochloric acid, and THF was removed. The obtained residue was extracted with ethyl acetate, and the extract was dried and evaporated. The residue was purified by silicagel column chromatography(hexane:ethyl acetate=1:2→1:20) to give 5,6-dihydro-2-(7-fluoro-4-propargyl-3-oxo-1,4-benzoxazin-6-yl)thiazolo[2,3-c][1,2,4]triazol-3(2H)-one(0.06g).
¹H-NMR(CDCl₃) δ 2.30(1H, t, J=6.9Hz), 3.85(2H, t, J=6.9Hz), 4.10(2H, t, J=6.9Hz), 4.67(2H, s), 6.89(1H, d, J=10.1Hz), 7.31(1H, d, J=6.9Hz).
mp:211-213 °C
IR(Nujol;cm⁻¹) 3298, 1723, 1689.

### Example 28

### 5,6-Dihydro-2-(4-cyano-5-ethylsulfonylamino-2-fluorophenyl)-7H-[1,2,4]triazolo[3,4-b][1,3]thiazin-3(2H)-one (Compound No. E-1)

(1) Phenylhydrazone of the Compound No.3-6(0.6g. 2.24mmol) and triethylamine(2.27g, 22.4mmol) were dissolved in THF(20ml). On the one hand, triphosgene (22.2g, 7.5mmol) was dissolved in THF (20ml). and pyridine (1.77g, 22.4mmol) was added dropwise to it at room temperature, and then the mixture was stirred for 10 minutes. The later solution was added dropwise to the above solution of hydrazone at room temperature, and the resulting mixture was stirred for 12 hours at the same temperature. The same quantity of THF solution of phosgene-pyridine was prepared and added to the above-mentioned resultant mixture, and the mixture was stirred for 36 hours. Icewater was added to the reaction mixture, and the mixture was neutralized with hydrochloric acid, and THF was removed. The obtained residue was extracted with ethyl acetate, and the extract was dried and evaporated, to give 5,6-dihydro-2-(4-cyano-2,5-difluorophenyl)-7H-[1,2,4]triazolo[3,4-b][1,3]thiazine-3(2H)-one(0.5g).
   ¹H-NMR(CDCl₃) δ 2.30-2.40(2H, s), 3.16(2H, t, J=5.8Hz), 3.82(2H, t, J=6.1Hz), 7.42-7.49(1H, m), 7.57-7.64(1H, m).
(2) The compound prepared in example 28-(1)(0.5g, 1.7mmol), ethanesulfonamide(0.38g, 3.49mmol) and potassium carbonate(0.48g, 3.48mmol) were added to DMSO(20ml), and the mixture was stirred at 70 °C for 36 hours. After cooling, the reaction mixture was added to icewater, and the resultant was neutralized with concentrated hydrochloric acid and then extracted with ethyl acetate. The extract was dried, and the solvent was removed. The residue was purified with silicagel column chromatography (hexane:ethyl acetate=1:2), to give 5,6-dihydro-2-(4-cyano-5-ethylsulfonylamino-2-fluoro phenyl)-7H-[1,2,4]triazolo[3,4-b][1,3]thiazin-3(2H)-one(0.35g).
   ¹H-NMR(CDCl₃) δ 1.4(3H, t, J=7.4Hz), 3.11-3.30(4H, m), 3.82(2H, t, J=6.1Hz), 6.90(1H, br s), 7.45(1H, d, J=9.4Hz), 8.01(1H, d, J=6.4Hz).
   mp:193-195 °C
   IR(Nujol;cm⁻¹) 3246, 2229, 1721.

### Example 29

### 5,6,7,8-Tetrahydro-2-(4-chloro-2-fluoro-5-propargyloxyphenyl)-[1,2,4]triazolo[4,3-a]pyrimidin-3(2H)-one (Compound No. F-1)

(1) 3,4,5,6-Tetrahydro-2-methylthiopyrimidine (6.0g, 23.3mmol) and triethylamine(7.1g, 70.2mmol) were dissolved in THF(50ml). To the solution cooled on ice, ethyl chloroformate (5.1g, 47mmol) was added dropwise, and the mixture was stirred for 1 hour at room temperature. The reaction mixture was added to icewater, and THF was removed. The mixrure was extracted by ethyl acetate, and the extract was dried, and the solvent was removed. To the residue, diethyl ether was added and the crystal precipitated was collected by filtration and dried to give 3,4,5,6-tetrahydro-3-ethoxycarbonyl-2-methylthiopyrimidine(2.2g).
   ¹H-NMR(CDCl₃) δ 1.33(3H, s), 1.78-1.91(2H, m), 2.26(3H, s), 3.54(2H, t, J=5.7Hz), 3.70(2H, t, J=6.0Hz), 4.24(2H, q, J=7.1Hz).
(2) The compound prepared in example 29-(1)(0.13g, 0.66mmol) and 4-chloro-2-fluoro-5-propargyloxyphenylhydrazine (0.165g, 0.66mmol) were dissolved in 2-propanol (10ml). To the solution, trifluoroacetic acid (cat.) was added at room temperature and the mixture was refluxed under heating for 2 hours. After cooling, the reaction mixture was evaporated, neutralized with sodium bicarbonate water and extracted with ethyl acetate. The extract was dried, and the solvent was removed. Diethyl ether was added to the obtained residue, and the crystal precipitated was collected by filtration and dried to give 5,6,7,8-tetrahydro-2-(4-chloro-2-fluoro-5-propargyloxyphenyl)-[1,2,4]triazolo[4,3-a]pyrimidin-3(2H)-one (0.1g).
   ¹H-NMR(CDCl₃) δ 2.05-2.20(2H, m), 2.56(1H, t, J=2.4Hz), 3.35-3.45(2H, m), 3.74(2H, t, J=6.0Hz), 4.15(1H, s), 4.75(2H, d, J=2.4Hz), 7.22-7.31(2H, m).
   mp: 172-174 °C
   IR(Nujol;cm⁻¹) 3389, 3322, 1724.

### Example 30

### 5,6,7,8-Tetrahydro-8-methyl-2-(4-cyano-5-ethylsulfonylamino-2-fluorophenyl)-[1,2,4]triazolo [4,3-a]pyrimidin-3(2H)-one (Compound No. F-2)

(1) 3,4,5,6-Tetrahydro-2-pyrimidinethiol (5.0g, 38.5mmol) was dissolved in acetone (20ml), to this solution, methyl iodide(11.0g, 77.5mmol) was added dropwise under cooling with ice, and the resulting mixture was stirred at room temperature for 1 hour. The crystal separated was collected by filtration and dried to give 3,4,5,6-tetrahydro-3-methyl-2-methylthiopyrimidine hydroiodic acid salt(9.0g).
   ¹H-NMR(DMSO-d₆) δ 1.91-2.03(2H, m), 2.63(3H, s), 3.19(3H, s), 3.38(2H, t, J=5.7Hz), 3.52(2H, t, J=5.9Hz), 8.95(1H, br s).
(2) The compound prepared in example 30-(1) (3.0g, 11.0mmol) and hydrazine monohydrate (5.5g, 110mmol) were dissolved in ethanol (20ml), and this solution was refluxed under heating for 3 hours. After cooling, the reaction mixture was concentrated to dryness, to give 3,4,5,6-tetrahydro-3-methyl-2-hydrazinopyrimidine (2.2g).
   ¹H-NMR(CDCl₃) δ 2.05-2.11(2H, m), 3.24(3H, s), 3.40-3.48(4H, m), 4.09(2H, br s), 7.15(2H, br s).
(3) The compound prepared in example 30-(2)(0.5g, crude) was dissolved in ethyl chloroformate (5ml), and this solution was refluxed under heating for 1 hour. After cooling, the crystal separated was collected by filtration, the obtained crystal was dissolved in methanol, and to this solution, potassium carbonate (0.1g, 0.72mmol) was added. The resulting mixture was stirred at room temperature for 12 hours. The insoluble was filtered off, and the filtrate was evaporated. Diethyl ether was added to the residue, and the crystal separated was collected by filtration and dried to give 5,6,7,8-tetrahydro-8-methyl-[1,2,4]triazolo[4,3-a]pyrimidin-3(2H)-one(0.14g).
   ¹H-NMR(CDCl₃) δ 2.04-2.16(2H, m), 2.92(3H, s), 3.19(2H, t, J=5.7Hz), 3.62(2H, t, J=6.2Hz), 8.95(1H, br s).
(4) The compound prepared in example 30-(3)(0.1g, 0.65mmol) and 1,3,5-trifluorobenzonitrile (0.1g, 0.64mmol) were dissolved in DMSO (5ml), to this solution, potassium carbonate (0.1g, 0.72mmol) was added at room temperature, and the resulting mixture was stirred at 80 °C for 2 hours. After cooling, the reaction mixture was added to icewater, and the crystal separated was collected by filtration, washed with water and dried to give 5,6,7,8-tetrahydro-8-methyl-2-(4-cyano-2,5-difluorop henyl)-[1,2,4]triazolo[4,3-a]pyrimidine-3(2H)-one(0. 1g).
   ¹H-NMR(CDCl₃) δ 2.13-2.22(2H, m), 3.00(3H, s), 3.29(2H, t, J=6.1Hz), 3.71(2H, t, J=6.3Hz), 7.37-7.45(1H, m), 7.56-7.64(1H, m).
(5) The compound prepared in example 30-(4) (0.5g, 1.72mmol), ethanesulfonamide (0.38g, 3.48mmol) and potassium carbonate (0.48g, 3.48mmol) were added to DMSO(10ml), and the mixture was stirred at 90 °C for 12 hours and then at 110 °C for 4 hours. After cooling, the reaction mixture was added to icewater, neutralized with concentrated hydrochloric acid and extracted with ethyl acetate, and the extract was dried, and the solvent was removed. The obtained residue was purified by silicagel column chromatography(hexane:ethylacetate=1:5) to give 5,6,7,8-tetrahydro-8-methyl-2-(4-cyano-5-ethylsulfon ylamino-2-fluorophenyl)-[1,2,4]-triazolo[4,3-a]pyrim idin-3(2H)-one(0.35g).
   ¹H-NMR(CDCl₃) δ 1.43(3H, t, J=7.4Hz), 2.10-2.20(2H, m), 3.00(3H, s), 3.22(2H, q), 3.28(2H, t, J=6.3Hz), 3.71(2H, t, J=6.2Hz), 6.99(1H, br s), 7.40(1H, d, J=9.5Hz), 7.99(1H, d, J=6.4Hz).
   mp:179-181 °C
   IR(Nujol;cm⁻¹) 3100, 2100, 1708.

The compounds obtained by similar way to example 1 to 30 are shown in table 1 to 8 with the compounds obtained in example 1 to 30.

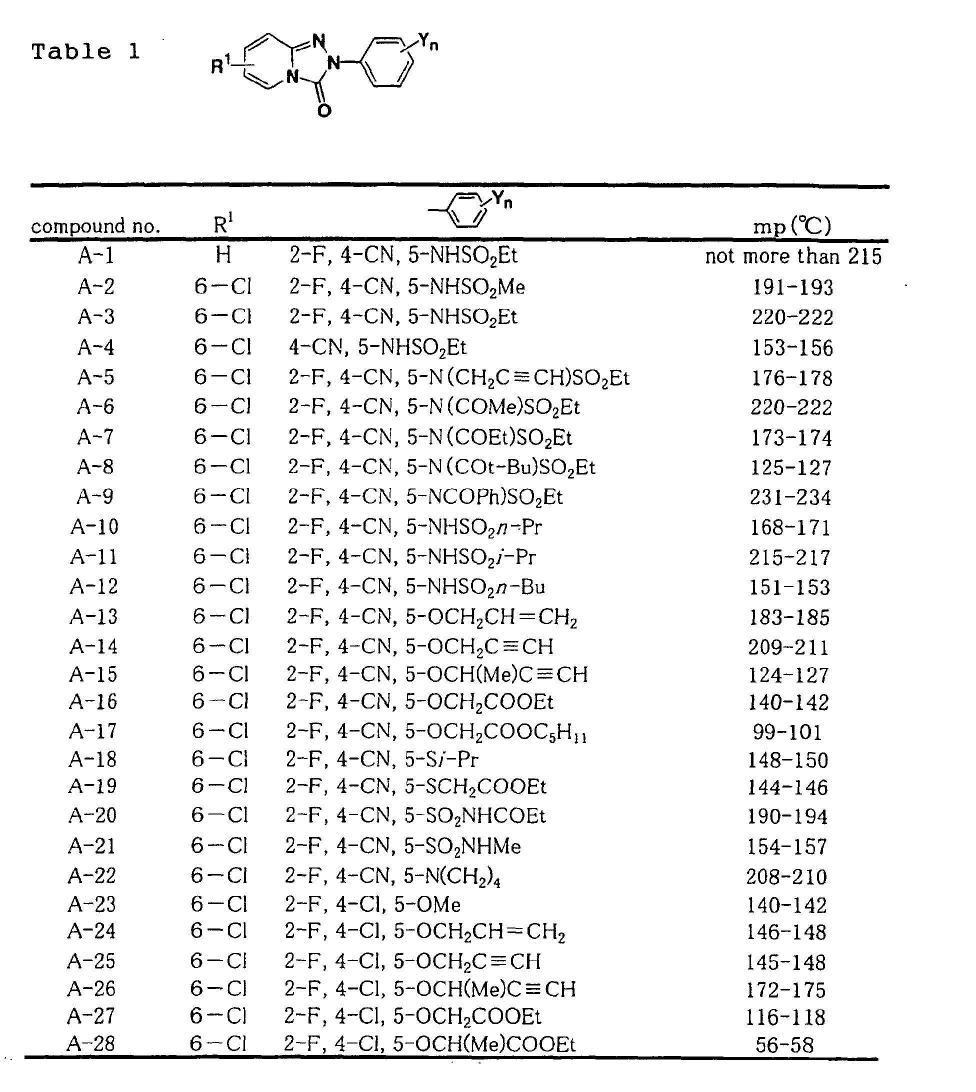

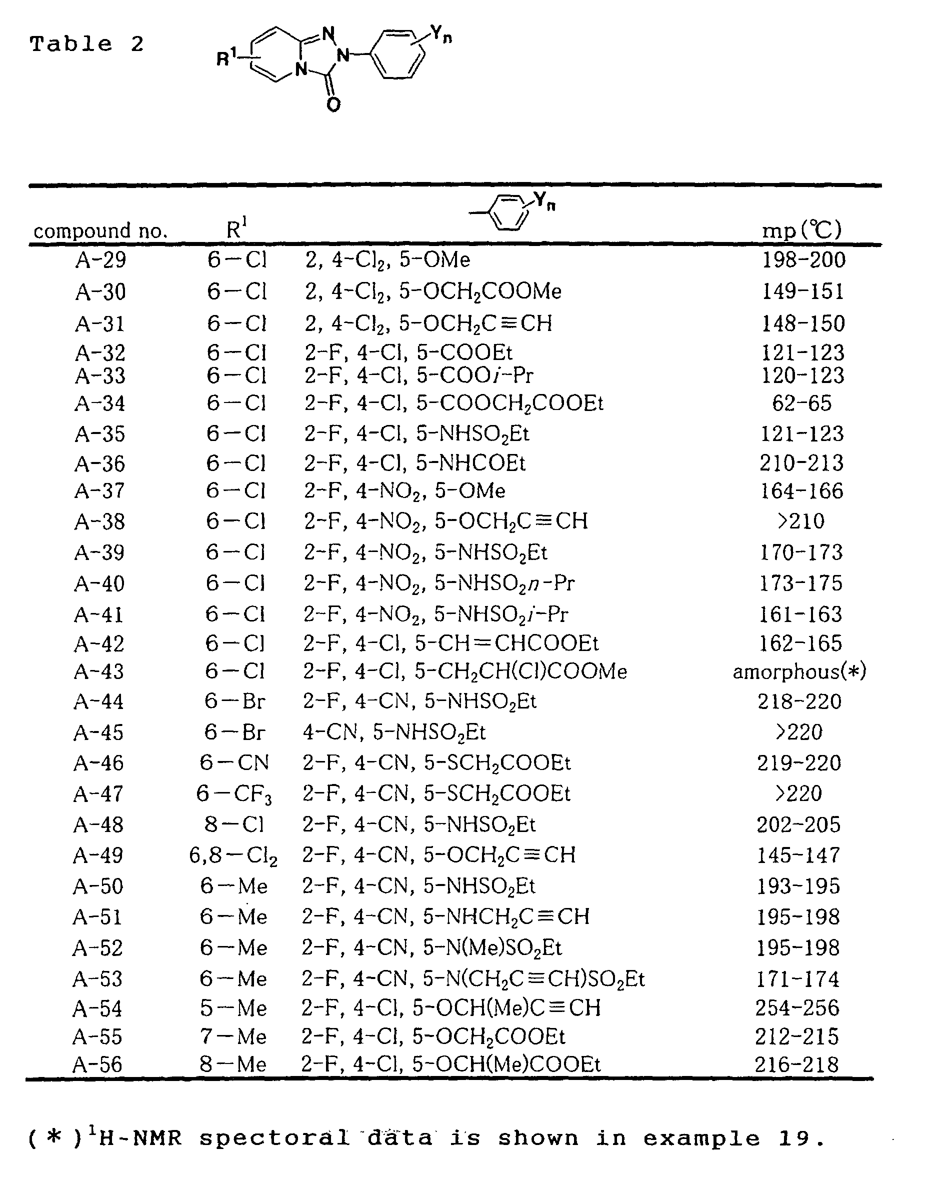

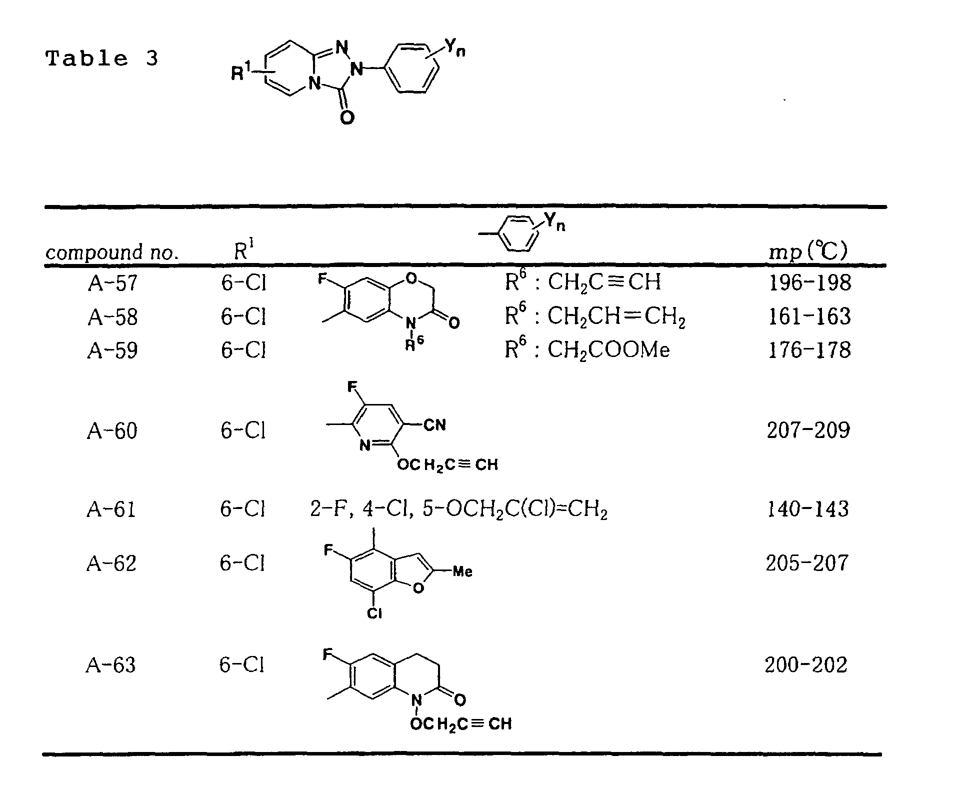

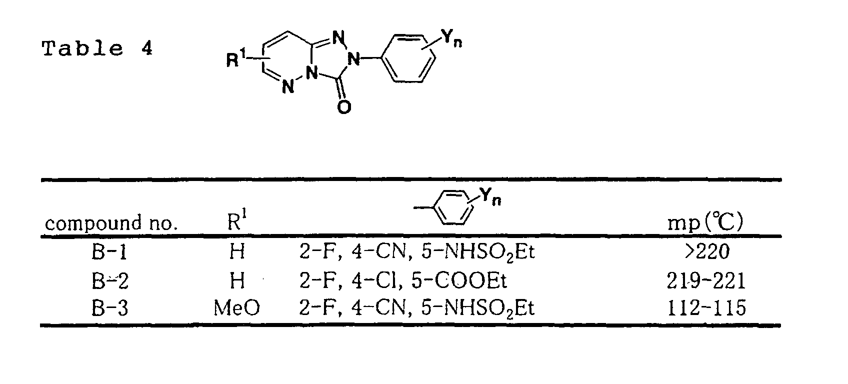

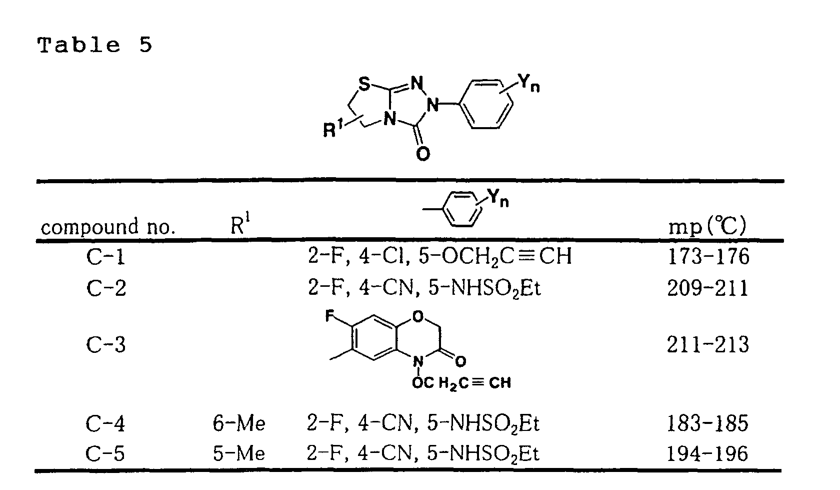

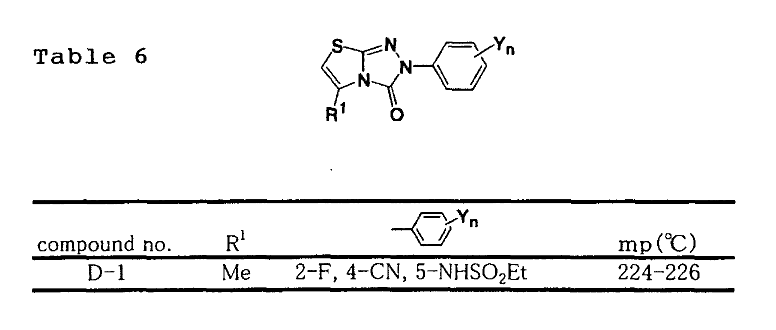

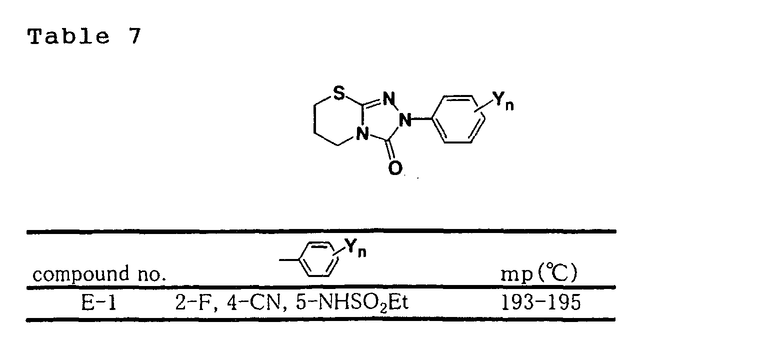

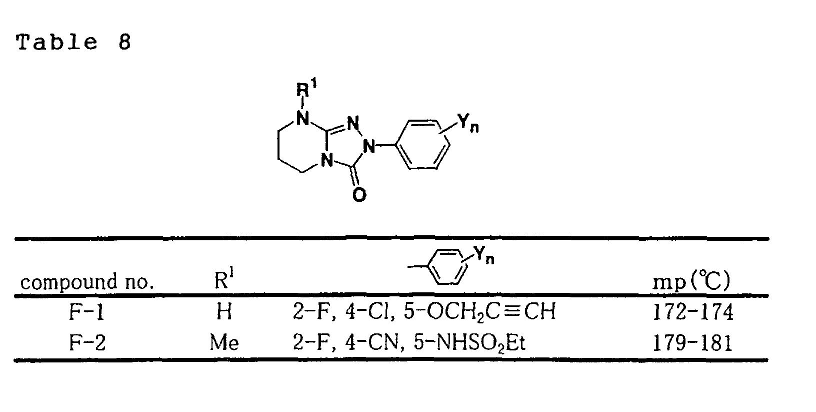

### FORMULATION EXAMPLE

### Formulation Example 1

### Wettable powder

Compound No. A-1 (10 wt%), TweenZO™ (20 wt%), white carbon (40 wt%) and clay (30 wt%) were mixed and ground to produce a wettable powder. (Diluting with water appropriately before use.)

### Formulation Example 2

### Wettable powder

Compound No. A-2 (80 wt%), sodium dodecylbenzenesulphonate (2 wt%), sodium naphthalenesulphonate (3 wt%) and clay (15 wt%) were mixed and ground to produce a wettable powder. (Diluting with water appropriately before use.)

### Formulation Example 3

### Wettable powder

Compound No. A-24 (5 wt%), polyoxyethylene glycol nonylphenylether (NONIPOL85™) (3 wt%), sodium ligninsulfonate (5 wt%) and clay (87 wt%) were mixed and ground to produce a wettable powder. (Diluting with water appropriately before use.)

### Formulation Example 4

### Emulsion

Compound No. A-35 (2 wt%), xylene (75 wt%). dimethylformamide (18 wt%) and polyoxyethylene glycol nonylphenylether (NONIPOL85™) (5 wt%) were mixed and ground to produce emulsion. (Diluting with water appropriately before use.)

### Formulation Example 5

### Flowable preparation

Compound No. A-57 (2 wt%), polyoxyethylene allylphenyletherformamide (NEW KALGON E-300™) (3 wt%), polyoxyethylene phenylphenolether sulfate (AGRIZOL FL-2017™) (2 wt%), special polyol polymer (AGRIZOL FL-104FA™) (15 wt%), white carbon (2 wt%), ethylene glycol (10 wt%) and water (66 wt%) were mixed and wet ground to produce a suspended flowable preparation. (Diluting with water appropriately before use.)

### TEST EXAMPLE

### Test Example 1

### Herbicidal effects against weeds (post emergence)

After filling 300g field soil in jiffypot™ having diameter of 10 cm, the soil was steam sterilized. Seeds of velvetleaf, redroot pigweed, tall morningglory and corn onto the surface of the soil were scattered, and then covered with the soil to a thickness of 0.5 cm for the weed, 1 cm for the crop. When the weed and the crop reached the designated growth stage, they were treated with the compound to be tested.

### Preparation of the test solution and the method of treatment

The compound to be tested was dissolved in acetone which contain Tween 20™ and diluted with deionized water to prepare the test solution for 10g/a and 1g/a use. The test solution was sprayed on the pots in a device for spraying using spraygun.

### Method of indicating the effect and phytotoxicity

The effect of the compound tested on the weeds and phytotoxicity on the crops were evaluated two weeks after treatment with the compound as 6 grades from 0 to 5 shown in the Table 9 and 10.

The test results were shown in the Table 11 and 12.

In the Table 11 and 12, the herbicidal effects were indicated by the following evaluation index.

**Table 9**

| Index | Effect | Weed control (%) |
|---|---|---|
| 0 | no effect | 0 |
| 1 | small | 0.1 to 50.0 |
| 2 | middle | 50.1 to 75.0 |
| 3 | big | 75.1 to 87.5 |
| 4 | maximum | 87.6 to 99.9 |
| 5 | maximum(all dead) | 100 |

The phytotoxicity was indicated by the following evaluation index.

**Table 10**

| Index | Effect | Crop injury (%) |
|---|---|---|
| 0 | no effect | 0 |
| 1 | small | 0.1 to 12.5 |
| 2 | middle | 12.6 to 25.0 |
| 3 | big | 25.1 to 50.0 |
| 4 | maximum | 50.1 to 99.9 |
| 5 | maximum(all dead) | 100 |

**Table 11**

| Compound | g/a | Evaluation index | | | |
|---|---|---|---|---|---|
| No. | | Corn | Velvet leaf | Redroot pigweed | Tall morning glory |
| A - 3 | 1 | 1 | 5 | - | 4 |
| A - 6 | 10 | 1 | 5 | 5 | - |
| A - 7 | 10 | 1 | 5 | 5 | - |
| A - 8 | 1 | 1 | 5 | 5 | - |
| | 10 | 1 | 5 | 5 | - |
| A - 11 | 10 | 1 | 5 | 5 | - |
| A - 19 | 10 | 0 | 5 | - | 5 |
| A - 24 | 1 | 1 | 4 | 5 | - |
| | 10 | 1 | 5 | 5 | - |
| A - 25 | 1 | 0 | 5 | - | 5 |
| | 10 | 1 | 5 | - | 5 |
| A - 26 | 1 | 1 | 5 | 5 | - |
| | 10 | 1 | 5 | 5 | - |
| A - 27 | 10 | 0 | 5 | - | 5 |
| A - 28 | 1 | 0 | 4 | 5 | - |
| | 10 | 0 | 5 | 5 | - |
| A - 34 | 1 | 1 | 4 | 5 | - |
| A - 35 | 10 | 1 | 5 | 5 | - |
| A - 40 | 10 | 1 | 5 | 5 | - |
| A - 41 | 1 | 0 | 5 | 5 | - |
| | 10 | 2 | 5 | 5 | - |

**Table 12**

| Compound | g/a | Evaluation index | | | |
|---|---|---|---|---|---|
| No. | | Corn | Velvet leaf | Redroot pigweed | Tall morning glory |
| A - 43 | 1 | 0 | 5 | 5 | - |
| | 10 | 0 | 5 | 5 | - |
| A - 45 | 1 | 1 | 5 | 5 | - |
| A - 48 | 10 | 1 | 5 | - | 5 |
| A - 50 | 10 | 0 | 5 | - | 5 |
| A - 55 | 10 | 1 | 4 | 5 | - |
| A - 61 | 10 | 2 | 4 | 5 | - |
| A - 62 | 1 | 1 | 5 | 5 | - |
| | 10 | 1 | 5 | 5 | - |
| B - 3 | 10 | 1 | 4 | 5 | - |
| C - 2 | 1 | 0 | 5 | 5 | - |
| | 10 | 0 | 5 | 5 | - |
| C - 3 | 1 | 3 | 5 | 5 | - |
| | 10 | 4 | 5 | 5 | - |
| C - 4 | 1 | 2 | 5 | 5 | - |
| | 10 | 4 | 5 | 5 | - |
| C - 5 | 1 | 1 | 4 | 5 | - |
| | 10 | 1 | 5 | 5 | - |
| E - 1 | 1 | 1 | 5 | 5 | - |
| | 10 | 1 | 5 | 5 | - |
| F - 1 | 1 | 3 | 4 | 5 | - |
| | 10 | 3 | 5 | 5 | - |
| F - 2 | 1 | 1 | 4 | 5 | - |
| | 10 | 3 | 5 | 5 | - |

### Industrial Applicability

A compound (I) or a salt thereof has excellent herbicidal activity against a wide variety of weeds, for example, weeds in paddy field or in fields with a low dose. Moreover, it has little phytotoxicity on cultivated plants, for example, rice, wheat, barley, corn, beans, cotton, and so on, and shows an excellent selective herbicidal effect. Also, the selective herbicidal effect can maintain for a long time. It has little toxicity on mammals and fishes, does not pollute environment, so it can be used extreamly safely as a herbidical composition for paddy fields, fields, orchards or non-agricultural lands and so on.

## Claims

1. A bicyclic triazolone derivative of the formula (I):
J-Ar (I)
[Wherein J is one of the groups represented by the formula described bellow; 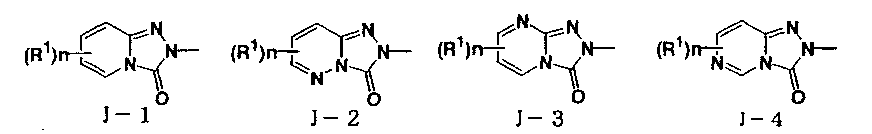 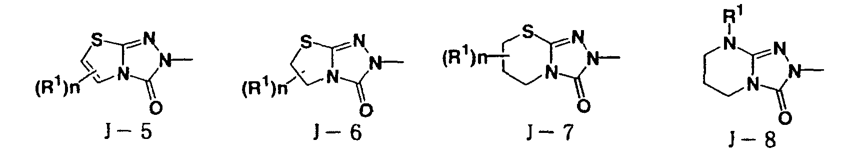 (wherein R¹ is hydrogen, halogen, a C₁₋₆alkyl group, a C₂₋₆alkenyl group, a C₂₋₆alkynyl group, a C₃₋₆cycloalkyl group, a C₇₋₁₂aralkyl group, a C₆₋₁₀aryl group, a C₁₋₆alkoxyC₁₋₄alkyl group, a C₁₋₆alkoxy group, a C₂₋₆alkenyloxy group, a C₂₋₆alkynyloxy group, a C₇₋₁₂aralkyloxy group, a C₁₋₆haloalkoxy group, a C₂₋₆haloalkenyl group, a C₆₋₁₀aryloxy group, a C₁₋₆alkylthio group, a C₂₋₆alkenylthio group, a C₂₋₆alkynylthio group, a C₇₋₁₂aralkylthio group, a C₆₋₁₀arylthio group, a C₁₋₆alkylsulfonyl group, a C₂₋₆alkenylsulfonyl group, a C₂₋₆alkynylsulfonyl group, a C₇₋₁₂aralkylsulfonyl group, a C₆₋₁₀arylsulfonyl group, a cyclic-1,3-dioxa-2-yl group, a cyclic-1,3-dithio-2-yl group, a C₁₋₇alkanoyl group, a C₇₋₁₁arylcarbonyl group, a C₂₋₇alkoxycarbonyl group, a C₈₋₁₃aralkyloxycarbonyl group, a mono-C₁₋₄ or di(C₁₋₄)alkylcarbamoyl group, an amino group, a C₁₋₇alkanoylamino group, a mono-C₁₋₄ or di(C₁₋₄)alkylamino group, a C₁₋₂alkylenedioxy group, nitro group, hydroxy group, mercapto group, cyano group, carboxyl group, or sulfo group, n is an integer from 1 to a substitutable maximum number),
Ar is a phenyl group which may be substituted, a pyridyl group or a condensed heterocyclic group with the phenyl group or the pyridyl group which may be substituted] or a salt thereof.

2. A bicyclic triazolone derivative or a salt thereof as claimed in claim 1, wherein Ar is one of the groups 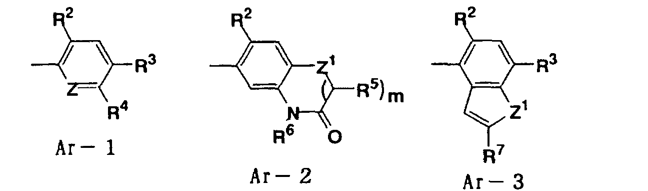 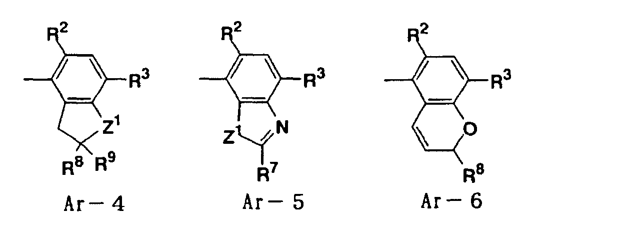 [wherein R² is hydrogen, halogen, a C₁₋₆alkyl group, a C₁₋₆haloalkyl group, a C₁₋₆alkoxy group or a C₁₋₆haloalkoxy group,
R³ is halogen, nitro group, cyano group, carbamoyl group, a hydroxylC₁₋₄alkyl group, a C₁₋₆alkoxyC₁₋₄alkyl group, a C₂₋₇alkoxycarbonyl group, a C₃₋₇alkenyloxycarbonyl group, a C₃₋₇alkynyloxycarbonyl group, or a C₇₋₁₂aralkyloxy group which may be substituted by a substituent or substituents selected from halogen, a C₁₋₆alkyl group, a C₁₋₆haloalkyl group, a C₁₋₆alkoxy group, a C₁₋₆haloalkoxy group, a C₂₋₆alkenyloxy group, a C₂₋₆alkynyloxy group, a C₂₋₇alkoxycarbonyl group, a C₃₋₇alkenyloxycarbonyl group, a C₃₋₇alkynyloxycarbonyl group, a C₂₋₇alkoxycarbonylC₁₋₄alkoxy group, a C₃₋₇alkenyloxycarbonylC₁₋₄alkoxy group, and a C₃₋₇alkynyloxycarbonylC₁₋₄alkoxy group, the number of the substituent for the substitution is an integer from 1 to a substitutable maximum number),
R⁴ is hydrogen, a C₁₋₆alkyl group, a C₃₋₆cycloalkyl group, a C₁₋₆haloalkyl group, hydroxyl group, mercapto group, a C₁₋₆alkoxy group, a C₁₋₆alkoxyC₁₋₄alkoxy group, a C₃₋₆cycloalkyloxy group, a C₁₋₆haloalkoxy group, a C₂₋₆alkenyloxy group, a C₂₋₆haloalkenyloxy group, a C₂₋₆alkynyloxy group, a C₆₋₁₀aryloxy group, a C₇₋₁₂aralkyloxy group, a C₁₋₆alkylthio group, a C₁₋₆alkoxyC₁₋₄alkylthio group, a C₃₋₆cycloalkylthio group, a C₁₋₆haloalkylthio group, a C₂₋₆alkenylthio group, a C₂₋₆haloalkenylthio group, a C₂₋₆alkynylthio group, a C₆₋₁₀arylthio group, a C₇₋₁₂aralkylthio group, C₁₋₆alkylsulfonyl group, a C₃₋₆cycloalkylsulfonyl group, a C₁₋₆haloalkylsulfonyl group, a C₂₋₆alkenylsulfonyl group, a C₂₋₆alkynylsulfonyl group, a cyclic amino group having one or two atoms selected from oxygen, sulfur and nitrogen or one of the groups represented by formulas; 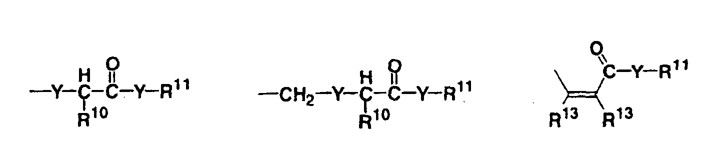 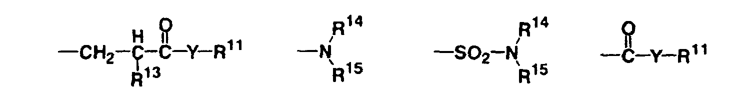 (Wherein Y is oxygen, sulfur or -N-R¹²,
R¹⁰ is hydrogen or a C₁₋₆alkyl group,
R¹¹ is hydrogen, a C₁₋₆alkyl group, a C₁₋₆haloalkyl group, a C₃₋₆cycloalkyl group, a C₂₋₆alkenyl group, a C₂₋₆haloalkenyl group, a C₂₋₆alkynyl group, a C₆₋₁₀aryl group, a C₇₋₁₂aralkyl group, a C₁₋₆alkoxyC₁₋₄alkyl group, a C₂₋₆alkenyloxyC₁₋₄alkyl group, a C₂₋₆alkynyloxyC₁₋₄alkyl group, a C₃₋₆cycloalkoxyC₁₋₄alkyl group, a C₂₋₇alkoxycarbonylC₁₋₄alkyl group, a C₃₋₇alkenyloxycarbonylC₁₋₄alkyl group, a C₃₋₇alkynyloxycarbonylC₁₋₄alkyl group, a C₄₋₇cycloalkoxycarbonylC₁₋₄alkyl group, a C₂₋₇haloalkoxycarbonylC₁₋₄alkyl group, a C₃₋₇haloalkenyloxycarbonylC₁₋₄alkyl group, or a C₇₋₁₂aralkyloxycarbonylC₁₋₄alkyl group,
R¹² is hydrogen, a C₁₋₆alkyl group, a C₁₋₆alkoxyC₁₋₄alkyl group, a C₁₋₇alkanoyl group, a C₇₋₁₁arylcarbonyl group, C₂₋₇alkoxycarbonyl group, a C₂₋₇haloalkoxycarbonyl group, or a C₃₋₇haloalkenyloxycarbonyl group,
R¹³ is hydrogen, halogen, or a C₁₋₆alkyl group, each of R¹⁴ and R¹⁵ is same or differnetly hydrogen, a C₁₋₆alkyl group, a C₂₋₆alkenyl group, a C₂₋₆alkynyl group, a C₃₋₆cycloalkyl group, a C₁₋₆alkoxyC₁₋₄alkyl group, a C₆₋₁₂aralkyl group, a C₁₋₄alkyl group which is substituted with 5- or 6- membered hetero ring containing nitrogen, oxygen or sulfur, a C₁₋₇alkanoyl group, a C₆₋₁₂arylcarbonyl group, a C₂₋₇haloalkylcarbonyl group, a C₂₋₇alkoxycarbonyl group, a C₃₋₇alkenyloxycarbonyl group, a C₃₋₇alkynyloxycarbonyl group, a C₄₋₇cycloalkyloxycarbonyl group, a C₂₋₇haloalkoxycarbonyl group, a C₃₋₇haloalkenyloxycarbonyl group, a C₁₋₆alkylsulfonyl group, a C₂₋₇alkenylsulfonyl group, a C₂₋₇alkynylsulfonyl group, a C₃₋₆cycloalkylsulfonyl group, a C₁₋₆haloalkylsulfonyl group, a C₆₋₁₀arylsulfonyl group, a C₇₋₁₂aralkylsulfonyl group, or a group represented by formula; 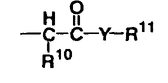 (wherein each of R¹⁰, R¹¹ and Y has the same meaning as described above),
R⁵ is hydrogen or a C₁₋₆alkyl group; m is 0 or 1,
R⁶ is hydrogen, a C₁₋₆alkyl group, a C₁₋₆haloalkyl group, a C₂₋₆alkenyl group, a C₂₋₆alkynyl group, a C₁₋₆alkoxyC₁₋₄alkyl group, a C₁₋₇alkanoyl group, a C₆₋₁₀arylcarbonyl group, a C₂₋₇alkoxycarbonyl group, a C₂₋₇haloalkoxycarbonyl group, a C₃₋₇alkenyloxycarbonyl group, a C₃₋₇haloalkenyloxycarbonyl group, a C₃₋₇alkynyloxycarbonyl group, a C₄₋₇cycloalkyloxycarbonyl group, a C₂₋₇alkoxycarbonylC₁₋₄alkyl group, a C₂₋₇haloalkoxycarbonylC₁₋₄alkyl group, a C₃₋₇alkenyloxycalbonylC₁₋₄alkyl group, a C₃₋₇haloalkenyloxycarbonylC₁₋₄alkyl group, or a C₃₋₇alkynyloxycarbonylC₁₋₄alkyl group,
R⁷ is hydrogen, halogen, a C₁₋₆alkyl group, a C₁₋₆alkoxy group, a C₁₋₆haloalkoxy group, a C₂₋₆alkenyloxy group, a C₂₋₆alkynyloxy group, a C₁₋₆alkylthio group, a C₁₋₆haloalkylthio group, a C₂₋₆alkenylthio group, a C₂₋₆alkynylthio group, a C₁₋₆haloalkyl group, a C₁₋₆alkoxyC₁₋₄alkyl group, a C₁₋₆alkylthioC₁₋₄alkyl group, or a C₁₋₆alkylsulfonylC₁₋₄alkyl group,
R⁸ is hydrogen, or a C₁₋₆alkyl group,
R⁹ is a C₁₋₆alkyl group,
Z is CH or nitrogen,
Z¹ is oxygen, sulfur or methylene.]

3. A bicyclic triazolone derivative or a salt thereof as claimed in claim 1, wherein J in the formula (I) is J-1.

4. A bicyclic triazolone derivative or a salt thereof as claimed in claim 1, wherein J in the formula (I) is J-2.

5. A bicyclic triazolone derivative or a salt thereof as claimed in claim 1, wherein J in the formula (I) is J-3.

6. A bicyclic triazolone derivative or a salt thereof as claimed in claim 1, wherein J in the formula (I) is J-4.

7. A bicyclic triazolone derivative or a salt thereof as claimed in claim 1, wherein J in the formula (I) is J-5.

8. A bicyclic triazolone derivative or a salt thereof as claimed in claim 1, wherein J in the formula (I) is J-6.

9. A bicyclic triazolone derivative or a salt thereof as claimed in claim 1, wherein J in the formula (I) is J-7.

10. A bicyclic triazolone derivative or a salt thereof as claimed in claim 1, wherein J in the formula (I) is J-8.

11. A herbicidal composition, which comprises a bicyclic triazolone derivative or a salt thereof as claimed in any of claims 1 to 10.
